# EUROPEAN PATENT APPLICATION

(11) **EP 3 575 325 A1**
(43) Date of publication of application: **04.12.2019**
(21) Application number: 17886078.9
(22) Date of filing: 27.12.2017
(51) Int. Cl.: C07K 19/00, C12N 15/62, C12N 15/63, C12N 5/10, G01N 33/68, A61K 35/17, A61P 35/00

(54) **MULTI-TARGET CHIMERIC ANTIGEN RECEPTOR**

(30) Priority: 29.12.2016 CN 201611246563
(71) Applicant: Timmune Biotech Inc., Tianjin 300000 (CN)
(72) Inventor: WANG, Lei, Tianjin 300000 (CN); GAO, Bin, Tianjin 300000 (CN); WU, Yasong, Tianjin 300000 (CN); SI, Yuan, Tianjin 300000 (CN); LV, Dan, Tianjin 300000 (CN); WEI, Qing, Tianjin 300000 (CN)
(74) Representative: ABG Intellectual Property Law, S.L.
(86) International application number: PCT/CN2017/118980
(87) International publication number: WO 2018/121604

(57) **Abstract**

Disclosed in the present invention is a multi-target chimeric antigen receptor. Provided in the present invention is a multi-target chimeric antigen receptor consisting of a main peptide chain and an auxiliary peptide chain; the main peptide chain comprises an antigen binding domain A, an auxiliary peptide chain connecting domain B, a transmembrane domain C and an intracellular signaling domain D; the auxiliary peptide chain comprises a main peptide chain connecting domain F; the antigen binding domain A is a polypeptide having an antigen-binding function; the auxiliary chain connecting domain B and the main peptide connecting domain F are combined with each other; the transmembrane domain C is a transmembrane domain of any membrane-binding protein or a transmembrane protein; and the intracellular signaling domain D comprises a primary signaling region. The multi-target chimeric antigen receptor of the present invention can bind to different antigens through the two antigen binding domains thereof, and mediates specific cell killing; and a cytokine and cytokine receptor complex playing the role of a cytokine are introduced into the multi-target chimeric antigen receptor of the present invention.

SEQUENCE LISTING

<110> TIMMUNE BIOTECH INC.
<120> Multi-target chimeric antigen receptor

<130> P18311EPPC

<140> EP 17886078.9
<141> 2017-12-27

<150> CN 201611246563.4
<151> 2016-12-29

<160> 54

<170> PatentIn version 3.5

<210> 1
<211> 245
<212> PRT
<213> Synthetic sequence
<220>
<223>
<400> 1

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ser Lys Tyr
            20                  25                  30

Leu Asn Trp Tyr Gln Gln Lys Pro Asp Gly Thr Val Lys Leu Leu Ile
        35                  40                  45

Tyr His Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asn Thr Leu Pro Tyr
                85                  90                  95

Thr Phe Gly Gly Gly Thr Lys Leu Glu Ile Thr Gly Ser Thr Ser Gly
            100                 105                 110

Ser Gly Lys Pro Gly Ser Gly Glu Gly Ser Thr Lys Gly Glu Val Lys
        115                 120                 125

Leu Gln Glu Ser Gly Pro Gly Leu Val Ala Pro Ser Gln Ser Leu Ser
    130                 135                 140

Val Thr Cys Thr Val Ser Gly Val Ser Leu Pro Asp Tyr Gly Val Ser
145                 150                 155                 160

Trp Ile Arg Gln Pro Pro Arg Lys Gly Leu Glu Trp Leu Gly Val Ile
                165                 170                 175

Trp Gly Ser Glu Thr Thr Tyr Tyr Asn Ser Ala Leu Lys Ser Arg Leu
            180                 185                 190

Thr Ile Ile Lys Asp Asn Ser Lys Ser Gln Val Phe Leu Lys Met Asn
        195                 200                 205

Ser Leu Gln Thr Asp Asp Thr Ala Ile Tyr Tyr Cys Ala Lys His Tyr
    210                 215                 220

Tyr Tyr Gly Gly Ser Tyr Ala Met Asp Tyr Trp Gly Gln Gly Thr Ser
225                 230                 235                 240

Val Thr Val Ser Ser
                245

<210>  2
<211>  150
<212>  PRT
<220>
<223>
<400>  2

<210>  3
<211>  114
<212>  PRT
<220>
<223>
<400>  3

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asn Ser Leu Ser Ser Asn Gly Asn Val
65                  70                  75                  80

Thr Glu Ser Gly Cys Lys Glu Cys Glu Glu Leu Glu Lys Lys Asn Ile
                85                  90                  95

Lys Glu Phe Leu Gln Ser Phe Val His Ile Val Gln Met Phe Ile Asn
            100                 105                 110

Thr Ser

<210>  4
<211>  72
<212>  PRT
<220>
<223>
<400>  4

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Asp Pro Ala Leu Val His Gln
65                  70

<210>  5
<211>  69
<212>  PRT
<220>
<223>

<400>  5

Thr Thr Thr Pro Ala Pro Arg Pro Pro Thr Pro Ala Pro Thr Ile Ala
1               5                   10                  15

Ser Gln Pro Leu Ser Leu Arg Pro Glu Ala Cys Arg Pro Ala Ala Gly
            20                  25                  30

Gly Ala Val His Thr Arg Gly Leu Asp Phe Ala Cys Asp Ile Tyr Ile
        35                  40                  45

Trp Ala Pro Leu Ala Gly Thr Cys Gly Val Leu Leu Leu Ser Leu Val
    50                  55                  60

Ile Thr Leu Tyr Cys
65

<210>  6
<211>  112
<212>  PRT
<220>
<223>
<400>  6

Arg Val Lys Phe Ser Arg Ser Ala Asp Ala Pro Ala Tyr Lys Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210>  7
<211>  41
<212>  PRT
<220>
<223>
<400>  7

Arg Ser Lys Arg Ser Arg Leu Leu His Ser Asp Tyr Met Asn Met Thr
1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40

<210>  8
<211>  42
<212>  PRT
<220>
<223>
<400>  8

Lys Arg Gly Arg Lys Lys Leu Leu Tyr Ile Phe Lys Gln Pro Phe Met
1               5                   10                  15

Arg Pro Val Gln Thr Thr Gln Glu Glu Asp Gly Cys Ser Cys Arg Phe
            20                  25                  30

Pro Glu Glu Glu Glu Gly Gly Cys Glu Leu
        35                  40

<210>  9
<211>  547
<212>  PRT
<220>
<223>
<400>  9

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ser Lys Tyr

20                  25                  30

Leu Asn Trp Tyr Gln Gln Lys Pro Asp Gly Thr Val Lys Leu Leu Ile
        35                  40                  45

Tyr His Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asn Thr Leu Pro Tyr
                85                  90                  95

Thr Phe Gly Gly Gly Thr Lys Leu Glu Ile Thr Gly Ser Thr Ser Gly
            100                 105                 110

Ser Gly Lys Pro Gly Ser Gly Glu Gly Ser Thr Lys Gly Glu Val Lys
        115                 120                 125

Leu Gln Glu Ser Gly Pro Gly Leu Val Ala Pro Ser Gln Ser Leu Ser
    130                 135                 140

Val Thr Cys Thr Val Ser Gly Val Ser Leu Pro Asp Tyr Gly Val Ser
145                 150                 155                 160

Trp Ile Arg Gln Pro Pro Arg Lys Gly Leu Glu Trp Leu Gly Val Ile
                165                 170                 175

Trp Gly Ser Glu Thr Thr Tyr Tyr Asn Ser Ala Leu Lys Ser Arg Leu
            180                 185                 190

Thr Ile Ile Lys Asp Asn Ser Lys Ser Gln Val Phe Leu Lys Met Asn
        195                 200                 205

Ser Leu Gln Thr Asp Asp Thr Ala Ile Tyr Tyr Cys Ala Lys His Tyr
    210                 215                 220

Tyr Tyr Gly Gly Ser Tyr Ala Met Asp Tyr Trp Gly Gln Gly Thr Ser
225                 230                 235                 240

Val Thr Val Ser Ser Gly Gly Gly Gly Ser Ile Thr Cys Pro Pro Pro
                245                 250                 255

Met Ser Val Glu His Ala Asp Ile Trp Val Lys Ser Tyr Ser Leu Tyr
            260                 265                 270

Ser Arg Glu Arg Tyr Ile Cys Asn Ser Gly Phe Lys Arg Lys Ala Gly
        275                 280                 285

Thr Ser Ser Leu Thr Glu Cys Val Leu Asn Lys Ala Thr Asn Val Ala
    290                 295                 300

His Trp Thr Thr Pro Ser Leu Lys Cys Ile Arg Asp Pro Ala Leu Val
305                 310                 315                 320

His Gln Thr Thr Thr Pro Ala Pro Arg Pro Pro Thr Pro Ala Pro Thr
                325                 330                 335

Ile Ala Ser Gln Pro Leu Ser Leu Arg Pro Glu Ala Cys Arg Pro Ala
            340                 345                 350

Ala Gly Gly Ala Val His Thr Arg Gly Leu Asp Phe Ala Cys Asp Ile
        355                 360                 365

Tyr Ile Trp Ala Pro Leu Ala Gly Thr Cys Gly Val Leu Leu Leu Ser
    370                 375                 380

Leu Val Ile Thr Leu Tyr Cys Lys Arg Gly Arg Lys Lys Leu Leu Tyr
385                 390                 395                 400

Ile Phe Lys Gln Pro Phe Met Arg Pro Val Gln Thr Thr Gln Glu Glu
                405                 410                 415

Asp Gly Cys Ser Cys Arg Phe Pro Glu Glu Glu Glu Gly Gly Cys Glu
            420                 425                 430

Leu Arg Val Lys Phe Ser Arg Ser Ala Asp Ala Pro Ala Tyr Lys Gln
        435                 440                 445

Gly Gln Asn Gln Leu Tyr Asn Glu Leu Asn Leu Gly Arg Arg Glu Glu
    450                 455                 460

Tyr Asp Val Leu Asp Lys Arg Arg Gly Arg Asp Pro Glu Met Gly Gly
465                 470                 475                 480

Lys Pro Arg Arg Lys Asn Pro Gln Glu Gly Leu Tyr Asn Glu Leu Gln
                485                 490                 495

Lys Asp Lys Met Ala Glu Ala Tyr Ser Glu Ile Gly Met Lys Gly Glu
            500                 505                 510

Arg Arg Arg Gly Lys Gly His Asp Gly Leu Tyr Gln Gly Leu Ser Thr
        515                 520                 525

Ala Thr Lys Asp Thr Tyr Asp Ala Leu His Met Gln Ala Leu Pro Pro
    530                 535                 540

Arg Lys Leu
545

<210>  10
<211>  269
<212>  PRT
<220>
<223>
<400>  10

Pro Gly Trp Phe Leu Asp Ser Pro Asp Arg Pro Trp Asn Pro Pro Thr
1               5                   10                  15

Phe Ser Pro Ala Leu Leu Val Val Thr Glu Gly Asp Asn Ala Thr Phe
            20                  25                  30

Thr Cys Ser Phe Ser Asn Thr Ser Glu Ser Phe Val Leu Asn Trp Tyr
        35                  40                  45

Arg Met Ser Pro Ser Asn Gln Thr Asp Lys Leu Ala Ala Phe Pro Glu
    50                  55                  60

Asp Arg Ser Gln Pro Gly Gln Asp Cys Arg Phe Arg Val Thr Gln Leu
65                  70                  75                  80

Pro Asn Gly Arg Asp Phe His Met Ser Val Val Arg Ala Arg Arg Asn
                85                  90                  95

Asp Ser Gly Thr Tyr Leu Cys Gly Ala Ile Ser Leu Ala Pro Lys Ala
            100                 105                 110

Gln Ile Lys Glu Ser Leu Arg Ala Glu Leu Arg Val Thr Glu Arg Arg
        115                 120                 125

Ala Glu Val Pro Thr Ala His Pro Ser Pro Ser Pro Arg Pro Ala Gly
    130                 135                 140

Gln Phe Gln Thr Leu Val Gly Gly Gly Gly Ser Asn Trp Val Asn Val
145                 150                 155                 160

Ile Ser Asp Leu Lys Lys Ile Glu Asp Leu Ile Gln Ser Met His Ile
                165                 170                 175

Asp Ala Thr Leu Tyr Thr Glu Ser Asp Val His Pro Ser Cys Lys Val
            180                 185                 190

Thr Ala Met Lys Cys Phe Leu Leu Glu Leu Gln Val Ile Ser Leu Glu
        195                 200                 205

Ser Gly Asp Ala Ser Ile His Asp Thr Val Glu Asn Leu Ile Ile Leu
    210                 215                 220

Ala Asn Asn Ser Leu Ser Ser Asn Gly Asn Val Thr Glu Ser Gly Cys
225                 230                 235                 240

Lys Glu Cys Glu Glu Leu Glu Lys Lys Asn Ile Lys Glu Phe Leu Gln
                245                 250                 255

Ser Phe Val His Ile Val Gln Met Phe Ile Asn Thr Ser
            260                 265

<210>  11
<211>  21
<212>  PRT
<220>
<223>
<400>  11

Met Ala Leu Pro Val Thr Ala Leu Leu Leu Pro Leu Ala Leu Leu Leu
1               5                   10                  15

His Ala Ala Arg Pro
            20

<210>  12
<211>  22
<212>  PRT
<220>
<223>
<400>  12

Gly Ser Gly Ala Thr Asn Phe Ser Leu Leu Lys Gln Ala Gly Asp Val
1               5                   10                  15

Glu Glu Asn Pro Gly Pro
            20

<210>  13
<211>  1698
<212>  DNA
<220>
<223>
<400>  13
atggccttac cagtgaccgc cttgctcctg ccgctggcct tgctgctcca cgccgccagg      60
ccggatatcc agatgacaca gactacatcc tccctgtctg cctctctggg agacagagtc     120
accatcagtt gcagggcaag tcaggacatt agtaaatatt taaattggta tcagcagaaa     180
ccagatggaa ctgttaaact cctgatctac catacatcaa gattacactc aggagtccca     240
tcaaggttca gtggcagtgg gtctggaaca gattattctc tcaccattag caacctggag     300
caagaagata ttgccactta cttttgccaa cagggtaata cgcttccgta cacgttcgga     360

ggggggacca agctggagat cacaggctcc acctctggct ccggcaagcc cggatctggc 420
gagggctcca ccaagggcga ggtgaaactg caggagtcag gacctggcct ggtggcgccc 480
tcacagagcc tgtccgtcac atgcactgtc tcaggggtct cattacccga ctatggtgta 540
agctggattc gccagcctcc acgaaagggt ctggagtggc tgggagtaat atggggtagt 600
gaaaccacat actataattc agctctcaaa tccagactga ccatcatcaa ggacaactcc 660
aagagccaag ttttcttaaa aatgaacagt ctgcaaactg atgacacagc catttactac 720
tgtgccaaac attattacta cggtggtagc tatgctatgg actactgggg ccaaggaacc 780
tcagtcaccg tgagctcagg cggcggcgga tctatcacgt gccctccccc catgtccgtg 840
gaacacgcag acatctgggt caagagctac agcttgtact ccagggagcg gtacatttgt 900
aactctggtt tcaagcgtaa agccggcacg tccagcctga cggagtgcgt gttgaacaag 960
gccacgaatg tcgcccactg gacaaccccc agtctcaaat gcattagaga ccctgccctg 1020
gttcaccaaa ccacgacgcc agcgccgcga ccaccaacac cggcgcccac catcgcgtcg 1080
cagcccctgt ccctgcgccc agaggcgtgc cggccagcgg cggggggcgc agtgcacacg 1140
agggggctgg acttcgcctg tgacatctac atctgggcgc ccttggccgg gacttgtggg 1200
gtccttctcc tgtcactggt tatcaccctt tactgcaaac ggggcagaaa gaaactcctg 1260
tatatattca aacaaccatt tatgagacca gtacaaacta ctcaagagga agatggctgt 1320
agctgccgat ttccagaaga agaagaagga ggatgtgaac tgagagtgaa gttcagcagg 1380
agcgcagacg cccccgcgta caagcagggc cagaaccagc tctataacga actcaatcta 1440
ggacgaagag aggagtacga tgttttggac aagagacgtg gccgggaccc tgagatgggg 1500
ggaaagccga gaaggaagaa ccctcaggaa ggcctgtaca atgaactgca gaaagataag 1560
atggcggagg cctacagtga gattgggatg aaaggcgagc ggcggagggg caaggggcac 1620
gatggccttt accagggtct cagtacagcc accaaggaca cctacgacgc ccttcacatg 1680
caggccctgc cccctcgc 1698

<210> 14
<211> 879
<212> DNA
<220>
<223>
<400> 14
ggaagcggcg ccaccaactt ctccctgctg aagcaagctg gcgacgtgga agagaacccc 60

ggccccccag gatggttctt agactcccca gacaggccct ggaacccccc caccttctcc 120

ccagccctgc tcgtggtgac cgaaggggac aacgccacct tcacctgcag cttctccaac 180

acatcggaga gcttcgtgct aaactggtac cgcatgagcc ccagcaacca gacggacaag 240

ctggccgcct tccccgagga ccgcagccag cccggccagg actgccgctt ccgtgtcaca 300

caactgccca acgggcgtga cttccacatg agcgtggtca gggcccggcg caatgacagc 360

ggcacctacc tctgtggggc catctccctg gcccccaagg cgcagatcaa agagagcctg 420

cgggcagagc tcagggtgac agagagaagg gcagaagtgc ccacagccca ccccagcccc 480

tcacccaggc cagccggcca gttccaaacc ctggtgggcg gaggaggctc caactgggtg 540

aacgtcatct ccgacctcaa gaagatcgag gacctgatcc agagcatgca catcgacgcc 600

accctgtata ccgagagcga cgtgcacccc tcctgtaaag tgaccgccat gaagtgcttc 660

ctgctggagc tgcaggtgat cagcctggag agcggcgacg ccagcatcca tgacaccgtg 720

gagaacctga tcatcctggc caataacagc ctgagctcca acggcaacgt gaccgagagc 780

ggctgcaagg aatgcgagga gctggagaag aagaacatta aggagttcct gcagagcttc 840

gtccacatcg tgcagatgtt cattaacacc tcctaatga 879


<210> 15
<211> 125

<212>  PRT
<220>
<223>
<400>  15

Asp Ile Gln Leu Gln Ala Ser Gly Gly Gly Leu Val Gln Pro Gly Gly
1               5                   10                  15

Ser Leu Arg Leu Ser Cys Ala Ala Ser Gly Arg Thr Phe Asn Val Asp
            20                  25                  30

Ala Met Ala Trp Phe Arg Gln Thr Arg Gly Lys Glu Arg Glu Phe Val
        35                  40                  45

Ala Ala Ile Ser Arg Ser Gly Gly Ser Thr Tyr Tyr Ala Asp Ser Val
    50                  55                  60

Lys Gly Arg Phe Ser Ile Ser Lys Asp Asn Ala Lys Asn Thr Met Tyr
65                  70                  75                  80

Leu Gln Met Asn Ser Leu Lys Pro Glu Asp Thr Ala Ile Tyr Tyr Cys
                85                  90                  95

Ala Ala Ala Ile Tyr Trp Arg Gly Ser Tyr Tyr Thr Glu Gly Asn Tyr
            100                 105                 110

Asp Tyr Trp Gly Gln Arg Thr Gln Val Thr Val Ser Ser
        115                 120                 125

<210>  16
<211>  117
<212>  PRT
<220>
<223>
<400>  16

Asp Val Gln Leu Gln Ala Ser Gly Gly Gly Leu Val Gln Ala Gly Asp
1               5                   10                  15

Ser Leu Arg Leu Ser Cys Ala Ala Ser Gly Arg Thr Phe Ser Thr Tyr
            20                  25                  30

Asn Met Gly Trp Phe Arg Gln Ala Pro Gly Lys Asp Arg Glu Phe Val
        35                  40                  45

Ala Ala Ile Met Trp Ser Gly Gly Ser Thr Tyr Tyr Ala Asp Ser Val
    50                  55                  60

Lys Gly Arg Phe Thr Ile Ser Lys Asp Asn Ala Lys Asn Thr Val Tyr
65                  70                  75                  80

Leu Gln Met Asn Ser Leu Lys Pro Glu Asp Thr Ala Val Tyr Phe Cys
                85                  90                  95

Trp Ser Thr Asp Asp Tyr Gly Val Asp Ser Trp Gly Gln Gly Thr Gln
            100                 105                 110

Val Thr Val Ser Ser
        115

<210>  17
<211>  243
<212>  PRT
<220>
<223>
<400>  17

Glu Val Gln Leu Val Glu Ser Gly Gly Gly Leu Val Gln Pro Gly Arg
1               5                   10                  15

Ser Leu Arg Leu Ser Cys Ala Ala Ser Gly Phe Thr Phe Asn Asp Tyr
            20                  25                  30

Ala Met His Trp Val Arg Gln Ala Pro Gly Lys Gly Leu Glu Trp Val
        35                  40                  45

Ser Thr Ile Ser Trp Asn Ser Gly Ser Ile Gly Tyr Ala Asp Ser Val
    50                  55                  60

Lys Gly Arg Phe Thr Ile Ser Arg Asp Asn Ala Lys Lys Ser Leu Tyr
65                  70                  75                  80

Leu Gln Met Asn Ser Leu Arg Ala Glu Asp Thr Ala Leu Tyr Tyr Cys
                85                  90                  95

Ala Lys Asp Ile Gln Tyr Gly Asn Tyr Tyr Tyr Gly Met Asp Val Trp
            100                 105                 110

Gly Gln Gly Thr Thr Val Thr Val Ser Ser Gly Gly Gly Gly Ser Gly
        115                 120                 125

Gly Gly Gly Ser Gly Gly Gly Gly Ser Glu Ile Val Leu Thr Gln Ser
    130                 135                 140

Pro Ala Thr Leu Ser Leu Ser Pro Gly Glu Arg Ala Thr Leu Ser Cys
145                 150                 155                 160

Arg Ala Ser Gln Ser Val Ser Ser Tyr Leu Ala Trp Tyr Gln Gln Lys
                165                 170                 175

Pro Gly Gln Ala Pro Arg Leu Leu Ile Tyr Asp Ala Ser Asn Arg Ala
            180                 185                 190

Thr Gly Ile Pro Ala Arg Phe Ser Gly Ser Gly Ser Gly Thr Asp Phe
        195                 200                 205

Thr Leu Thr Ile Ser Ser Leu Glu Pro Glu Asp Phe Ala Val Tyr Tyr
    210                 215                 220

Cys Gln Gln Arg Ser Asn Trp Pro Ile Thr Phe Gly Gln Gly Thr Arg
225                 230                 235                 240

Leu Glu Ile

<210> 18
<211> 249
<212> PRT
<220>
<223>
<400> 18

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Thr Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Glu Val Thr Gly Asp Leu Glu Asp Ala Phe Asp
            100                 105                 110

Ile Trp Gly Gln Gly Thr Met Val Thr Val Ser Ser Gly Ser Thr Ser
        115                 120                 125

Gly Ser Gly Lys Pro Gly Ser Gly Glu Gly Ser Thr Lys Gly Asp Ile
    130                 135                 140

Gln Met Thr Gln Ser Pro Ser Ser Leu Ser Ala Ser Val Gly Asp Arg
145                 150                 155                 160

Val Thr Ile Thr Cys Arg Ala Ser Gln Thr Ile Trp Ser Tyr Leu Asn
                165                 170                 175

Trp Tyr Gln Gln Arg Pro Gly Lys Ala Pro Asn Leu Leu Ile Tyr Ala
            180                 185                 190

Ala Ser Ser Leu Gln Ser Gly Val Pro Ser Arg Phe Ser Gly Arg Gly
        195                 200                 205

Ser Gly Thr Asp Phe Thr Leu Thr Ile Ser Ser Leu Gln Ala Glu Asp
    210                 215                 220

Phe Ala Thr Tyr Tyr Cys Gln Gln Ser Tyr Ser Ile Pro Gln Thr Phe
225                 230                 235                 240

Gly Gln Gly Thr Lys Leu Glu Ile Lys
                245

<210>  19
<211>  118
<212>  PRT
<220>
<223>
<400>  19

Gln Leu Leu Glu Ser Gly Gly Gly Leu Val Gln Pro Gly Gly Ser Leu
1               5                   10                  15

Arg Leu Ser Cys Ala Ala Ser Gly Tyr Arg Ile Ser Ser Asp Asp Met
            20                  25                  30

Ser Trp Val Arg Gln Ala Pro Gly Lys Gly Leu Glu Trp Val Ser Thr
        35                  40                  45

Ile Tyr Glu Thr Asp Gly Ser Thr Tyr Tyr Ala Asp Ser Val Lys Gly
    50                  55                  60

Arg Phe Thr Ile Ser Arg Asp Asn Ser Lys Asn Thr Leu Tyr Leu Gln
65                  70                  75                  80

Met Asn Ser Leu Arg Ala Glu Asp Thr Ala Val Tyr Tyr Cys Ala Arg
                85                  90                  95

Ala Asn Phe Tyr Ser Glu Gln Pro Phe Gln Phe Trp Gly Gln Gly Thr
            100                 105                 110

Leu Val Thr Val Ser Ser
        115

<210>  20
<211>  182
<212>  PRT
<220>
<223>
<400>  20

Met Lys Val Leu Gln Glu Pro Thr Cys Val Ser Asp Tyr Met Ser Ile
1               5                   10                  15

Ser Thr Cys Glu Trp Lys Met Asn Gly Pro Thr Asn Cys Ser Thr Glu
            20                  25                  30

Leu Arg Leu Leu Tyr Gln Leu Val Phe Leu Leu Ser Glu Ala His Thr
        35                  40                  45

Cys Ile Pro Glu Asn Asn Gly Gly Ala Gly Cys Val Cys His Leu Leu
    50                  55                  60

Met Asp Asp Val Val Ser Ala Asp Asn Tyr Thr Leu Asp Leu Trp Ala
65                  70                  75                  80

Gly Gln Gln Leu Leu Trp Lys Gly Ser Phe Lys Pro Ser Glu His Val
                85                  90                  95

Lys Pro Arg Ala Pro Gly Asn Leu Thr Val His Thr Asn Val Ser Asp
            100                 105                 110

Thr Leu Leu Leu Thr Trp Ser Asn Pro Tyr Pro Pro Asp Asn Tyr Leu
        115                 120                 125

Tyr Asn His Leu Thr Tyr Ala Val Asn Ile Trp Ser Glu Asn Asp Pro
    130                 135                 140

Ala Asp Phe Arg Ile Tyr Asn Val Thr Tyr Leu Glu Pro Ser Leu Arg
145                 150                 155                 160

Ile Ala Ala Ser Thr Leu Lys Ser Gly Ile Ser Tyr Arg Ala Arg Val
                165                 170                 175

Arg Ala Trp Ala Gln Cys
            180

<210>  21
<211>  129
<212>  PRT

<220>
<223>
<400> 21

His Lys Cys Asp Ile Thr Leu Gln Glu Ile Ile Lys Thr Leu Asn Ser
1               5                   10                  15

Leu Thr Glu Gln Lys Thr Leu Cys Thr Glu Leu Thr Val Thr Asp Ile
            20                  25                  30

Phe Ala Ala Ser Lys Asn Thr Thr Glu Lys Glu Thr Phe Cys Arg Ala
        35                  40                  45

Ala Thr Val Leu Arg Gln Phe Tyr Ser His His Glu Lys Asp Thr Arg
    50                  55                  60

Cys Leu Gly Ala Thr Ala Gln Gln Phe His Arg His Lys Gln Leu Ile
65                  70                  75                  80

Arg Leu Leu Lys Arg Leu Asp Arg Asn Leu Trp Gly Leu Ala Gly Leu
                85                  90                  95

Asn Ser Cys Pro Val Lys Glu Ala Asn Gln Ser Thr Leu Glu Asn Phe
            100                 105                 110

Leu Glu Arg Leu Lys Thr Ile Met Arg Glu Lys Tyr Ser Lys Cys Ser
        115                 120                 125

Ser

<210> 22
<211> 27
<212> PRT
<220>
<223>
<400> 22

Phe Trp Val Leu Val Val Val Gly Gly Val Leu Ala Cys Tyr Ser Leu
1               5                   10                  15

Leu Val Thr Val Ala Phe Ile Ile Phe Trp Val
            20                  25

<210> 23
<211> 425
<212> PRT
<220>
<223>
<400> 23

Asp Ile Gln Leu Gln Ala Ser Gly Gly Gly Leu Val Gln Pro Gly Gly

1               5                   10                  15

Ser Leu Arg Leu Ser Cys Ala Ala Ser Gly Arg Thr Phe Asn Val Asp
            20                  25                  30

Ala Met Ala Trp Phe Arg Gln Thr Arg Gly Lys Glu Arg Glu Phe Val
        35                  40                  45

Ala Ala Ile Ser Arg Ser Gly Gly Ser Thr Tyr Tyr Ala Asp Ser Val
    50                  55                  60

Lys Gly Arg Phe Ser Ile Ser Lys Asp Asn Ala Lys Asn Thr Met Tyr
65                  70                  75                  80

Leu Gln Met Asn Ser Leu Lys Pro Glu Asp Thr Ala Ile Tyr Tyr Cys
                85                  90                  95

Ala Ala Ala Ile Tyr Trp Arg Gly Ser Tyr Tyr Thr Glu Gly Asn Tyr
            100                 105                 110

Asp Tyr Trp Gly Gln Arg Thr Gln Val Thr Val Ser Ser Gly Gly Gly
        115                 120                 125

Gly Ser Ile Thr Cys Pro Pro Pro Met Ser Val Glu His Ala Asp Ile
    130                 135                 140

Trp Val Lys Ser Tyr Ser Leu Tyr Ser Arg Glu Arg Tyr Ile Cys Asn
145                 150                 155                 160

Ser Gly Phe Lys Arg Lys Ala Gly Thr Ser Ser Leu Thr Glu Cys Val
                165                 170                 175

Leu Asn Lys Ala Thr Asn Val Ala His Trp Thr Thr Pro Ser Leu Lys
            180                 185                 190

Cys Ile Arg Asp Pro Ala Leu Val His Gln Thr Thr Thr Pro Ala Pro
        195                 200                 205

Arg Pro Pro Thr Pro Ala Pro Thr Ile Ala Ser Gln Pro Leu Ser Leu
    210                 215                 220

Arg Pro Glu Ala Cys Arg Pro Ala Ala Gly Gly Ala Val His Thr Arg
225                 230                 235                 240

Gly Leu Asp Phe Ala Cys Asp Ile Tyr Ile Trp Ala Pro Leu Ala Gly
                245                 250                 255

Thr Cys Gly Val Leu Leu Leu Ser Leu Val Ile Thr Leu Tyr Cys Lys
        260                 265                 270

Arg Gly Arg Lys Lys Leu Leu Tyr Ile Phe Lys Gln Pro Phe Met Arg
    275                 280                 285

Pro Val Gln Thr Thr Gln Glu Glu Asp Gly Cys Ser Cys Arg Phe Pro
    290                 295                 300

Glu Glu Glu Glu Gly Gly Cys Glu Leu Arg Val Lys Phe Ser Arg Ser
305                 310                 315                 320

Ala Asp Ala Pro Ala Tyr Lys Gln Gly Gln Asn Gln Leu Tyr Asn Glu
                325                 330                 335

Leu Asn Leu Gly Arg Arg Glu Glu Tyr Asp Val Leu Asp Lys Arg Arg
        340                 345                 350

Gly Arg Asp Pro Glu Met Gly Gly Lys Pro Arg Arg Lys Asn Pro Gln
    355                 360                 365

Glu Gly Leu Tyr Asn Glu Leu Gln Lys Asp Lys Met Ala Glu Ala Tyr
    370                 375                 380

Ser Glu Ile Gly Met Lys Gly Glu Arg Arg Arg Gly Lys Gly His Asp
385                 390                 395                 400

Gly Leu Tyr Gln Gly Leu Ser Thr Ala Thr Lys Asp Thr Tyr Asp Ala
                405                 410                 415

Leu His Met Gln Ala Leu Pro Pro Arg
        420                 425

<210>  24
<211>  418
<212>  PRT
<220>
<223>
<400>  24

Gln Leu Leu Glu Ser Gly Gly Gly Leu Val Gln Pro Gly Gly Ser Leu
1               5                   10                  15

Arg Leu Ser Cys Ala Ala Ser Gly Tyr Arg Ile Ser Ser Asp Asp Met
            20                  25                  30

Ser Trp Val Arg Gln Ala Pro Gly Lys Gly Leu Glu Trp Val Ser Thr
        35                  40                  45

Ile Tyr Glu Thr Asp Gly Ser Thr Tyr Tyr Ala Asp Ser Val Lys Gly
    50                  55                  60

Arg Phe Thr Ile Ser Arg Asp Asn Ser Lys Asn Thr Leu Tyr Leu Gln
65                  70                  75                  80

Met Asn Ser Leu Arg Ala Glu Asp Thr Ala Val Tyr Tyr Cys Ala Arg
                85                  90                  95

Ala Asn Phe Tyr Ser Glu Gln Pro Phe Gln Phe Trp Gly Gln Gly Thr
            100                 105                 110

Leu Val Thr Val Ser Ser Gly Gly Gly Gly Ser Ile Thr Cys Pro Pro
        115                 120                 125

Pro Met Ser Val Glu His Ala Asp Ile Trp Val Lys Ser Tyr Ser Leu
    130                 135                 140

Tyr Ser Arg Glu Arg Tyr Ile Cys Asn Ser Gly Phe Lys Arg Lys Ala
145                 150                 155                 160

Gly Thr Ser Ser Leu Thr Glu Cys Val Leu Asn Lys Ala Thr Asn Val
                165                 170                 175

Ala His Trp Thr Thr Pro Ser Leu Lys Cys Ile Arg Asp Pro Ala Leu
            180                 185                 190

Val His Gln Thr Thr Thr Pro Ala Pro Arg Pro Pro Thr Pro Ala Pro
        195                 200                 205

Thr Ile Ala Ser Gln Pro Leu Ser Leu Arg Pro Glu Ala Cys Arg Pro
    210                 215                 220

Ala Ala Gly Gly Ala Val His Thr Arg Gly Leu Asp Phe Ala Cys Asp
225                 230                 235                 240

Ile Tyr Ile Trp Ala Pro Leu Ala Gly Thr Cys Gly Val Leu Leu Leu
                245                 250                 255

Ser Leu Val Ile Thr Leu Tyr Cys Lys Arg Gly Arg Lys Lys Leu Leu
            260                 265                 270

Tyr Ile Phe Lys Gln Pro Phe Met Arg Pro Val Gln Thr Thr Gln Glu
        275                 280                 285

Glu Asp Gly Cys Ser Cys Arg Phe Pro Glu Glu Glu Glu Gly Gly Cys
    290                 295                 300

Glu Leu Arg Val Lys Phe Ser Arg Ser Ala Asp Ala Pro Ala Tyr Lys
305                 310                 315                 320

Gln Gly Gln Asn Gln Leu Tyr Asn Glu Leu Asn Leu Gly Arg Arg Glu
                325                 330                 335

Glu Tyr Asp Val Leu Asp Lys Arg Arg Gly Arg Asp Pro Glu Met Gly
            340                 345                 350

Gly Lys Pro Arg Arg Lys Asn Pro Gln Glu Gly Leu Tyr Asn Glu Leu
        355                 360                 365

Gln Lys Asp Lys Met Ala Glu Ala Tyr Ser Glu Ile Gly Met Lys Gly
    370                 375                 380

Glu Arg Arg Arg Gly Lys Gly His Asp Gly Leu Tyr Gln Gly Leu Ser
385                 390                 395                 400

Thr Ala Thr Lys Asp Thr Tyr Asp Ala Leu His Met Gln Ala Leu Pro
                405                 410                 415

Pro Arg

<210>  25
<211>  376
<212>  PRT
<220>
<223>
<400>  25

Gln Leu Leu Glu Ser Gly Gly Gly Leu Val Gln Pro Gly Gly Ser Leu
1               5                   10                  15

Arg Leu Ser Cys Ala Ala Ser Gly Tyr Arg Ile Ser Ser Asp Asp Met
            20                  25                  30

Ser Trp Val Arg Gln Ala Pro Gly Lys Gly Leu Glu Trp Val Ser Thr
        35                  40                  45

Ile Tyr Glu Thr Asp Gly Ser Thr Tyr Tyr Ala Asp Ser Val Lys Gly
    50                  55                  60

Arg Phe Thr Ile Ser Arg Asp Asn Ser Lys Asn Thr Leu Tyr Leu Gln
65                  70                  75                  80

Met Asn Ser Leu Arg Ala Glu Asp Thr Ala Val Tyr Tyr Cys Ala Arg
                85                  90                  95

Ala Asn Phe Tyr Ser Glu Gln Pro Phe Gln Phe Trp Gly Gln Gly Thr
            100                 105                 110

Leu Val Thr Val Ser Ser Gly Gly Gly Gly Ser Ile Thr Cys Pro Pro
        115                 120                 125

Pro Met Ser Val Glu His Ala Asp Ile Trp Val Lys Ser Tyr Ser Leu
    130                 135                 140

Tyr Ser Arg Glu Arg Tyr Ile Cys Asn Ser Gly Phe Lys Arg Lys Ala
145                 150                 155                 160

Gly Thr Ser Ser Leu Thr Glu Cys Val Leu Asn Lys Ala Thr Asn Val
                165                 170                 175

Ala His Trp Thr Thr Pro Ser Leu Lys Cys Ile Arg Asp Pro Ala Leu
            180                 185                 190

Val His Gln Phe Trp Val Leu Val Val Val Gly Gly Val Leu Ala Cys
        195                 200                 205

Tyr Ser Leu Leu Val Thr Val Ala Phe Ile Ile Phe Trp Val Lys Arg
    210                 215                 220

Gly Arg Lys Lys Leu Leu Tyr Ile Phe Lys Gln Pro Phe Met Arg Pro
225                 230                 235                 240

Val Gln Thr Thr Gln Glu Glu Asp Gly Cys Ser Cys Arg Phe Pro Glu
                245                 250                 255

Glu Glu Glu Gly Gly Cys Glu Leu Arg Val Lys Phe Ser Arg Ser Ala
            260                 265                 270

Asp Ala Pro Ala Tyr Lys Gln Gly Gln Asn Gln Leu Tyr Asn Glu Leu
        275                 280                 285

Asn Leu Gly Arg Arg Glu Glu Tyr Asp Val Leu Asp Lys Arg Arg Gly
    290                 295                 300

Arg Asp Pro Glu Met Gly Gly Lys Pro Arg Arg Lys Asn Pro Gln Glu
305                 310                 315                 320

Gly Leu Tyr Asn Glu Leu Gln Lys Asp Lys Met Ala Glu Ala Tyr Ser
                325                 330                 335

Glu Ile Gly Met Lys Gly Glu Arg Arg Arg Gly Lys Gly His Asp Gly
            340                 345                 350

Leu Tyr Gln Gly Leu Ser Thr Ala Thr Lys Asp Thr Tyr Asp Ala Leu
        355                 360                 365

His Met Gln Ala Leu Pro Pro Arg
    370                 375

<210>  26
<211>  528
<212>  PRT
<220>
<223>
<400>  26

Gln Leu Leu Glu Ser Gly Gly Gly Leu Val Gln Pro Gly Gly Ser Leu
1               5                   10                  15

Arg Leu Ser Cys Ala Ala Ser Gly Tyr Arg Ile Ser Ser Asp Asp Met
            20                  25                  30

Ser Trp Val Arg Gln Ala Pro Gly Lys Gly Leu Glu Trp Val Ser Thr
        35                  40                  45

Ile Tyr Glu Thr Asp Gly Ser Thr Tyr Tyr Ala Asp Ser Val Lys Gly
    50                  55                  60

Arg Phe Thr Ile Ser Arg Asp Asn Ser Lys Asn Thr Leu Tyr Leu Gln
65                  70                  75                  80

Met Asn Ser Leu Arg Ala Glu Asp Thr Ala Val Tyr Tyr Cys Ala Arg
                85                  90                  95

Ala Asn Phe Tyr Ser Glu Gln Pro Phe Gln Phe Trp Gly Gln Gly Thr
            100                 105                 110

Leu Val Thr Val Ser Ser Gly Gly Gly Gly Ser Met Lys Val Leu Gln
        115                 120                 125

Glu Pro Thr Cys Val Ser Asp Tyr Met Ser Ile Ser Thr Cys Glu Trp
    130                 135                 140

Lys Met Asn Gly Pro Thr Asn Cys Ser Thr Glu Leu Arg Leu Leu Tyr
145                 150                 155                 160

Gln Leu Val Phe Leu Leu Ser Glu Ala His Thr Cys Ile Pro Glu Asn
                165                 170                 175

Asn Gly Gly Ala Gly Cys Val Cys His Leu Leu Met Asp Asp Val Val
            180                 185                 190

Ser Ala Asp Asn Tyr Thr Leu Asp Leu Trp Ala Gly Gln Gln Leu Leu
        195                 200                 205

Trp Lys Gly Ser Phe Lys Pro Ser Glu His Val Lys Pro Arg Ala Pro
    210                 215                 220

Gly Asn Leu Thr Val His Thr Asn Val Ser Asp Thr Leu Leu Leu Thr
225                 230                 235                 240

Trp Ser Asn Pro Tyr Pro Pro Asp Asn Tyr Leu Tyr Asn His Leu Thr
                245                 250                 255

Tyr Ala Val Asn Ile Trp Ser Glu Asn Asp Pro Ala Asp Phe Arg Ile
            260                 265                 270

Tyr Asn Val Thr Tyr Leu Glu Pro Ser Leu Arg Ile Ala Ala Ser Thr
        275                 280                 285

Leu Lys Ser Gly Ile Ser Tyr Arg Ala Arg Val Arg Ala Trp Ala Gln
    290                 295                 300

Cys Thr Thr Thr Pro Ala Pro Arg Pro Pro Thr Pro Ala Pro Thr Ile
305                 310                 315                 320

Ala Ser Gln Pro Leu Ser Leu Arg Pro Glu Ala Cys Arg Pro Ala Ala
                325                 330                 335

Gly Gly Ala Val His Thr Arg Gly Leu Asp Phe Ala Cys Asp Ile Tyr
            340                 345                 350

Ile Trp Ala Pro Leu Ala Gly Thr Cys Gly Val Leu Leu Leu Ser Leu
        355                 360                 365

Val Ile Thr Leu Tyr Cys Lys Arg Gly Arg Lys Lys Leu Leu Tyr Ile
    370                 375                 380

Phe Lys Gln Pro Phe Met Arg Pro Val Gln Thr Thr Gln Glu Glu Asp
385                 390                 395                 400

Gly Cys Ser Cys Arg Phe Pro Glu Glu Glu Glu Gly Gly Cys Glu Leu
                405                 410                 415

Arg Val Lys Phe Ser Arg Ser Ala Asp Ala Pro Ala Tyr Lys Gln Gly
            420                 425                 430

Gln Asn Gln Leu Tyr Asn Glu Leu Asn Leu Gly Arg Arg Glu Glu Tyr

435                 440                 445

Asp Val Leu Asp Lys Arg Arg Gly Arg Asp Pro Glu Met Gly Gly Lys
    450                 455                 460

Pro Arg Arg Lys Asn Pro Gln Glu Gly Leu Tyr Asn Glu Leu Gln Lys
465                 470                 475                 480

Asp Lys Met Ala Glu Ala Tyr Ser Glu Ile Gly Met Lys Gly Glu Arg
                485                 490                 495

Arg Arg Gly Lys Gly His Asp Gly Leu Tyr Gln Gly Leu Ser Thr Ala
            500                 505                 510

Thr Lys Asp Thr Tyr Asp Ala Leu His Met Gln Ala Leu Pro Pro Arg
        515                 520                 525

<210>  27
<211>  417
<212>  PRT
<220>
<223>
<400>  27

Gln Leu Leu Glu Ser Gly Gly Gly Leu Val Gln Pro Gly Gly Ser Leu
1               5                   10                  15

Arg Leu Ser Cys Ala Ala Ser Gly Tyr Arg Ile Ser Ser Asp Asp Met
            20                  25                  30

Ser Trp Val Arg Gln Ala Pro Gly Lys Gly Leu Glu Trp Val Ser Thr
        35                  40                  45

Ile Tyr Glu Thr Asp Gly Ser Thr Tyr Tyr Ala Asp Ser Val Lys Gly
    50                  55                  60

Arg Phe Thr Ile Ser Arg Asp Asn Ser Lys Asn Thr Leu Tyr Leu Gln
65                  70                  75                  80

Met Asn Ser Leu Arg Ala Glu Asp Thr Ala Val Tyr Tyr Cys Ala Arg
                85                  90                  95

Ala Asn Phe Tyr Ser Glu Gln Pro Phe Gln Phe Trp Gly Gln Gly Thr
            100                 105                 110

Leu Val Thr Val Ser Ser Gly Gly Gly Gly Ser Ile Thr Cys Pro Pro
        115                 120                 125

Pro Met Ser Val Glu His Ala Asp Ile Trp Val Lys Ser Tyr Ser Leu

130                 135                 140

Tyr Ser Arg Glu Arg Tyr Ile Cys Asn Ser Gly Phe Lys Arg Lys Ala
145                 150                 155                 160

Gly Thr Ser Ser Leu Thr Glu Cys Val Leu Asn Lys Ala Thr Asn Val
                165                 170                 175

Ala His Trp Thr Thr Pro Ser Leu Lys Cys Ile Arg Asp Pro Ala Leu
            180                 185                 190

Val His Gln Thr Thr Thr Pro Ala Pro Arg Pro Pro Thr Pro Ala Pro
        195                 200                 205

Thr Ile Ala Ser Gln Pro Leu Ser Leu Arg Pro Glu Ala Cys Arg Pro
    210                 215                 220

Ala Ala Gly Gly Ala Val His Thr Arg Gly Leu Asp Phe Ala Cys Asp
225                 230                 235                 240

Ile Tyr Ile Trp Ala Pro Leu Ala Gly Thr Cys Gly Val Leu Leu Leu
                245                 250                 255

Ser Leu Val Ile Thr Leu Tyr Cys Arg Ser Lys Arg Ser Arg Leu Leu
            260                 265                 270

His Ser Asp Tyr Met Asn Met Thr Pro Arg Arg Pro Gly Pro Thr Arg
        275                 280                 285

Lys His Tyr Gln Pro Tyr Ala Pro Pro Arg Asp Phe Ala Ala Tyr Arg
    290                 295                 300

Ser Arg Val Lys Phe Ser Arg Ser Ala Asp Ala Pro Ala Tyr Lys Gln
305                 310                 315                 320

Gly Gln Asn Gln Leu Tyr Asn Glu Leu Asn Leu Gly Arg Arg Glu Glu
                325                 330                 335

Tyr Asp Val Leu Asp Lys Arg Arg Gly Arg Asp Pro Glu Met Gly Gly
            340                 345                 350

Lys Pro Arg Arg Lys Asn Pro Gln Glu Gly Leu Tyr Asn Glu Leu Gln
        355                 360                 365

Lys Asp Lys Met Ala Glu Ala Tyr Ser Glu Ile Gly Met Lys Gly Glu
    370                 375                 380

Arg Arg Arg Gly Lys Gly His Asp Gly Leu Tyr Gln Gly Leu Ser Thr
385                 390                 395                 400

Ala Thr Lys Asp Thr Tyr Asp Ala Leu His Met Gln Ala Leu Pro Pro
                405                 410                 415

Arg

<210>  28
<211>  450
<212>  PRT
<220>
<223>
<400>  28

Pro Gly Trp Phe Leu Asp Ser Pro Asp Arg Pro Trp Asn Pro Pro Thr
1               5                   10                  15

Phe Ser Pro Ala Leu Leu Val Val Thr Glu Gly Asp Asn Ala Thr Phe
            20                  25                  30

Thr Cys Ser Phe Ser Asn Thr Ser Glu Ser Phe Val Leu Asn Trp Tyr
        35                  40                  45

Arg Met Ser Pro Ser Asn Gln Thr Asp Lys Leu Ala Ala Phe Pro Glu
    50                  55                  60

Asp Arg Ser Gln Pro Gly Gln Asp Cys Arg Phe Arg Val Thr Gln Leu
65                  70                  75                  80

Pro Asn Gly Arg Asp Phe His Met Ser Val Val Arg Ala Arg Arg Asn
                85                  90                  95

Asp Ser Gly Thr Tyr Leu Cys Gly Ala Ile Ser Leu Ala Pro Lys Ala
            100                 105                 110

Gln Ile Lys Glu Ser Leu Arg Ala Glu Leu Arg Val Thr Glu Arg Arg
        115                 120                 125

Ala Glu Val Pro Thr Ala His Pro Ser Pro Ser Pro Arg Pro Ala Gly
    130                 135                 140

Gln Phe Gln Thr Leu Val Gly Gly Gly Gly Ser Ile Thr Cys Pro Pro
145                 150                 155                 160

Pro Met Ser Val Glu His Ala Asp Ile Trp Val Lys Ser Tyr Ser Leu
                165                 170                 175

Tyr Ser Arg Glu Arg Tyr Ile Cys Asn Ser Gly Phe Lys Arg Lys Ala
        180                 185                 190

Gly Thr Ser Ser Leu Thr Glu Cys Val Leu Asn Lys Ala Thr Asn Val
    195                 200                 205

Ala His Trp Thr Thr Pro Ser Leu Lys Cys Ile Arg Asp Pro Ala Leu
    210                 215                 220

Val His Gln Thr Thr Thr Pro Ala Pro Arg Pro Pro Thr Pro Ala Pro
225                 230                 235                 240

Thr Ile Ala Ser Gln Pro Leu Ser Leu Arg Pro Glu Ala Cys Arg Pro
                245                 250                 255

Ala Ala Gly Gly Ala Val His Thr Arg Gly Leu Asp Phe Ala Cys Asp
            260                 265                 270

Ile Tyr Ile Trp Ala Pro Leu Ala Gly Thr Cys Gly Val Leu Leu Leu
        275                 280                 285

Ser Leu Val Ile Thr Leu Tyr Cys Lys Arg Gly Arg Lys Lys Leu Leu
    290                 295                 300

Tyr Ile Phe Lys Gln Pro Phe Met Arg Pro Val Gln Thr Thr Gln Glu
305                 310                 315                 320

Glu Asp Gly Cys Ser Cys Arg Phe Pro Glu Glu Glu Glu Gly Gly Cys
                325                 330                 335

Glu Leu Arg Val Lys Phe Ser Arg Ser Ala Asp Ala Pro Ala Tyr Lys
            340                 345                 350

Gln Gly Gln Asn Gln Leu Tyr Asn Glu Leu Asn Leu Gly Arg Arg Glu
        355                 360                 365

Glu Tyr Asp Val Leu Asp Lys Arg Arg Gly Arg Asp Pro Glu Met Gly
    370                 375                 380

Gly Lys Pro Arg Arg Lys Asn Pro Gln Glu Gly Leu Tyr Asn Glu Leu
385                 390                 395                 400

Gln Lys Asp Lys Met Ala Glu Ala Tyr Ser Glu Ile Gly Met Lys Gly
                405                 410                 415

Glu Arg Arg Arg Gly Lys Gly His Asp Gly Leu Tyr Gln Gly Leu Ser
            420                 425                 430

Thr Ala Thr Lys Asp Thr Tyr Asp Ala Leu His Met Gln Ala Leu Pro
        435                 440                 445

Pro Arg
    450

<210>  29
<211>  460
<212>  PRT
<220>
<223>
<400>  29

Gln Leu Leu Glu Ser Gly Gly Gly Leu Val Gln Pro Gly Gly Ser Leu
1               5                   10                  15

Arg Leu Ser Cys Ala Ala Ser Gly Tyr Arg Ile Ser Ser Asp Asp Met
            20                  25                  30

Ser Trp Val Arg Gln Ala Pro Gly Lys Gly Leu Glu Trp Val Ser Thr
        35                  40                  45

Ile Tyr Glu Thr Asp Gly Ser Thr Tyr Tyr Ala Asp Ser Val Lys Gly
    50                  55                  60

Arg Phe Thr Ile Ser Arg Asp Asn Ser Lys Asn Thr Leu Tyr Leu Gln
65                  70                  75                  80

Met Asn Ser Leu Arg Ala Glu Asp Thr Ala Val Tyr Tyr Cys Ala Arg
                85                  90                  95

Ala Asn Phe Tyr Ser Glu Gln Pro Phe Gln Phe Trp Gly Gln Gly Thr
            100                 105                 110

Leu Val Thr Val Ser Ser Gly Gly Gly Gly Ser Asn Trp Val Asn Val
        115                 120                 125

Ile Ser Asp Leu Lys Lys Ile Glu Asp Leu Ile Gln Ser Met His Ile
    130                 135                 140

Asp Ala Thr Leu Tyr Thr Glu Ser Asp Val His Pro Ser Cys Lys Val
145                 150                 155                 160

Thr Ala Met Lys Cys Phe Leu Leu Glu Leu Gln Val Ile Ser Leu Glu
                165                 170                 175

Ser Gly Asp Ala Ser Ile His Asp Thr Val Glu Asn Leu Ile Ile Leu
            180                 185                 190

Ala Asn Asn Ser Leu Ser Ser Asn Gly Asn Val Thr Glu Ser Gly Cys
        195                 200                 205

Lys Glu Cys Glu Glu Leu Glu Lys Lys Asn Ile Lys Glu Phe Leu Gln
    210                 215                 220

Ser Phe Val His Ile Val Gln Met Phe Ile Asn Thr Ser Thr Thr Thr
225                 230                 235                 240

Pro Ala Pro Arg Pro Pro Thr Pro Ala Pro Thr Ile Ala Ser Gln Pro
                245                 250                 255

Leu Ser Leu Arg Pro Glu Ala Cys Arg Pro Ala Ala Gly Gly Ala Val
            260                 265                 270

His Thr Arg Gly Leu Asp Phe Ala Cys Asp Ile Tyr Ile Trp Ala Pro
        275                 280                 285

Leu Ala Gly Thr Cys Gly Val Leu Leu Leu Ser Leu Val Ile Thr Leu
    290                 295                 300

Tyr Cys Lys Arg Gly Arg Lys Lys Leu Leu Tyr Ile Phe Lys Gln Pro
305                 310                 315                 320

Phe Met Arg Pro Val Gln Thr Thr Gln Glu Glu Asp Gly Cys Ser Cys
                325                 330                 335

Arg Phe Pro Glu Glu Glu Glu Gly Gly Cys Glu Leu Arg Val Lys Phe
            340                 345                 350

Ser Arg Ser Ala Asp Ala Pro Ala Tyr Lys Gln Gly Gln Asn Gln Leu
        355                 360                 365

Tyr Asn Glu Leu Asn Leu Gly Arg Arg Glu Glu Tyr Asp Val Leu Asp
    370                 375                 380

Lys Arg Arg Gly Arg Asp Pro Glu Met Gly Gly Lys Pro Arg Arg Lys
385                 390                 395                 400

Asn Pro Gln Glu Gly Leu Tyr Asn Glu Leu Gln Lys Asp Lys Met Ala
                405                 410                 415

Glu Ala Tyr Ser Glu Ile Gly Met Lys Gly Glu Arg Arg Arg Gly Lys
            420                 425                 430

Gly His Asp Gly Leu Tyr Gln Gly Leu Ser Thr Ala Thr Lys Asp Thr
        435                 440                 445

Tyr Asp Ala Leu His Met Gln Ala Leu Pro Pro Arg
    450                 455                 460

<210>  30
<211>  236
<212>  PRT
<220>
<223>
<400>  30

Asp Val Gln Leu Gln Ala Ser Gly Gly Gly Leu Val Gln Ala Gly Asp
1               5                   10                  15

Ser Leu Arg Leu Ser Cys Ala Ala Ser Gly Arg Thr Phe Ser Thr Tyr
            20                  25                  30

Asn Met Gly Trp Phe Arg Gln Ala Pro Gly Lys Asp Arg Glu Phe Val
        35                  40                  45

Ala Ala Ile Met Trp Ser Gly Gly Ser Thr Tyr Tyr Ala Asp Ser Val
    50                  55                  60

Lys Gly Arg Phe Thr Ile Ser Lys Asp Asn Ala Lys Asn Thr Val Tyr
65                  70                  75                  80

Leu Gln Met Asn Ser Leu Lys Pro Glu Asp Thr Ala Val Tyr Phe Cys
                85                  90                  95

Trp Ser Thr Asp Asp Tyr Gly Val Asp Ser Trp Gly Gln Gly Thr Gln
            100                 105                 110

Val Thr Val Ser Ser Gly Gly Gly Gly Ser Asn Trp Val Asn Val Ile
        115                 120                 125

Ser Asp Leu Lys Lys Ile Glu Asp Leu Ile Gln Ser Met His Ile Asp
    130                 135                 140

Ala Thr Leu Tyr Thr Glu Ser Asp Val His Pro Ser Cys Lys Val Thr
145                 150                 155                 160

Ala Met Lys Cys Phe Leu Leu Glu Leu Gln Val Ile Ser Leu Glu Ser
                165                 170                 175

Gly Asp Ala Ser Ile His Asp Thr Val Glu Asn Leu Ile Ile Leu Ala
            180                 185                 190

Asn Asn Ser Leu Ser Ser Asn Gly Asn Val Thr Glu Ser Gly Cys Lys
        195                 200                 205

Glu Cys Glu Glu Leu Glu Lys Lys Asn Ile Lys Glu Phe Leu Gln Ser
    210                 215                 220

Phe Val His Ile Val Gln Met Phe Ile Asn Thr Ser
225                 230                 235

<210>  31
<211>  363
<212>  PRT
<220>
<223>
<400>  31

Glu Val Gln Leu Val Glu Ser Gly Gly Gly Leu Val Gln Pro Gly Arg
1               5                   10                  15

Ser Leu Arg Leu Ser Cys Ala Ala Ser Gly Phe Thr Phe Asn Asp Tyr
            20                  25                  30

Ala Met His Trp Val Arg Gln Ala Pro Gly Lys Gly Leu Glu Trp Val
        35                  40                  45

Ser Thr Ile Ser Trp Asn Ser Gly Ser Ile Gly Tyr Ala Asp Ser Val
    50                  55                  60

Lys Gly Arg Phe Thr Ile Ser Arg Asp Asn Ala Lys Lys Ser Leu Tyr
65                  70                  75                  80

Leu Gln Met Asn Ser Leu Arg Ala Glu Asp Thr Ala Leu Tyr Tyr Cys
                85                  90                  95

Ala Lys Asp Ile Gln Tyr Gly Asn Tyr Tyr Tyr Gly Met Asp Val Trp
            100                 105                 110

Gly Gln Gly Thr Thr Val Thr Val Ser Ser Gly Gly Gly Gly Ser Gly
        115                 120                 125

Gly Gly Gly Ser Gly Gly Gly Gly Ser Glu Ile Val Leu Thr Gln Ser
    130                 135                 140

Pro Ala Thr Leu Ser Leu Ser Pro Gly Glu Arg Ala Thr Leu Ser Cys
145                 150                 155                 160

Arg Ala Ser Gln Ser Val Ser Ser Tyr Leu Ala Trp Tyr Gln Gln Lys
                165                 170                 175

Pro Gly Gln Ala Pro Arg Leu Leu Ile Tyr Asp Ala Ser Asn Arg Ala
            180                 185                 190

Thr Gly Ile Pro Ala Arg Phe Ser Gly Ser Gly Ser Gly Thr Asp Phe
        195                 200                 205

Thr Leu Thr Ile Ser Ser Leu Glu Pro Glu Asp Phe Ala Val Tyr Tyr
    210                 215                 220

Cys Gln Gln Arg Ser Asn Trp Pro Ile Thr Phe Gly Gln Gly Thr Arg
225                 230                 235                 240

Leu Glu Ile Lys Gly Gly Gly Gly Ser Asn Trp Val Asn Val Ile Ser
                245                 250                 255

Asp Leu Lys Lys Ile Glu Asp Leu Ile Gln Ser Met His Ile Asp Ala
            260                 265                 270

Thr Leu Tyr Thr Glu Ser Asp Val His Pro Ser Cys Lys Val Thr Ala
        275                 280                 285

Met Lys Cys Phe Leu Leu Glu Leu Gln Val Ile Ser Leu Glu Ser Gly
    290                 295                 300

Asp Ala Ser Ile His Asp Thr Val Glu Asn Leu Ile Ile Leu Ala Asn
305                 310                 315                 320

Asn Ser Leu Ser Ser Asn Gly Asn Val Thr Glu Ser Gly Cys Lys Glu
                325                 330                 335

Cys Glu Glu Leu Glu Lys Lys Asn Ile Lys Glu Phe Leu Gln Ser Phe
            340                 345                 350

Val His Ile Val Gln Met Phe Ile Asn Thr Ser
        355                 360

<210>  32
<211>  368
<212>  PRT
<220>
<223>
<400>  32

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Thr Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Glu Val Thr Gly Asp Leu Glu Asp Ala Phe Asp
            100                 105                 110

Ile Trp Gly Gln Gly Thr Met Val Thr Val Ser Ser Gly Ser Thr Ser
        115                 120                 125

Gly Ser Gly Lys Pro Gly Ser Gly Glu Gly Ser Thr Lys Gly Asp Ile
    130                 135                 140

Gln Met Thr Gln Ser Pro Ser Ser Leu Ser Ala Ser Val Gly Asp Arg
145                 150                 155                 160

Val Thr Ile Thr Cys Arg Ala Ser Gln Thr Ile Trp Ser Tyr Leu Asn
                165                 170                 175

Trp Tyr Gln Gln Arg Pro Gly Lys Ala Pro Asn Leu Leu Ile Tyr Ala
            180                 185                 190

Ala Ser Ser Leu Gln Ser Gly Val Pro Ser Arg Phe Ser Gly Arg Gly
        195                 200                 205

Ser Gly Thr Asp Phe Thr Leu Thr Ile Ser Ser Leu Gln Ala Glu Asp
    210                 215                 220

Phe Ala Thr Tyr Tyr Cys Gln Gln Ser Tyr Ser Ile Pro Gln Thr Phe
225                 230                 235                 240

Gly Gln Gly Thr Lys Leu Glu Ile Lys Gly Gly Gly Gly Ser Asn Trp
                245                 250                 255

Val Asn Val Ile Ser Asp Leu Lys Lys Ile Glu Asp Leu Ile Gln Ser
            260                 265                 270

Met His Ile Asp Ala Thr Leu Tyr Thr Glu Ser Asp Val His Pro Ser
        275                 280                 285

Cys Lys Val Thr Ala Met Lys Cys Phe Leu Leu Glu Leu Gln Val Ile

290                 295                 300

Ser Leu Glu Ser Gly Asp Ala Ser Ile His Asp Thr Val Glu Asn Leu
305                 310                 315                 320

Ile Ile Leu Ala Asn Asn Ser Leu Ser Ser Asn Gly Asn Val Thr Glu
                325                 330                 335

Ser Gly Cys Lys Glu Cys Glu Glu Leu Glu Lys Lys Asn Ile Lys Glu
            340                 345                 350

Phe Leu Gln Ser Phe Val His Ile Val Gln Met Phe Ile Asn Thr Ser
        355                 360                 365

<210>  33
<211>  237
<212>  PRT
<220>
<223>
<400>  33

Gln Leu Leu Glu Ser Gly Gly Gly Leu Val Gln Pro Gly Gly Ser Leu
1               5                   10                  15

Arg Leu Ser Cys Ala Ala Ser Gly Tyr Arg Ile Ser Ser Asp Asp Met
            20                  25                  30

Ser Trp Val Arg Gln Ala Pro Gly Lys Gly Leu Glu Trp Val Ser Thr
        35                  40                  45

Ile Tyr Glu Thr Asp Gly Ser Thr Tyr Tyr Ala Asp Ser Val Lys Gly
    50                  55                  60

Arg Phe Thr Ile Ser Arg Asp Asn Ser Lys Asn Thr Leu Tyr Leu Gln
65                  70                  75                  80

Met Asn Ser Leu Arg Ala Glu Asp Thr Ala Val Tyr Tyr Cys Ala Arg
                85                  90                  95

Ala Asn Phe Tyr Ser Glu Gln Pro Phe Gln Phe Trp Gly Gln Gly Thr
            100                 105                 110

Leu Val Thr Val Ser Ser Gly Gly Gly Gly Ser Asn Trp Val Asn Val
        115                 120                 125

Ile Ser Asp Leu Lys Lys Ile Glu Asp Leu Ile Gln Ser Met His Ile
    130                 135                 140

Asp Ala Thr Leu Tyr Thr Glu Ser Asp Val His Pro Ser Cys Lys Val

145                 150                 155                 160

Thr Ala Met Lys Cys Phe Leu Leu Glu Leu Gln Val Ile Ser Leu Glu
                165                 170                 175

Ser Gly Asp Ala Ser Ile His Asp Thr Val Glu Asn Leu Ile Ile Leu
            180                 185                 190

Ala Asn Asn Ser Leu Ser Ser Asn Gly Asn Val Thr Glu Ser Gly Cys
        195                 200                 205

Lys Glu Cys Glu Glu Leu Glu Lys Lys Asn Ile Lys Glu Phe Leu Gln
    210                 215                 220

Ser Phe Val His Ile Val Gln Met Phe Ile Asn Thr Ser
225                 230                 235

<210>  34
<211>  227
<212>  PRT
<220>
<223>
<400>  34

Pro Gly Trp Phe Leu Asp Ser Pro Asp Arg Pro Trp Asn Pro Pro Thr
1               5                   10                  15

Phe Ser Pro Ala Leu Leu Val Val Thr Glu Gly Asp Asn Ala Thr Phe
            20                  25                  30

Thr Cys Ser Phe Ser Asn Thr Ser Glu Ser Phe Val Leu Asn Trp Tyr
        35                  40                  45

Arg Met Ser Pro Ser Asn Gln Thr Asp Lys Leu Ala Ala Phe Pro Glu
    50                  55                  60

Asp Arg Ser Gln Pro Gly Gln Asp Cys Arg Phe Arg Val Thr Gln Leu
65                  70                  75                  80

Pro Asn Gly Arg Asp Phe His Met Ser Val Val Arg Ala Arg Arg Asn
                85                  90                  95

Asp Ser Gly Thr Tyr Leu Cys Gly Ala Ile Ser Leu Ala Pro Lys Ala
            100                 105                 110

Gln Ile Lys Glu Ser Leu Arg Ala Glu Leu Arg Val Thr Glu Arg Arg
        115                 120                 125

Ala Glu Val Pro Thr Ala His Pro Ser Pro Ser Pro Arg Pro Ala Gly

130                 135                 140

Gln Phe Gln Thr Leu Val Gly Gly Gly Gly Ser Ile Thr Cys Pro Pro
145                 150                 155                 160

Pro Met Ser Val Glu His Ala Asp Ile Trp Val Lys Ser Tyr Ser Leu
                165                 170                 175

Tyr Ser Arg Glu Arg Tyr Ile Cys Asn Ser Gly Phe Lys Arg Lys Ala
            180                 185                 190

Gly Thr Ser Ser Leu Thr Glu Cys Val Leu Asn Lys Ala Thr Asn Val
        195                 200                 205

Ala His Trp Thr Thr Pro Ser Leu Lys Cys Ile Arg Asp Pro Ala Leu
    210                 215                 220

Val His Gln
225

<210>  35
<211>  1575
<212>  DNA
<220>
<223>
<400>  35
atgccacctc ctcgcctcct cttcttcctc ctcttcctca cccccatgga agtcaggccc      60

gaggaacctc tagtggtgaa ggtggaagag ggagataacg ctgtgctgca gtgcctcaag     120

gggacctcag atggccccac tcagcagctg acctggtctc gggagtcccc gcttaaaccc     180

ttcttaaaac tcagcctggg gctgccaggc ctgggaatcc acatgaggcc cctggccatc     240

tggcttttca tcttcaacgt ctctcaacag atggggggct tctacctgtg ccagccgggg      300

cccccctctg agaaggcctg gcagcctggc tggacagtca atgtggaggg cagcggggag      360

ctgttccggt ggaatgtttc ggacctaggt ggcctgggct gtggcctgaa gaacaggtcc     420

tcagagggcc ccagctcccc ttccgggaag ctcatgagcc ccaagctgta tgtgtgggcc     480

aaagaccgcc ctgagatctg ggagggagag cctccgtgtc tcccaccgag ggacagcctg     540

aaccagagcc tcagccagga cctcaccatg gcccctggct ccacactctg gctgtcctgt     600

ggggtacccc ctgactctgt gtccaggggc cccctctcct ggacccatgt gcaccccaag     660

gggcctaagt cattgctgag cctagagctg aaggacgatc gcccggccag agatatgtgg      720

gtaatggaga cgggtctgtt gttgccccgg gccacagctc aagacgctgg aaagtattat     780

tgtcaccgtg gcaacctgac catgtcattc cacctggaga tcactgctcg gccagtacta     840

tggcactggc tgctgaggac tggtggctgg aagggatccg agcccaaatc ttgtgagaag     900

acccacacat gcccaccgtg cccagcacct gaactcctgg ggggaccgtc agtgttcctc 960

ttccccccaa aacccaagga caccctcatg atctcccgga cccctgaggt cacatgcgtg 1020

gtggtggacg tgagccacga ggaccctgag gtcaagttca actggtacgt ggacggcgtg 1080

gaggtgcata atgccaagac aaagccgcgg gaggagcagt acaacagcac gtaccgtgtg 1140

gtcagcgtcc tcaccgtcct gcaccaggac tggctgaatg gcaaggagta caagtgcaag 1200

gtctccaaca aagccctccc agcccccatc gagaaaacca tctccaaagc caaagggcag 1260

ccccgagaac cacaggtgta caccctgccc ccatcccggg atgagctgac caagaaccag 1320

gtcagcctga cttgcctggt caaaggcttc tatcccagcg acatcgccgt ggagtgggag 1380

agcaatgggc agccggagaa caactacaag accacgcctc ccgtgctgga ctccgacggc 1440

tccttcttcc tctactccaa gctcaccgtg gacaagagca ggtggcagca ggggaacgtg 1500

ttctcatgct ccgtgatgca tgaggctctg cacaaccact acacgcagaa gagcctctcc 1560

ctgtctccgg gtaaa 1575

<210> 36
<211> 1671
<212> DNA
<220>
<223>
<400> 36
atgccacctc ctcgcctcct cttcttcctc ctcttcctca cccccatgga agtcaggccc 60

gaggaacctc tagtggtgaa ggtggaagag ggagataacg ctgtgctgca gtgcctcaag 120

gggacctcag atggccccac tcagcagctg acctggtctc gggagtcccc gcttaaaccc 180

ttcttaaaac tcagcctggg gctgccaggc ctgggaatcc acatgaggcc cctggccatc 240

tggcttttca tcttcaacgt ctctcaacag atggggggct tctacctgtg ccagccgggg 300

cccccctctg agaaggcctg gcagcctggc tggacagtca atgtggaggg cagcggggag 360

ctgttccggt ggaatgtttc ggacctaggt ggcctgggct gtggcctgaa gaacaggtcc 420

tcagagggcc ccagctcccc ttccgggaag ctcatgagcc ccaagctgta tgtgtgggcc 480

aaagaccgcc ctgagatctg ggagggagag cctccgtgtc tcccaccgag ggacagcctg 540

aaccagagcc tcagccagga cctcaccatg gcccctggct ccacactctg gctgtcctgt 600

ggggtacccc ctgactctgt gtccaggggc cccctctcct ggacccatgt gcaccccaag 660

gggcctaagt cattgctgag cctagagctg aaggacgatc gcccggccag agatatgtgg 720

gtaatggaga cgggtctgtt gttgccccgg gccacagctc aagacgctgg aaagtattat 780

tgtcaccgtg gcaacctgac catgtcattc cacctggaga tcactgctcg gccagtacta 840

tggcactggc tgctgaggac tggtggctgg aaggtctcag ctgtgacttt ggcttatctg 900

atcttctgcc tgtgttccct tgtgggcatt cttcatcttc aaagagccct ggtcctgagg 960

aggaaaagaa agcgaatgac tgaccccacc aggagattct tcaaagtgac gcctccccca 1020

ggaagcgggc cccagaacca gtacgggaac gtgctgtctc tccccacacc cacctcaggc 1080

ctcggacgcg cccagcgttg ggccgcaggc ctggggggca ctgccccgtc ttatggaaac 1140

ccgagcagcg acgtccaggc ggatggagcc ttggggtccc ggagcccgcc gggagtgggc 1200

ccagaagaag aggaagggga gggctatgag gaacctgaca gtgaggagga ctccgagttc 1260

tatgagaacg actccaacct tgggcaggac cagctctccc aggatggcag cggctacgag 1320

aaccctgagg atgagcccct gggtcctgag gatgaagact ccttctccaa cgctgagtct 1380

tatgagaacg aggatgaaga gctgacccag ccggtcgcca ggacaatgga cttcctgagc 1440

cctcatgggt cagcctggga ccccagccgg gaagcaacct ccctggggtc ccagtcctat 1500

gaggatatga gaggaatcct gtatgcagcc ccccagctcc gctccattcg gggccagcct 1560

ggacccaatc atgaggaaga tgcagactct tatgagaaca tggataatcc cgatgggcca 1620

gacccagcct ggggaggagg gggccgcatg ggcacctgga gcaccaggtg a 1671

<210> 37
<211> 1773
<212> DNA
<220>
<223>
<400> 37
atggaagatg ccaaaaacat taagaagggc ccagcgccat tctacccact cgaagacggg 60

accgccggcg agcagctgca caaagccatg aagcgctacg ccctggtgcc cggcaccatc 120

gcctttaccg acgcacatat cgaggtggac attacctacg ccgagtactt cgagatgagc 180

gttcggctgg cagaagctat gaagcgctat gggctgaata caaaccatcg gatcgtggtg 240

tgcagcgaga atagcttgca gttcttcatg cccgtgttgg gtgccctgtt catcggtgtg 300

gctgtggccc cagctaacga catctacaac gagcgcgagc tgctgaacag catgggcatc 360

agccagccca ccgtcgtatt cgtgagcaag aaagggctgc aaaagatcct caacgtgcaa 420

aagaagctac cgatcataca aaagatcatc atcatggata gcaagaccga ctaccagggc 480

ttccaaagca tgtacacctt cgtgacttcc catttgccac ccggcttcaa cgagtacgac 540

ttcgtgcccg agagcttcga ccgggacaaa accatcgccc tgatcatgaa cagtagtggc 600

agtaccggat tgcccaaggg cgtagcccta ccgcaccgca ccgcttgtgt ccgattcagt 660

catgcccgcg accccatctt cggcaaccag atcatccccg acaccgctat cctcagcgtg 720

gtgccatttc accacggctt cggcatgttc accacgctgg gctacttgat ctgcggcttt 780

cgggtcgtgc tcatgtaccg cttcgaggag gagctattct tgcgcagctt gcaagactat 840

aagattcaat ctgccctgct ggtgcccaca ctatttagct tcttcgctaa gagcactctc 900

atcgacaagt acgacctaag caacttgcac gagatcgcca gcggcggggc gccgctcagc 960

aaggaggtag gtgaggccgt ggccaaacgc ttccacctac caggcatccg ccagggctac 1020

ggcctgacag aaacaaccag cgccattctg atcacccccg aaggggacga caagcctggc 1080

gcagtaggca aggtggtgcc cttcttcgag gctaaggtgg tggacttgga caccggtaag 1140

acactgggtg tgaaccagcg cggcgagctg tgcgtccgtg gccccatgat catgagcggc 1200

tacgttaaca accccgaggc tacaaacgct ctcatcgaca aggacggctg gctgcacagc 1260

ggcgacatcg cctactggga cgaggacgag cacttcttca tcgtggaccg gctgaagagc 1320

ctgatcaaat acaagggcta ccaggtagcc ccagccgaac tggagagcat cctgctgcaa 1380

caccccaaca tcttcgacgc cggggtcgcc ggcctgcccg acgacgatgc cggcgagctg 1440

cccgccgcag tcgtcgtgct ggaacacggt aaaaccatga ccgagaagga gatcgtggac 1500

tatgtggcca gccaggttac aaccgccaag aagctgcgcg gtggtgttgt gttcgtggac 1560

gaggtgccta aaggactgac cggcaagttg gacgcccgca agatccgcga gattctcatt 1620

aaggccaaga agggcggcaa gatcgccgtg aattctcacg gcttccctcc cgaggtggag 1680

gagcaggccg ccggcaccct gcccatgagc tgcgcccagg agagcggcat ggatagacac 1740

cctgctgctt gcgccagcgc caggatcaac gtc 1773

<210> 38
<211> 470
<212> PRT
<220>
<223>
<400> 38

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ser Lys Tyr
            20                  25                  30

Leu Asn Trp Tyr Gln Gln Lys Pro Asp Gly Thr Val Lys Leu Leu Ile
        35                  40                  45

Tyr His Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asn Thr Leu Pro Tyr
                85                  90                  95

Thr Phe Gly Gly Gly Thr Lys Leu Glu Ile Thr Gly Ser Thr Ser Gly
            100                 105                 110

Ser Gly Lys Pro Gly Ser Gly Glu Gly Ser Thr Lys Gly Glu Val Lys
        115                 120                 125

Leu Gln Glu Ser Gly Pro Gly Leu Val Ala Pro Ser Gln Ser Leu Ser
    130                 135                 140

Val Thr Cys Thr Val Ser Gly Val Ser Leu Pro Asp Tyr Gly Val Ser
145                 150                 155                 160

Trp Ile Arg Gln Pro Pro Arg Lys Gly Leu Glu Trp Leu Gly Val Ile
                165                 170                 175

Trp Gly Ser Glu Thr Thr Tyr Tyr Asn Ser Ala Leu Lys Ser Arg Leu
            180                 185                 190

Thr Ile Ile Lys Asp Asn Ser Lys Ser Gln Val Phe Leu Lys Met Asn
        195                 200                 205

Ser Leu Gln Thr Asp Asp Thr Ala Ile Tyr Tyr Cys Ala Lys His Tyr
    210                 215                 220

Tyr Tyr Gly Gly Ser Tyr Ala Met Asp Tyr Trp Gly Gln Gly Thr Ser
225                 230                 235                 240

Val Thr Val Ser Ser Thr Thr Thr Pro Ala Pro Arg Pro Pro Thr Pro
                245                 250                 255

Ala Pro Thr Ile Ala Ser Gln Pro Leu Ser Leu Arg Pro Glu Ala Cys
            260                 265                 270

Arg Pro Ala Ala Gly Gly Ala Val His Thr Arg Gly Leu Asp Phe Ala
        275                 280                 285

Cys Asp Ile Tyr Ile Trp Ala Pro Leu Ala Gly Thr Cys Gly Val Leu
    290                 295                 300

Leu Leu Ser Leu Val Ile Thr Leu Tyr Cys Lys Arg Gly Arg Lys Lys
305                 310                 315                 320

Leu Leu Tyr Ile Phe Lys Gln Pro Phe Met Arg Pro Val Gln Thr Thr
                325                 330                 335

Gln Glu Glu Asp Gly Cys Ser Cys Arg Phe Pro Glu Glu Glu Glu Gly
            340                 345                 350

Gly Cys Glu Leu Arg Val Lys Phe Ser Arg Ser Ala Asp Ala Pro Ala
        355                 360                 365

Tyr Lys Gln Gly Gln Asn Gln Leu Tyr Asn Glu Leu Asn Leu Gly Arg
    370                 375                 380

Arg Glu Glu Tyr Asp Val Leu Asp Lys Arg Arg Gly Arg Asp Pro Glu
385                 390                 395                 400

Met Gly Gly Lys Pro Arg Arg Lys Asn Pro Gln Glu Gly Leu Tyr Asn
                405                 410                 415

Glu Leu Gln Lys Asp Lys Met Ala Glu Ala Tyr Ser Glu Ile Gly Met
            420                 425                 430

Lys Gly Glu Arg Arg Arg Gly Lys Gly His Asp Gly Leu Tyr Gln Gly
        435                 440                 445

Leu Ser Thr Ala Thr Lys Asp Thr Tyr Asp Ala Leu His Met Gln Ala
    450                 455                 460

Leu Pro Pro Arg Lys Leu
465                 470

<210> 39
<211>  284
<212>  PRT
<213>  Synthetic sequence
<220>
<223>
<400>  39

Pro Gly Trp Phe Leu Asp Ser Pro Asp Arg Pro Trp Asn Pro Pro Thr
1               5                   10                  15

Phe Ser Pro Ala Leu Leu Val Val Thr Glu Gly Asp Asn Ala Thr Phe
            20                  25                  30

Thr Cys Ser Phe Ser Asn Thr Ser Glu Ser Phe Val Leu Asn Trp Tyr
        35                  40                  45

Arg Met Ser Pro Ser Asn Gln Thr Asp Lys Leu Ala Ala Phe Pro Glu
    50                  55                  60

Asp Arg Ser Gln Pro Gly Gln Asp Cys Arg Phe Arg Val Thr Gln Leu
65                  70                  75                  80

Pro Asn Gly Arg Asp Phe His Met Ser Val Val Arg Ala Arg Arg Asn
                85                  90                  95

Asp Ser Gly Thr Tyr Leu Cys Gly Ala Ile Ser Leu Ala Pro Lys Ala

100                 105                 110

Gln Ile Lys Glu Ser Leu Arg Ala Glu Leu Arg Val Thr Glu Arg Arg
        115                 120                 125

Ala Glu Val Pro Thr Ala His Pro Ser Pro Ser Pro Arg Pro Ala Gly
    130                 135                 140

Gln Phe Gln Thr Leu Val Gly Gly Gly Gly Ser His Lys Cys Asp Ile
145                 150                 155                 160

Thr Leu Gln Glu Ile Ile Lys Thr Leu Asn Ser Leu Thr Glu Gln Lys
                165                 170                 175

Thr Leu Cys Thr Glu Leu Thr Val Thr Asp Ile Phe Ala Ala Ser Lys
            180                 185                 190

Asn Thr Thr Glu Lys Glu Thr Phe Cys Arg Ala Ala Thr Val Leu Arg
        195                 200                 205

Gln Phe Tyr Ser His His Glu Lys Asp Thr Arg Cys Leu Gly Ala Thr
    210                 215                 220

Ala Gln Gln Phe His Arg His Lys Gln Leu Ile Arg Leu Leu Lys Arg
225                 230                 235                 240

Leu Asp Arg Asn Leu Trp Gly Leu Ala Gly Leu Asn Ser Cys Pro Val
                245                 250                 255

Lys Glu Ala Asn Gln Ser Thr Leu Glu Asn Phe Leu Glu Arg Leu Lys
            260                 265                 270

Thr Ile Met Arg Glu Lys Tyr Ser Lys Cys Ser Ser
        275                 280
<210>40
<211> 1338
<212> DNA
<213>  Synthetic sequence
<220>
<223>
<400>  40
atggccttac cagtgaccgc cttgctcctg ccgctggcct tgctgctcca cgccgccagg      60
ccggatatcc aactgcaagc tagcggggga ggcttggtgc agcctggggg gtctctgaga     120
ctctcctgtg cagcctctgg acgcaccttc aatgtcgatg ctatggcttg gttccgccag     180
actcgcggga aggagcgtga gtttgtagca gctattagcc ggagtggtgg tagcacgtac     240
tatgcagact ccgtgaaggg ccgattcagc atctccaaag acaacgccaa aaacacgatg     300
tatctgcaaa tgaacagcct caaacctgag gacacggcca tttattactg tgcagctgca     360
atctactggc gtggtagtta ctacactgaa ggcaactatg actactgggg ccagaggacc     420
caggtcaccg tgagctcagg cggcggcgga tctatcacgt gccctccccc catgtccgtg     480
gaacacgcag acatctgggt caagagctac agcttgtact ccagggagcg gtacatttgt     540
aactctggtt tcaagcgtaa agccggcacg tccagcctga cggagtgcgt gttgaacaag     600
gccacgaatg tcgcccactg gacaaccccc agtctcaaat gcattagaga ccctgccctg     660

gttcaccaaa ccacgacgcc agcgccgcga ccaccaacac cggcgcccac catcgcgtcg 720
cagcccctgt ccctgcgccc agaggcgtgc cggccagcgg cggggggcgc agtgcacacg 780
agggggctgg acttcgcctg tgacatctac atctgggcgc ccttggccgg gacttgtggg 840
gtccttctcc tgtcactggt tatcaccctt tactgcaaac ggggcagaaa gaaactcctg 900
tatatattca aacaaccatt tatgagacca gtacaaacta ctcaagagga agatggctgt 960
agctgccgat ttccagaaga agaagaagga ggatgtgaac tgagagtgaa gttcagcagg 1020
agcgcagacg cccccgcgta caagcagggc cagaaccagc tctataacga actcaatcta 1080
ggacgaagag aggagtacga tgttttggac aagagacgtg gccgggaccc tgagatgggg 1140
ggaaagccga gaaggaagaa ccctcaggaa ggcctgtaca atgaactgca gaaagataag 1200
atggcggagg cctacagtga gattgggatg aaaggcgagc ggcggagggg caaggggcac 1260
gatggccttt accagggtct cagtacagcc accaaggaca cctacgacgc ccttcacatg 1320
caggccctgc cccctcgc 1338

<210>41
<211>1320
<212> DNA
<213> Synthetic sequence
<220>
<223>
<400> 41

atgtacagga tgcaactcct gtcttgcatt gcactaagtc ttgcacttgt cacgaattct 60
caggtgcagc tgttggagtc tgggggaggc ttggtacagc ctggggggtc cctgcgtctc 120
tcctgtgcag cctccggata taggattagc tctgacgata tgagctgggt ccgccaggct 180
ccagggaagg gtctagagtg ggtatcaacc atttatgaga cagacggtag cacatactac 240
gcagactccg tgaagggccg gttcaccatc tcccgtgaca attccaagaa cacgctgtat 300
ctgcaaatga acagcctgcg tgccgaggac accgcggtat attattgcgc gagagctaat 360
ttttatagtg agcagccctt ccagttttgg ggtcagggaa ccctggtcac cgtctcgagc 420
ggcggcggcg gatctatcac gtgccctccc cccatgtccg tggaacacgc agacatctgg 480
gtcaagagct acagcttgta ctccagggag cggtacattt gtaactctgg tttcaagcgt 540
aaagccggca cgtccagcct gacggagtgc gtgttgaaca aggccacgaa tgtcgcccac 600
tggacaaccc ccagtctcaa atgcattaga gaccctgccc tggttcacca aaccacgacg 660
ccagcgccgc gaccaccaac accggcgccc accatcgcgt cgcagcccct gtccctgcgc 720
ccagaggcgt gccggccagc ggcggggggc gcagtgcaca cgagggggct ggacttcgcc 780
tgtgacatct acatctgggc gcccttggcc gggacttgtg gggtccttct cctgtcactg 840
gttatcaccc tttactgcaa acggggcaga aagaaactcc tgtatatatt caaacaacca 900
tttatgagac cagtacaaac tactcaagag gaagatggct gtagctgccg atttccagaa 960
gaagaagaag gaggatgtga actgagagtg aagttcagca ggagcgcaga cgcccccgcg 1020
tacaagcagg gccagaacca gctctataac gaactcaatc taggacgaag agaggagtac 1080
gatgttttgg acaagagacg tggccgggac cctgagatgg ggggaaagcc gagaaggaag 1140
aaccctcagg aaggcctgta caatgaactg cagaaagata agatggcgga ggcctacagt 1200
gagattggga tgaaaggcga gcggcggagg ggcaaggggc acgatggcct ttaccagggt 1260
ctcagtacag ccaccaagga cacctacgac gcccttcaca tgcaggccct gccccctcgc 1320

<210>42
<211> 1317
<212> DNA
<213> Synthetic sequence
<220>
<223>
<400> 42

atgtacagga tgcaactcct gtcttgcatt gcactaagtc ttgcacttgt cacgaattct 60
caggtgcagc tgttggagtc tgggggaggc ttggtacagc ctggggggtc cctgcgtctc 120
tcctgtgcag cctccggata taggattagc tctgacgata tgagctgggt ccgccaggct 180
ccagggaagg gtctagagtg ggtatcaacc atttatgaga cagacggtag cacatactac 240
gcagactccg tgaagggccg gttcaccatc tcccgtgaca attccaagaa cacgctgtat 300
ctgcaaatga acagcctgcg tgccgaggac accgcggtat attattgcgc gagagctaat 360
ttttatagtg agcagccctt ccagttttgg ggtcagggaa ccctggtcac cgtctcgagc 420
ggcggcggcg gatctatcac gtgccctccc cccatgtccg tggaacacgc agacatctgg 480
gtcaagagct acagcttgta ctccagggag cggtacattt gtaactctgg tttcaagcgt 540
aaagccggca cgtccagcct gacggagtgc gtgttgaaca aggccacgaa tgtcgcccac 600
tggacaaccc ccagtctcaa atgcattaga gaccctgccc tggttcacca aaccacgacg 660

ccagcgccgc gaccaccaac accggcgccc accatcgcgt cgcagcccct gtccctgcgc 720
ccagaggcgt gccggccagc ggcggggggc gcagtgcaca cgagggggct ggacttcgcc 780
tgtgacatct acatctgggc gcccttggcc gggacttgtg gggtccttct cctgtcactg 840
gttatcaccc tttactgcag gagtaagagg agcaggctcc tgcacagtga ctacatgaac 900
atgactcccc gccgccccgg gcccacccgc aagcattacc agccctatgc ccccccacgc 960
gacttcgcag cctatcgctc cagagtgaag ttcagcagga gcgcagacgc ccccgcgtac 1020
aagcagggcc agaaccagct ctataacgaa ctcaatctag gacgaagaga ggagtacgat 1080
gttttggaca agagacgtgg ccgggaccct gagatggggg gaaagccgag aaggaagaac 1140
cctcaggaag gcctgtacaa tgaactgcag aaagataaga tggcggaggc ctacagtgag 1200
attgggatga aaggcgagcg gcggaggggc aaggggcacg atggccttta ccagggtctc 1260
agtacagcca ccaaggacac ctacgacgcc cttcacatgc aggccctgcc ccctcgc 1317

<210>43
<211> 1650
<212> DNA
<213> Synthetic sequence
<220>
<223>
<400> 43
atgtacagga tgcaactcct gtcttgcatt gcactaagtc ttgcacttgt cacgaattct 60
caggtgcagc tgttggagtc tgggggaggc ttggtacagc ctggggggtc cctgcgtctc 120
tcctgtgcag cctccggata taggattagc tctgacgata tgagctgggt ccgccaggct 180
ccagggaagg gtctagagtg ggtatcaacc atttatgaga cagacggtag cacatactac 240
gcagactccg tgaagggccg gttcaccatc tcccgtgaca attccaagaa cacgctgtat 300
ctgcaaatga acagcctgcg tgccgaggac accgcggtat attattgcgc gagagctaat 360
ttttatagtg agcagccctt ccagttttgg ggtcagggaa ccctggtcac cgtctcgagc 420
ggcggcggcg gatctatgaa ggtcttgcag gagcccacct gcgtctccga ctacatgagc 480
atctctactt gcgagtggaa gatgaatggt cccaccaatt gcagcaccga gctccgcctg 540
ttgtaccagc tggtttttct gctctccgaa gcccacacgt gtatccctga gaacaacgga 600
ggcgcggggt gcgtgtgcca cctgctcatg gatgacgtgg tcagtgcgga taactataca 660
ctggacctgt gggctgggca gcagctgctg tggaagggct ccttcaagcc cagcgagcat 720
gtgaaaccca gggccccagg aaacctgaca gttcacacca atgtctccga cactctgctg 780
ctgacctgga gcaacccgta tccccctgac aattacctgt ataatcatct cacctatgca 840
gtcaacattt ggagtgaaaa cgacccggca gatttcagaa tctataacgt gacctaccta 900
gaaccctccc tccgcatcgc agccagcacc ctgaagtctg ggatttccta cagggcacgg 960
gtgagggcct gggctcagtg caccacgacg ccagcgccgc gaccaccaac accggcgccc 1020
accatcgcgt cgcagcccct gtccctgcgc ccagaggcgt gccggccagc ggcggggggc 1080
gcagtgcaca cgagggggct ggacttcgcc tgtgacatct acatctgggc gcccttggcc 1140
gggacttgtg gggtccttct cctgtcactg gttatcaccc tttactgcaa acggggcaga 1200
aagaaactcc tgtatatatt caaacaacca tttatgagac cagtacaaac tactcaagag 1260
gaagatggct gtagctgccg atttccagaa gaagaagaag gaggatgtga actgagagtg 1320
aagttcagca ggagcgcaga cgcccccgcg tacaagcagg gccagaacca gctctataac 1380
gaactcaatc taggacgaag agaggagtac gatgttttgg acaagagacg tggccgggac 1440
cctgagatgg ggggaaagcc gagaaggaag aaccctcagg aaggcctgta caatgaactg 1500
cagaaagata agatggcgga ggcctacagt gagattggga tgaaaggcga gcggcggagg 1560
ggcaaggggc acgatggcct ttaccagggt ctcagtacag ccaccaagga cacctacgac 1620
gcccttcaca tgcaggccct gccccctcgc 1650
<210>44
<211> 1194
<212> DNA
<213> Synthetic sequence
<220>
<223>
<400> 44
atgtacagga tgcaactcct gtcttgcatt gcactaagtc ttgcacttgt cacgaattct 60
caggtgcagc tgttggagtc tgggggaggc ttggtacagc ctggggggtc cctgcgtctc 120
tcctgtgcag cctccggata taggattagc tctgacgata tgagctgggt ccgccaggct 180
ccagggaagg gtctagagtg ggtatcaacc atttatgaga cagacggtag cacatactac 240
gcagactccg tgaagggccg gttcaccatc tcccgtgaca attccaagaa cacgctgtat 300
ctgcaaatga acagcctgcg tgccgaggac accgcggtat attattgcgc gagagctaat 360
ttttatagtg agcagccctt ccagttttgg ggtcagggaa ccctggtcac cgtctcgagc 420
ggcggcggcg gatctatcac gtgccctccc cccatgtccg tggaacacgc agacatctgg 480
gtcaagagct acagcttgta ctccagggag cggtacattt gtaactctgg tttcaagcgt 540

aaagccggca cgtccagcct gacggagtgc gtgttgaaca aggccacgaa tgtcgcccac 600
tggacaaccc ccagtctcaa atgcattaga gaccctgccc tggttcacca attttgggtg 660
ctggtggtgg ttggtggagt cctggcttgc tatagcttgc tagtaacagt ggcctttatt 720
attttctggg tgaaacgggg cagaaagaaa ctcctgtata tattcaaaca accatttatg 780
agaccagtac aaactactca agaggaagat ggctgtagct gccgatttcc agaagaagaa 840
gaaggaggat gtgaactgag agtgaagttc agcaggagcg cagacgcccc cgcgtacaag 900
cagggccaga accagctcta taacgaactc aatctaggac gaagagagga gtacgatgtt 960
ttggacaaga gacgtggccg ggaccctgag atggggggaa agccgagaag gaagaaccct 1020
caggaaggcc tgtacaatga actgcagaaa gataagatgg cggaggccta cagtgagatt 1080
gggatgaaag gcgagcggcg gaggggcaag gggcacgatg gcctttacca gggtctcagt 1140
acagccacca aggacaccta cgacgccctt cacatgcagg ccctgccccc tcgc 1194

<210>45
<211>1410
<212> DNA
<213> Synthetic sequence
<220>
<223>
<400> 45
atgtacagga tgcaactcct gtcttgcatt gcactaagtc ttgcacttgt cacgaattct 60
ccaggatggt tcttagactc cccagacagg ccctggaacc cccccacctt ctccccagcc 120
ctgctcgtgg tgaccgaagg ggacaacgcc accttcacct gcagcttctc caacacatcg 180
gagagcttcg tgctaaactg gtaccgcatg agccccagca accagacgga caagctggcc 240
gccttccccg aggaccgcag ccagcccggc caggactgcc gcttccgtgt cacacaactg 300
cccaacgggc gtgacttcca catgagcgtg gtcagggccc ggcgcaatga cagcggcacc 360
tacctctgtg gggccatctc cctggccccc aaggcgcaga tcaaagagag cctgcgggca 420
gagctcaggg tgacagagag aagggcagaa gtgcccacag cccaccccag cccctcaccc 480
aggccagccg gccagttcca aaccctggtg ggcggcggcg gatctatcac gtgccctccc 540
cccatgtccg tggaacacgc agacatctgg gtcaagagct acagcttgta ctccagggag 600
cggtacattt gtaactctgg tttcaagcgt aaagccggca cgtccagcct gacggagtgc 660
gtgttgaaca aggccacgaa tgtcgcccac tggacaaccc ccagtctcaa atgcattaga 720
gaccctgccc tggttcacca aaccacgacg ccagcgccgc gaccaccaac accggcgccc 780
accatcgcgt cgcagcccct gtccctgcgc ccagaggcgt gccggccagc ggcggggggc 840
gcagtgcaca cgagggggct ggacttcgcc tgtgacatct acatctgggc gcccttggcc 900
gggacttgtg gggtccttct cctgtcactg gttatcaccc tttactgcaa acggggcaga 960
aagaaactcc tgtatatatt caaacaacca tttatgagac cagtacaaac tactcaagag 1020
gaagatggct gtagctgccg atttccagaa gaagaagaag gaggatgtga actgagagtg 1080
aagttcagca ggagcgcaga cgcccccgcg tacaagcagg gccagaacca gctctataac 1140
gaactcaatc taggacgaag agaggagtac gatgttttgg acaagagacg tggccgggac 1200
cctgagatgg ggggaaagcc gagaaggaag aaccctcagg aaggcctgta caatgaactg 1260
cagaaagata agatggcgga ggcctacagt gagattggga tgaaaggcga gcggcggagg 1320
ggcaaggggc acgatggcct ttaccagggt ctcagtacag ccaccaagga cacctacgac 1380
gcccttcaca tgcaggccct gccccctcgc 1410

<210>46
<211> 1446
<212> DNA
<213> Synthetic sequence
<220>
<223>
<400> 46
atgtacagga tgcaactcct gtcttgcatt gcactaagtc ttgcacttgt cacgaattct 60
caggtgcagc tgttggagtc tgggggaggc ttggtacagc ctggggggtc cctgcgtctc 120
tcctgtgcag cctccggata taggattagc tctgacgata tgagctgggt ccgccaggct 180
ccagggaagg gtctagagtg ggtatcaacc atttatgaga cagacggtag cacatactac 240
gcagactccg tgaagggccg gttcaccatc tcccgtgaca attccaagaa cacgctgtat 300
ctgcaaatga acagcctgcg tgccgaggac accgcggtat attattgcgc gagagctaat 360
ttttatagtg agcagccctt ccagttttgg ggtcagggaa ccctggtcac cgtctcgagc 420
ggcggcggcg gatctaactg ggtgaacgtc atctccgacc tcaagaagat cgaggacctg 480
atccagagca tgcacatcga cgccaccctg tataccgaga gcgacgtgca cccctcctgt 540
aaagtgaccg ccatgaagtg cttcctgctg gagctgcagg tgatcagcct ggagagcggc 600
gacgccagca tccatgacac cgtggagaac ctgatcatcc tggccaataa cagcctgagc 660

tccaacggca acgtgaccga gagcggctgc aaggaatgcg aggagctgga gaagaagaac 720
attaaggagt tcctgcagag cttcgtccac atcgtgcaga tgttcattaa cacctccacc 780
acgacgccag cgccgcgacc accaacaccg gcgcccacca tcgcgtcgca gcccctgtcc 840
ctgcgcccag aggcgtgccg gccagcggcg gggggcgcag tgcacacgag ggggctggac 900
ttcgcctgtg acatctacat ctgggcgccc ttggccggga cttgtggggt ccttctcctg 960
tcactggtta tcacccttta ctgcaaacgg ggcagaaaga aactcctgta tatattcaaa 1020
caaccattta tgagaccagt acaaactact caagaggaag atggctgtag ctgccgattt 1080
ccagaagaag aagaaggagg atgtgaactg agagtgaagt tcagcaggag cgcagacgcc 1140
cccgcgtaca agcagggcca gaaccagctc tataacgaac tcaatctagg acgaagagag 1200
gagtacgatg ttttggacaa gagacgtggc cgggaccctg agatgggggg aaagccgaga 1260
aggaagaacc ctcaggaagg cctgtacaat gaactgcaga aagataagat ggcggaggcc 1320
tacagtgaga ttgggatgaa aggcgagcgg cggaggggca aggggcacga tggcctttac 1380
cagggtctca gtacagccac caaggacacc tacgacgccc ttcacatgca ggccctgccc 1440
cctcgc 1446

<210>47
<211>780
<212> DNA
<213> Synthetic sequence
<220>
<223>
<400> 47
ggaagcggcg ccaccaactt ctccctgctg aagcaagctg gcgacgtgga agagaacccc 60
ggccccgatg tgcagctgca agctagcgga ggcggcctgg tgcaagctgg agactccctg 120
aggctgagct gcgccgctag cggaaggacc ttcagcacct acaacatggg ctggttcagg 180
caggcccccg gaaaggacag ggagttcgtg gccgccatta tgtggtccgg cggcagcacc 240
tactatgccg acagcgtgaa gggcaggttc acaatctcca aggacaacgc caagaacacc 300
gtgtacctgc agatgaactc cctgaagccc gaggatacag ccgtgtactt ctgctggagc 360
accgatgatt acggagtcga cagctggggc cagggaaccc aggtgaccgt gagctccggc 420
ggaggaggct ccaactgggt gaacgtcatc tccgacctca agaagatcga ggacctgatc 480
cagagcatgc acatcgacgc caccctgtat accgagagcg acgtgcaccc ctcctgtaaa 540
gtgaccgcca tgaagtgctt cctgctggag ctgcaggtga tcagcctgga gagcggcgac 600
gccagcatcc atgacaccgt ggagaacctg atcatcctgg ccaataacag cctgagctcc 660
aacggcaacg tgaccgagag cggctgcaag gaatgcgagg agctggagaa gaagaacatt 720
aaggagttcc tgcagagctt cgtccacatc gtgcagatgt tcattaacac ctcctaatga 780

<210>48
<211>1158
<212> DNA
<213> Synthetic sequence
<220>
<223>
<400> 48
ggaagcggcg ccaccaactt ctccctgctg aagcaagctg gcgacgtgga agagaacccc 60
ggccccgagg tgcagctggt ggagagcgga ggcggactgg tgcagcctgg cagatccctc 120
agactgtcct gtgccgcctc cggcttcacc ttcaacgact acgccatgca ctgggtgagg 180
caggcccccg gaaaaggact ggagtgggtg tccaccatca gctggaactc cggatccatc 240
ggctacgccg actccgtgaa gggcaggttc accatctcca gggataacgc caagaagtcc 300
ctgtacctgc agatgaacag cctgagagcc gaggacaccg ccctgtacta ctgcgccaag 360
gacatccagt acggaaacta ctactacggc atggacgtgt ggggacaagg caccaccgtg 420
acagtgagca gcggcggagg aggatccgga ggaggcggat ccggaggagg aggatccgag 480
atcgtgctga cccagagccc tgctaccctg agcctgagcc ctggcgagag agccaccctg 540
tcctgcaggg ctagccagtc cgtgtccagc tacctggcct ggtaccagca gaagcccggc 600
caagccccta ggctgctgat ctacgacgcc agcaataggg ccacaggcat ccccgccaga 660
tttagcggca gcggctccgg aaccgacttc acactgacca tcagctccct ggagcccgaa 720
gacttcgccg tgtattactg ccagcagagg agcaactggc ccatcacctt cggacagggc 780
accagactgg agatcggcgg aggaggctcc aactgggtga acgtcatctc cgacctcaag 840
aagatcgagg acctgatcca gagcatgcac atcgacgcca ccctgtatac cgagagcgac 900
gtgcacccct cctgtaaagt gaccgccatg aagtgcttcc tgctggagct gcaggtgatc 960
agcctggaga gcggcgacgc cagcatccat gacaccgtgg agaacctgat catcctggcc 1020
aataacagcc tgagctccaa cggcaacgtg accgagagcg gctgcaagga atgcgaggag 1080
ctggagaaga agaacattaa ggagttcctg cagagcttcg tccacatcgt gcagatgttc 1140
attaacacct cctaatga 1158

<210>49
<211> 1176
<212> DNA
<213>  Synthetic sequence
<220>
<223>
<400>  49
ggaagcggcg ccaccaactt ctccctgctg aagcaagctg gcgacgtgga agagaacccc      60
ggcccccagg tgcagctgca acagagcggc cctggactgg tgaagcccag ccagacactg     120
agcctcacct gcgccattag cggcgacagc gtgagctcca attccgccgc ctggaattgg     180
atcagacaga gcacaagcag gggcctggag tggctgggca ggacctacta caggagcaag     240
tggtacaacg actacgccgt gtccgtgaag agcaggatca caatcaaccc cgacaccagc     300
aagaaccagt tcagcctgca gctcaacagc gtcacccccg aagacaccgc cgtgtactac     360
tgcgccagag aggtgaccgg cgacctggag gacgccttcg acatctgggg ccaaggcacc     420
atggtgaccg tgagctccgg ctccacaagc ggaagcggca aacctggctc cggcgagggt     480
tctaccaaag gcgacatcca gatgacccag agcccttcct ccctgagcgc cagcgtgggc     540
gatagagtga caattacctg cagggccagc cagaccatct ggagctacct gaactggtac     600
cagcagaggc ccggaaaggc ccccaacctg ctgatctacg ccgcttccag cctgcagagc     660
ggcgtgccta gcaggttctc cggcagaggc agcggcaccg atttcaccct gacaatcagc     720
agcctgcagg ccgaagattt tgccacctac tactgccagc agagctacag catcccccag     780
accttcggcc agggcaccaa actggagatc aagggcggag gaggctccaa ctgggtgaac     840
gtcatctccg acctcaagaa gatcgaggac ctgatccaga gcatgcacat cgacgccacc     900
ctgtataccg agagcgacgt gcacccctcc tgtaaagtga ccgccatgaa gtgcttcctg     960
ctggagctgc aggtgatcag cctggagagc ggcgacgcca gcatccatga caccgtggag    1020
aacctgatca tcctggccaa taacagcctg agctccaacg gcaacgtgac cgagagcggc    1080
tgcaaggaat gcgaggagct ggagaagaag aacattaagg agttcctgca gagcttcgtc    1140
cacatcgtgc agatgttcat taacacctcc taatga                              1176

<210>50
<211> 924
<212> DNA
<213>  Synthetic sequence
<220>
<223>
<400> 50
ggaagcggcg ccaccaactt ctccctgctg aagcaagctg gcgacgtgga agagaacccc      60
ggccccccag gatggttctt agactcccca gacaggccct ggaacccccc caccttctcc     120
ccagccctgc tcgtggtgac cgaaggggac aacgccacct tcacctgcag cttctccaac     180
acatcggaga gcttcgtgct aaactggtac cgcatgagcc ccagcaacca gacggacaag     240
ctggccgcct tccccgagga ccgcagccag cccggccagg actgccgctt ccgtgtcaca     300
caactgccca acgggcgtga cttccacatg agcgtggtca gggcccggcg caatgacagc     360
ggcacctacc tctgtggggc catctccctg gcccccaagg cgcagatcaa agagagcctg     420
cgggcagagc tcagggtgac agagagaagg gcagaagtgc ccacagccca ccccagcccc     480
tcacccaggc cagccggcca gttccaaacc ctggtgggcg gaggaggctc ccacaagtgc     540
gatatcacct tacaggagat catcaaaact ttgaacagcc tcacagagca gaagactctg     600
tgcaccgagt tgaccgtaac agacatcttt gctgcctcca agaacacaac tgagaaggaa     660
accttctgca gggctgcgac tgtgctccgg cagttctaca gccaccatga gaaggacact     720
cgctgcctgg gtgcgactgc acagcagttc cacaggcaca agcagctgat ccgactcctg     780
aaacggctcg acaggaacct ctggggcctg gcgggcttga attcctgtcc tgtgaaggaa     840
gccaaccaga gtacgttgga aaacttcttg gaaaggctaa agacgatcat gagagagaaa     900
tattcaaagt gttcgagcta atga                                            924
<210>51
<211> 789
<212> DNA
<213>  Synthetic sequence
<220>
<223>
<400>  51
ggaagcggcg ccaccaactt ctccctgctg aagcaagctg gcgacgtgga agagaacccc      60
ggcccccagg tgcagctgtt ggagtctggg ggaggcttgg tacagcctgg ggggtccctg     120
cgtctctcct gtgcagcctc cggatatagg attagctctg acgatatgag ctgggtccgc     180
caggctccag ggaagggtct agagtgggta tcaaccattt atgagacaga cggtagcaca     240

tactacgcag actccgtgaa gggccggttc accatctccc gtgacaattc caagaacacg 300
ctgtatctgc aaatgaacag cctgcgtgcc gaggacaccg cggtatatta ttgcgcgaga 360
gctaattttt atagtgagca gcccttccag ttttggggtc agggaaccct ggtcaccgtc 420
tcgagcggcg gaggaggctc caactgggtg aacgtcatct ccgacctcaa gaagatcgag 480
gacctgatcc agagcatgca catcgacgcc accctgtata ccgagagcga cgtgcacccc 540
tcctgtaaag tgaccgccat gaagtgcttc ctgctggagc tgcaggtgat cagcctggag 600
agcggcgacg ccagcatcca tgacaccgtg gagaacctga tcatcctggc caataacagc 660
ctgagctcca acggcaacgt gaccgagagc ggctgcaagg aatgcgagga gctggagaag 720
aagaacatta aggagttcct gcagagcttc gtccacatcg tgcagatgtt cattaacacc 780
tcctaatga 789

<210>52
<211> 414
<212> DNA
<213> Synthetic sequence
<220>
<223>
<400>52
ggaagcggcg ccaccaactt ctccctgctg aagcaagctg gcgacgtgga agagaacccc 60
ggccccaact gggtgaacgt catctccgac ctcaagaaga tcgaggacct gatccagagc 120
atgcacatcg acgccaccct gtataccgag agcgacgtgc acccctcctg taaagtgacc 180
gccatgaagt gcttcctgct ggagctgcag gtgatcagcc tggagagcgg cgacgccagc 240
atccatgaca ccgtggagaa cctgatcatc ctggccaata acagcctgag ctccaacggc 300
aacgtgaccg agagcggctg caaggaatgc gaggagctgg agaagaagaa cattaaggag 360
ttcctgcaga gcttcgtcca catcgtgcag atgttcatta acacctccta atga 414

<210>53
<211>288
<212> DNA
<213> Synthetic sequence
<220>
<223>
<400> 53

ggaagcggcg ccaccaactt ctccctgctg aagcaagctg gcgacgtgga agagaacccc 60
ggccccatca cgtgccctcc ccccatgtcc gtggaacacg cagacatctg ggtcaagagc 120
tacagcttgt actccaggga gcggtacatt tgtaactctg gtttcaagcg taaagccggc 180
acgtccagcc tgacggagtg cgtgttgaac aaggccacga atgtcgccca ctggacaacc 240
cccagtctca aatgcattag agaccctgcc ctggttcacc aataatga 288

<210>54
<211> 753
<212> DNA
<213> Synthetic sequence
<220>
<223>
<400> 54
ggaagcggcg ccaccaactt ctccctgctg aagcaagctg gcgacgtgga agagaacccc 60
ggccccccag gatggttctt agactcccca gacaggccct ggaacccccc caccttctcc 120
ccagccctgc tcgtggtgac cgaaggggac aacgccacct tcacctgcag cttctccaac 180
acatcggaga gcttcgtgct aaactggtac cgcatgagcc ccagcaacca gacggacaag 240
ctggccgcct tccccgagga ccgcagccag cccggccagg actgccgctt ccgtgtcaca 300
caactgccca acgggcgtga cttccacatg agcgtggtca gggcccggcg caatgacagc 360
ggcacctacc tctgtggggc catctccctg gcccccaagg cgcagatcaa agagagcctg 420
cgggcagagc tcagggtgac agagagaagg gcagaagtgc ccacagccca ccccagcccc 480
tcacccaggc cagccggcca gttccaaacc ctggtgggcg gaggaggctc catcacgtgc 540
cctcccccca tgtccgtgga acacgcagac atctgggtca agagctacag cttgtactcc 600
agggagcggt acatttgtaa ctctggtttc aagcgtaaag ccggcacgtc cagcctgacg 660
gagtgcgtgt tgaacaaggc cacgaatgtc gcccactgga caacccccag tctcaaatgc 720
attagagacc ctgccctggt tcaccaataa tga 753

## Description

### Field of the invention

The present invention relates to the field of biotechnology, and in particular to a multi-targeting chimeric antigen receptor.

### Background of the invention

The chimeric antigen receptor (CAR) is usually a transmembrane fusion protein consisting mainly of an antigen binding domain, a transmembrane domain and an intracellular signaling domain (patent CN 105392888 A). Immune cells (eg, T cells or NK cells) will be conferred antigen specificity upon expression of a chimeric antigen receptor.

The antigen-binding domain of the CAR molecule determines the specific targeting of immune cells. The most A typical CAR often uses a single-chain variable fragment (scFv) as its antigen-binding domain consisting of the variable region of the heavy chain of the antibody and the light chain variable region joined by a linker. Because of its simple structure, there is only one peptide chain, which is suitable for integration into the CAR molecule as part of the fusion protein. When the tumor antigen itself is a receptor, the antigen binding domain of the CAR can also be replaced by its ligand specific for the receptor.

The second unit structure of the CAR molecule is the transmembrane (TM), which allows CAR molecules to be expressed on the cell surface across the membrane. Usually CAR molecules are armed on T cells for tumor treatment, therefore, many transmembrane regions of T cell-expressing proteins, such as CD3ζ, CD4, CD8 or CD28 can be used as CAR transmembrane domain. It has been reported in the literature that the expression level of CAR with the transmembrane region of CD28 is higher than that with the transmembrane region of CD3ζ. In addition, in some CAR molecular structures, a spacer/hinge region can be inserted between the antigen domain and the transmembrane domain, which can reduce the steric hindrance of the antigen structure and the targeted antigen. Fregmental peptides of IgG1, IgG4, IgD and CD8 are often used for such purpose.

The intracellular signaling region is the most important part of the CAR molecule. It is responsible for transmitting intracellular signals and the activation and proliferation of immune cells. The early design of the intracellular signaling domain used one CD3ζ stimulation signal only, also known as the first generation CAR. The same as the CD3 ζ molecule, there are only 3 ITAMs without the second signal of costimulation. Therefore, the first generation CAR has only weak anti-tumor effect since T cell is difficult to proliferate due to lack of the second signal when T cells bind to the antigen of the tumor, so it is less effective in clinical applications. On this basis, CAR was modified to integrate the active domain of the CD28 molecule that transmits the second signal in the CAR, hence called as the second generation CAR. Addition of co-stimulatory signals to CAR-T cells can enhance the survival of CAR-T cells in vivo and enable cells to kill their tumor cells while maintain proliferation, improving the anti-tumor capacity of CAR-T cells. Therefore, the second-generation CAR showed good anti-tumor effects in clinical applications. For example, Alvavez-Vallina and Hawkins et al. inserted the costimulatory molecule CD28 signal fragment into the CAR molecule, demonstrating that T cells can be stimulated by the corresponding antigen (Alvarez-Vallina L, Hawkins R E., 1996). Therefore, the second generation CAR showed a good anti-tumor effect in clinical applications. However, in the absence of exogenous co-stimulatory molecules, all second generations of CAR could not produce sufficient IL-2 to promote T cell proliferation. However, if transgenic B7 costimulatory molecules were given, these transgenic CAR-T cells could produce IL- 2 to promote proliferation themselves. Based on this, a third-generation CAR was generated, in which the intracellular signal region has another co-stimulatory molecule in addition to the CD28 molecule, usually OX40 or 4-1BB (Till BG, Jensen MC, Wang J, et al., 2012). Compared with the 2nd generation CAR, the 3rd generation CAR has considerable cytotoxicity while its ability to expand in vivo and produce cytokines is better (Pule MA, Straathof KC, Dotti G, et al., 2005). However, recent studies have shown that the functional activity of CAR-T cells is related to the intracellular receptors provided by the target cells in addition to their intracellular signal sequences. Whether the third-generation CARs are superior to the second-generation CARs remains to be clinically validated.

Chimeric antigen receptor T cell therapy has achieved good results in B-cell-derived tumors. The clinical outcome of the treatment with murine antibody based CART19 ("CTL019") has shown promising results in the establishment of complete response for CLL patients and children ALL. Relief (Kalos M, Levine BL, Porter DL, et al., 2011; Porter et al., 2011; Gruppet al., 2013), opened up a new era of adoptive treatment of cancer.

In vitro expansion of chimeric antigen receptor-immune cell therapy T cells or NK cells relies on IL-2 stimulation, so a certain amount of IL-2 must be added to the T and NK cell culture media (Bodnar et al, 2008; Grund et al., 2005). In order to simplify the culture of NK cell and in view of the side effects of toxicity with IL-2 in clinical applications, researchers have genetically engineered IL-2 dependent NK92 cells to stably express IL-2, which makes the addition of IL-2 in the culture medium unnecessary (Shigeki Nagashima, 1998; YK TAM, 1999). IL-15 is functionally similar to IL-2 and shares the same beta and gamma receptor unit. Studies indicate that IL-2 or IL-15 is required for survival and proliferation of NK cells and CD8+ T cells (Boyman et al., 2007). Therefore, the researchers constructed an IL-15-expressing NK cell line and found that IL-15-expressing NK92 cells could greatly reduce the amount of IL-2 required in the medium (JIAN ZHANG, 2004; Jiang et al., 2008; 03152968.2). Although IL-15 and IL-2 share the same βγ receptor units, but each has a specific α receptor, it was found that IL-15Rα-sushi (the sushi domain of IL-15 receptor α) is a super-agonist in IL-15. Agonists can greatly enhance the function of IL-15 (Han et al., 2011; Mortier et al., 2006) (patent: 201280037114.2; 201510358540.1), and the complex of IL-15 and IL-15Rα-sushi can be completely used instead of the role of IL-2 in supporting the growth of T/NK cells (Peter S. Kim 1, 2016; Rosario et al., 2016), NK/CD8+ T cells are activated and their cytotoxicity to kill tumors is increased. Therefore, there is a trend towards using the fusion of IL-15 and IL-15Rα-sushi complex or other functional cytokine and receptor complex in CAR construct to improve the efficacy of cellular immunotherapy.

At the same time, tumours are treated by a single target, tumour cells will escape by down-regulating the expression level of this target, and it will alleviate this problem to treat tumour by two targets (patent: 201710005395.8; 201510733585.2; 201710640609) ;201610353118.1). Aresearcher, for example, can use a linker to connect sequences of two different antibodies or antigen ligands together as an extracellular portion of a chimeric antigen receptor, allowing Car-T cells to recognize two targets, which stops the decline in efficacy due to tumour immune escape (Hegde M, Mukherjee M, Grada Z, et al., 2016; Schneider D, Xiong Y, Wu D, et al., 2017). Furthermore, researchers used an antibody to a biomarker named 5B9 tag as the extracellular domain of CAR-T, and a group of antibodies targeting different tumour targets were fused to the 5B9 tag. Car-T could obtainability to recognize different targets by the binding of different antibodies to respective antigens (Cartellieri M, Feldmann A, Koristka S, et al., 2016).

In addition, if one of the chimeric antigen receptors is made to be an Immune checkpoint-related site, the activity of the immune cells can be greatly enhanced by blocking the negative signal transmitted by the immunological checkpoint. For example, PD-1 (programmed death 1) and its receptor PD-L1, PD-L2 are important regulators of T cell activity (Okazaki and Honjo, 2007). The binding of PD-1 on the surface of T cells to PD-L1/2 on other cells causes inhibition of T cells, which plays an important role in the process of avoiding autoimmune diseases and producing immune tolerance in humans, but some cells infected by pathogenic microorganisms and cancer cells can also evade T cell surveillance by up-regulating their PD-L1/2 on then or PD-1 expression on T-cell, leading to disease (Freeman et al., 2000; Keir et al., 2008; Parry et al., 2005).). Therefore, the researchers explored antibodies against PD-1 or PD-L1 to bind antigen to block this signalling pathway, which can significantly improve T cell activity and enhance the body's resistance to pathogenic microorganisms and cancer (Topalian et Al., 2012; Yanan Guo1, 2016). A combination of CAR-T therapy targeting MSLN with PD1 antibodies has shown that this combination increases the ability of CAR-T cells to kill tumour cells (Cherkassky L, Morello A, Villenavargas J, et al., 2016). Furthermore, researchers have constructed a so-called chimeric antigen receptor switching system by linking immune checkpoint molecules PD1 or CTLA-4 to the intracellular signal region. In this way, the negative signal through immune checkpoints from tumour cells and tumour microenvironment was changed to increase the efficiency of CAR-T cells in treating tumours and reducing the risk of off-target effects (Liu X, Ranganathan R, Jiang S, et al., 2016; Fedorov VD, Themeli M, Sadelain M., 2013). A number of clinical trials have demonstrated that PD-1/PD-L1 antibodies for the treatments of melanoma (Cho et al., 2016; Hamid et al., 2013), multiple myeloma (Badros et al., 2015), leukemia (Pork et al., 2014) have good therapeutic effects (Patent No.: 200380109929.8, 201310258289.2, 201180019629.5). Therefore, modification of the CAR molecule torecognize multiple targetsplays an important role in cellular immunotherapy.

### Invention disclosure

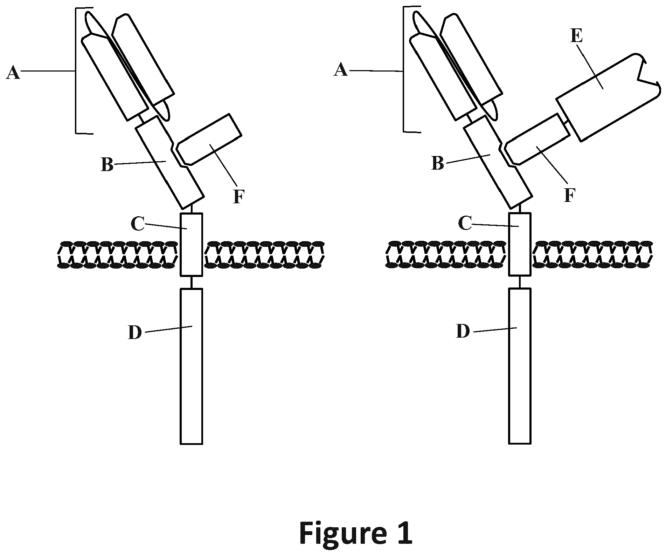

It is an object of the present invention to provide a multi-target chimeric antigen receptor (Fig. 1).

The multi-target chimeric antigen receptor provided by the invention consists of a main peptide chain and a co-peptide chain;
The main peptide chain includes an antigen binding domain A, a co-peptide chain domain B, a transmembrane domain C and an intracellular signalling domain D;
The co-peptide chain comprises a main peptide chain linking domain F;
The antigen binding domain A is a polypeptide having a function of binding antigen;
The co-peptide chain linking domain B and the main peptide chain linking domain F are combined with each other;
The co-peptide chain domain B and the main peptide chain domain F are cytokines and corresponding cytokine receptors or cytokines and corresponding fragments of cytokine receptors that can bind to each other;
The transmembrane domain C is a transmembrane region of any membrane-bound protein or a transmembrane region of a transmembrane protein;
The intracellular signalling domain D comprises a primary signalling region.
Among the above multi-target chimeric antigen receptors,
The co-peptide chain further includes an antigen binding domain E;
The antigen binding domain E is a polypeptide having a function to bind an antigen;
The antigen binding domain E and the antigen binding domain A are the same or different.

Among the above multi-target chimeric antigen receptors,
The intracellular signalling domain D also includes a costimulatory signalling region.

Among the above multi-target chimeric antigen receptors,
The polypeptide having a function to bind to an antigen is an antibody capable of binding an antigen, a ligand capable of binding an antigen, or a receptor capable of binding an antigen.

Among the above multi-target chimeric antigen receptors,
The antibody capable of binding to the antigen is an intact antibody, a Fab of an antibody, an Fc of an antibody, an scFv, a VHH, , a full-length polypeptide or a partial fragment of a VL or VH of an antibody;
The antigen-binding ligand or the antigen-binding receptor is a full-length polypeptide or a partial fragment of the ligand or receptor.

Among the above multi-target chimeric antigen receptors,
The antigens to which the antigen binding domain A and the antigen binding domain E can bind are cell surface antigens or complexes of MHC molecules with polypeptide.

Among the above multi-target chimeric antigen receptors,
The antigen is a cancer associated antigen;
Or in one embodiment, the antigen is brain cancer, bladder cancer, breast cancer, cervical cancer, colorectal cancer, liver cancer, kidney cancer, lymphoma, leukemia, lung cancer, melanoma, metastatic melanoma, mesothelioma , neuroblastoma, ovarian cancer, prostate cancer, pancreatic cancer, renal cancer, skin cancer, thymoma, sarcoma, non-Hodgkin's lymphoma, Hodgkin's lymphoma, uterine cancer-associated antigen, or any combination thereof.

Among the above multi-target chimeric antigen receptors,
The antigen bound by the antigen binding domain A and the antigen binding domain E is as follows: CD123, CD19, CD20, CD22, CD37, ROR1, mesothelin, CD33/IL3Ra, c-Met, BCMA, PSMA, EGFRvIII , GD-2, NY-ESO-1, MAGEA3, β-human chorionic gonadotropin, AFP, RAGE-1, MN-CAIX, human telomerase reverse transcriptase, RU1, RU2 (AS), hsp70 -2, M-CSF, PSA, PAP, LAGE-la, p53, Prostein, PSMA, Her2/neu, telomerase, PCTA-1, MAGE, ELF2M, IGF-I, IGF-II, IGF-I receptor , BCR-ABL, E2A-PRL, H4-RET, 1GH-IGK, MYL-RAR, GP100, Mart1, TSP-180, MAGE-4, MAGE-5, MAGE-6, RAGE, p185erbB2, p180erbB-3, c -met, nm-23H1, TAG-72, CA 19-9, CA 72-4, CAM 17.1, NuMa, K-ras, β-catenin, CDK4, Mum-1, p15, p16, 43-9F, 5T4 , 791Tgp72, β-HCG, BCA225, BTAA, CA 125, CA 15-3\CA27.29\BCAA, CA 195, CA 242, CA-50, WT1, CD68, FGF-5, G250, EpCAM, MA-50, MG7-Ag, MOV 18, NB/70K, RCAS1, SDCCAG16, TA-90, TAAL6, TAG72, TLP, p53, Ras, TPS, Epstein Barr virus antigen EBVA and human papillomavirus (HPV) E6 and E7 orany combination thereof.

Or the antigen bound by the antigen binding domain A and the antigen binding domain E is a complex of MHC and a short peptide of the above antigen, wherein the antigen is the antigen which the binding domain A and the antigen binding domain E can bind.

Among the above multi-target chimeric antigen receptors,
The antigen bound by the antigen binding domain A and the antigen binding domain E is CD19, CD20, BCMA, CD22, CD33/IL3Ra, Her2, PDL1, NY-ESO-1, GP100, Mart1, WT1 or any combination thereof. ;
Or the antigen bound by the antigen binding domain A and the antigen binding domain E is a complex of MHC and the above short peptide of the antigen.

Among the above multi-target chimeric antigen receptors,
The cytokine and corresponding cytokine receptor are cytokines and corresponding cytokine receptors in the gamma chain cytokine family.

Among the above multi-target chimeric antigen receptors,
The cytokine and corresponding cytokine receptor in the yc cytokine family are IL15 and IL15Rα, IL4 and IL4Rα or IL2 and IL2Rα;
Alternatively, the cytokine and the corresponding cytokine receptor in the yc cytokine family are polypeptides in which IL15 and IL15Rα, IL4 and IL4Rα, IL2 or IL2Rα have more than 75% homology.

Among the above multi-target chimeric antigen receptors,
The primary signalling region is the signalling region of CD3-ζ, FcεRIγ, FcRγ, FcRβ, CD3γ, CD3δ, CD3ε, CD5, CD22, CD79a, CD79b, and CD66d, or a combination of above signalling regions;
Or the primary signalling region is a CD3-ζ signalling region with protein sequence of sequence 6 or a polypeptide with greater than 75% homology to the CD3-ζ signalling region.

Among the above multi-target chimeric antigen receptors,
The costimulatory signalling regions are CD27, CD28, 4-1BB (CD137), OX40, CD30, CD40, PD-1, ICOS, lymphocyte function-associated antigen-1, CD2, CD7, LIGHT, NKG2C, B7-H3signalling region or the signalling region of the ligand specifically binding to CD83;or any combination of one or more of the above.

Or the costimulatory signalling region is specifically a combination of one or both of a CD28 signalling region and a 4-1BB signalling region;
Or the costimulatory signalling region is a combination of one or both of polypeptides withgreater than 75% homology to the CD28 signalling region, the 4-1BB signalling region.

Among the above multi-target chimeric antigen receptors,
In the multi-target chimeric antigen receptor, the antigen binding domain A or E is an Anti-CD 19-ScFv, AntiMHC/GP100-VHH, AntiMHC/WT1-VH, AntiCD20-ScFv, AntiCD22-ScFv or PD1 extracellular domain. One or a combination of the two.

The co-peptide linkage domain is IL15Rαsushi, IL4Rα-N-FN3, IL15 or IL4;
The transmembrane domain is a transmembrane region of CD8 or a transmembrane region of CD28;
The intracellular signalling domain is a polypeptide obtained by fusing a CD3ζ signalling region to a 4-1BB signalling region or a CD3ζ signalling region to a CD28 signalling region;
The main peptide chain linking domain is IL15, IL4, IL15Rαsushi or IL4Rα-N-FN3;
Or, in the multi-target chimeric antigen receptor, the antigen binding domain A is antiCD19-ScFv;
The co-peptide chain linking domain is IL15Rasushi;
The transmembrane domain is a transmembrane region of CD8;
The intracellular signalling domain is a polypeptide obtained by fusing a CD3ζ signalling region with a 4-1BB (CD137) signalling region;
The antigen binding domain E is an extracellular region of PD1;
The main peptide chain joining domain is IL15.

The co-peptide chain domain and the main peptide chain domain are most preferably IL15 and IL15Rasushi. When IL15 and IL15Rαsushi are used as the co-peptide chain domain (B) and the main peptide chain domain (F), they stimulate the proliferation of NK cells or T cells, allowing the expression of this multi-targeting chimeric receptor on NK cell or T cell.

The costimulatory signalling region is particularly preferred from the signalling region of one of the following proteins: CD28 and 4-1BB (CD137), or a combination of both.

Among the above multi-target chimeric antigen receptors,
The amino acid sequence of the AntiCD19-ScFv is listed as sequence 1;
The amino acid sequence of the AntiMHC/GP100-VHH is listed as sequence 15;
The amino acid sequence of the AntiMHC/Mart1-VHH is listed as sequence 16;
The amino acid sequence of the AntiCD20-ScFv is listed as sequence 17;
The amino acid sequence of the AntiCD22-ScFv is listed as the sequence 18;
The amino acid sequence of the AntiMHC/WT1-VH is listed as the sequence 19;
The sequence of the extracellular domain of PD1 is listed as sequence 2; the amino acid sequence of the IL15Rαsushi is listed as sequence 4;
The amino acid sequence of the IL4Rα-N-FN3 is listed as the sequence 20;
The amino acid sequence of the transmembrane region of CD8 is listed as sequence 5;
The amino acid sequence of the transmembrane region of CD28 is listed as sequence 22;
The amino acid sequence of the CD3ζ signalling region is listed as sequence 6;
The amino acid sequence of the 4-1BB signalling region is listed as sequence 8;
The amino acid sequence of the CD28 signal is listed as sequence 7;
The amino acid sequence of IL15 is listed as sequence 3;
The amino acid sequence of IL4 is listed as sequence 21.

Among the above multi-target chimeric antigen receptors,
The amino acid sequence of the main peptide chain of the multi-target chimeric antigen receptor is any one of sequence 9, sequence 23, sequence 24, sequence 25, sequence 26, sequence 27, sequence 28 or sequence 29;
The amino acid sequence of the co-peptide chain of the receptor is any one of sequence 3, sequence 4, sequence 10, sequence 30, sequence 31, sequence 32, sequence 33 or sequence 34.

Another object of the invention is to provide a nucleic acid molecule encoding a multi-target chimeric antigen receptor as described above.

The nucleic acid molecule encoding the multi-target chimeric antigen receptor provided by the present invention comprises a nucleic acid molecule encoding the main peptide chain or a nucleic acid molecule encoding the co-peptide chain.

A nucleic acid sequence encoding a receptor molecule can be obtained using recombinant methods known in the art, such as, for example, by screening a library from a cell expressing the gene, by obtaining the gene from a vector known to include the gene, or by utilizing standard techniques for direct isolation from cells and tissues containing the gene, or synthesis of polynucleotides chemically.

Recombinant vectors, expression cassettes, recombinant bacteria, cells or recombinant viruses containing the above nucleic acid molecules are also within the scope of the present invention.

The above recombinant vector comprises the above nucleic acid sequence or combination. In one embodiment, a nucleic acid encoding a primary peptide chain (X) or a co-peptide chain (Y) can be ligated to a promoter, and the construct is incorporated into an expression vector to express a primary peptide chain (X) or a co-peptide chain (Y). A typical cloning vector comprises a transcriptional and translational terminator, an initial sequence and a promoter that can be used to modulate the expression of a desired nucleic acid sequence. For example, lentiviral vectors are a suitable tool for achieving long-term stable inheritance of genes because they allow long-term, stable integration of genes and their replication in daughter cells. Lentiviral vectors have the added advantage of exceeding vectors derived from oncogenic retroviruses such as murine leukemia viruses because they can transduce non-dividing cells, such as hepatocytes. They also have the added advantage of low immunogenicity. The chimeric antigen receptor provided by the present invention comprises two peptide chains which can be expressed in the same cell in a known manner, including but not limited to co-transfection of respectively vectors encoding a main peptide chain (X) and a co-peptide chain (Y), or an expression vector containing two sets of expression frameworks with a nucleic acid sequence encoding a main peptide chain (X) and a nucleic acid sequence encoding a co-peptide chain (Y), or the nucleic acid sequences encoding a main peptide chain (X) and the co-peptide chain (Y) is ligated in tandem into an expression framework, and both peptide chains are expressed by inserting a ribosome binding site between the nucleic acid sequences of the main peptide chain (X) and the co-peptide chain (Y).

The cell mentioned above, is a prokaryotic cell, a yeast cell or a mammalian cell;
Or the mammalian cell is specifically a human cell;
Or the human cell is specifically an immune cell,
Alternatively, the immune cell is specifically a T cell or an NK cell.

The usage of the above-described multi-target chimeric antigen receptor, the above nucleic acid molecule or the above recombinant vector, expression cassette, recombinant strain, cell or recombinant virus or kit in immunotherapy is also within the scope of protection of the present invention;
Or the usage of the above-described multi-target chimeric antigen receptor, the above nucleic acid molecule or the above recombinant vector, expression cassette, recombinant strain, cell or recombinant virus or kit for preparing an immunotherapeutic product is also the scope of protection of the present invention..

The usage of the above multi-target chimeric antigen receptor, the above nucleic acid molecule or the above recombinant vector, expression cassette, recombinant strain, cell or recombinant virus or kit for immune cell culture and/or promotion of immune cell proliferation. The scope of invention protection;
The usage of the above-mentioned multi-target chimeric antigen receptor, the above nucleic acid molecule or the above recombinant vector, expression cassette, recombinant strain, cell or recombinant virus or kit in preparing immune cell culture and/or products for promoting immune cell proliferation is also within the scope of the invention.

The use of the above-described multi-target chimeric antigen receptor, the above nucleic acid molecule or the above recombinant vector, expression cassette, recombinant strain, cell or recombinant virus or kit for immunoassay is also within the scope of protection of the present invention;
Or the usage of multi-target chimeric antigen receptor, the nucleic acid molecule or the recombinant vector and the expression cassette, recombinant bacterium, a cell or a recombinant virus or a kit for making an immunodetection kit, in the invention is also a protection scope of the present invention;
Or the usage of the multi-target chimeric antigen receptor, the nucleic acid molecule or the recombinant vector and the expression cassette, recombinant bacteria, cells or recombinant viruses or kits for the diagnosis of tumours is also within the protection scope of the invention;
The usage of multi-target chimeric antigen receptor, the nucleic acid molecule or the recombinant vector, the expression cassette and the recombinant bacterium, the recombinant cell or the recombinant virus or the kit in preparation of a product for the treatment or the diagnosis of a tumour is also a protection scope of the invention;
The usage of multi-target chimeric antigen receptor, the nucleic acid molecule or the recombinant vector, the expression cassette and the recombinant bacterium, the recombinant cell or the recombinant virus or the kit in inhibiting or killing tumour cells is also the protection scope of the invention;
The usage of multi-target chimeric antigen receptor, the nucleic acid molecule or the recombinant vector, the expression cassette and the recombinant bacterium, the recombinant cell or the recombinant virus or the kit in preparation of a product for inhibiting or killing tumour cells is also a protection scope of the invention;
The usage of multi-target chimeric antigen receptor, the nucleic acid molecule or the recombinant vector, the expression cassette and the recombinant bacterium, the recombinant cell or the recombinant virus or the kit in inhibiting or killing the target cell expressing the antigen is also the protection scope of the invention;
The usage of multi-target chimeric antigen receptor, the nucleic acid molecule or the recombinant vector, the expression cassette and the recombinant bacterium, the transgenic cell or the recombinant virus or the kit in preparation of a target cell product for inhibiting or killing target cell with the antigen is also a protection scope of the invention.

The immune therapy is used for inhibiting or killing tumor cells through immune cells;
Or, the immune cells are T cells or NK cells and the like;
Or, the antigen is a cancer-related antigen;
Or the antigen is the related antigen of brain cancer, bladder cancer, breast cancer, cervical cancer, colorectal cancer, liver cancer and kidney cancer, lymphoma, leukemia, lung cancer, melanoma, metastatic melanoma, mesothelioma and neuroblastoma, ovarian cancer, prostate cancer, pancreatic cancer, kidney cancer, skin cancer, thymoma and sarcoma, a non-Hodgkin lymphoma, a Hodgkin lymphoma, an uterine cancer or any combination thereof;
Or, the tumour is any one of the following: brain cancer, bladder cancer, breast cancer, cervical cancer, colorectal cancer, liver cancer and kidney cancer, lymphoma, leukemia, lung cancer, melanoma, metastatic melanoma, mesothelioma and neuroblastoma, ovarian cancer, prostate cancer, pancreatic cancer, kidney cancer, skin cancer, thymoma and sarcoma, the non-Hodgkin lymphoma, the Hodgkin lymphoma and the uterine cancer; or the combination of above.
Or, the target cells are prokaryotic cells, yeast cells or mammalian cells;
Or, the mammal cells are specifically human cells;
Or, the human cells are immune cells,
Or, the immune cells are specifically T cells or NK cells
The antigen in the target cell expressing the antigen is an antigen which can be bound by a multi-target chimeric antigen receptor
The IL15Rαsushi in the invention is the Sushi fragment of alpha chain of IL15 receptor, IL15Rαsushi for short, and has the ability to bind to the IL15.

In the invention, the IL4Rα-N-F3 is a fragment from the alpha chain of IL4 receptor, with the function of binding to the IL4.

The antigen binding domain (A) of the main peptide chain and the co-peptide chain linking domain (E) of the present invention have a function to bind to an antigen. The antigen bound by antigen-binding domain is a protein produced by tumor cells causing immune responses, in particular T-cell-mediated immune responses. The selection of the antigen binding domain of the invention will depend on the specific type of the disease to be treated. The tumour antigen is well known in the field:
In one embodiment, the tumour antigens mentioned herein include, for example, neuroglioma-related antigens, cancer embryo antigen (CEA)), beta-human chorionic gonadotropin and alpha-fetal protein (AFP)), lectin-reacted AFP, thyroglobulin, RAGE-1, MN-CA/IX, human telomerase reverse transcriptase, RU1, RU2 (AS), enterocarboxylesterase, mut HSP70-2, M-CSF, prostate enzyme, prostate-specific antigen (PSA), PAP, NY-ESO -1, LAGE-la, p53, prostein, PSM, Her2/neu, Survivin, telomerase, prostate-cancer tumor antigen -1 (PCTA -1),MAGE, ELF2M, neutral leukocyte elastase, Ephrin B2, CD22, an insulin growth factor (IGF))-I, IGF-II, IGF-I receptors and Mesothelin.

In one embodiment, the tumour antigen includes one or more antigen cancer epitopes associated with a malignant tumour. A malignant tumour expresses many proteins that can be used as a target antigen for immune attack. These molecules include, but are not limited to, tissue-specific antigen such as MART -1, tyrosinase and gp100 in melanoma, and prostate acid phosphatase (PAP) and prostate-specific antigen (PSA)in prostate cancer). Other target molecules belong to the group of transformation-related molecules, such as oncogenes HER -2/Neu ERB -2. The target antigen of the other group is a fetal cancer antigen such as **carcinoembryonic antigen**(CEA). In B-cell lymphoma, and the tumour-specific individual genotype immunoglobulin forms a true tumour-specific immunoglobulin antigen which is unique to an individual tumour. The B-cell differentiation antigens such as CD19, CD20 and CD37 are other candidates of target antigens in B-cell lymphoma. Some of these antigens (CEA, HER -2, CD19, CD20, individual genotypes) have been successfully used as targets for a passive therapy using a monoclonal antibody.

In one embodiment, the tumour antigen mentioned in the invention can also be a tumour-specific antigen (TSA) or a tumour associated antigen (TAA). The TSA is unique to tumour cells and does not occur on other cells of the body. By contrast, the TAA-related antigens are not unique to tumour cells and can also be expressed on normal cells under the condition that the immune tolerance state of the antigen cannot be induced. The antigen expression on the tumour can occur under the disease condition that the immune system can respond to the antigen. TAA can be the antigen appeared during embryonic developmenta nd expressed on normal cells when the immune system is immature and cannot respond, or they can be antigens that are normally present at extremely low levels on normal cells while expressed at higher levels on tumour cells;
Examples of TSA or TAA antigens include but not limited to the following: differentiation antigens such as MART-1/MelanA, gp100 (Pmel 17), tyrosinase, TRP-1, TRP-2,tumour-specific multi-pedigree antigens such as MAGE-1, MAGE-3, BAGE, GAGE-1, GAGE-2, p15;over-expressed **embryonic antigens such as CEA, over-expressed** cancerogenic genes and mutated tumour-suppressor genes such as p53, Ras, HER-2/neu; unique tumour antigens generated by chromosome translocation such as BCR-ABL, E2A-PRL, H4-RET, IGH-IGK, MYL-RAR;And viral antigens, such as Epstein Barr virus antigen EBVA and human papilloma virus (HPV) antigen E6 and E7. Other protein antigens including TSP-180, MAGE-4, MAGE-5, MAGE-6, RAGE, NY-ESO-1, p185erbB2, p180erbB-3, c-met, nm-23H1, PSA, TAG-72, CA 19-9, CA72-4, CAM 17.1, NuMa, K-ras, beta-catenin, CDK4, Mum-1, p15, p16, 43-9F,5T4, 791Tgp72, alpha-fetal protein, beta-HCG,BCA225, BTAA, CA 125, CA 15-3/CA, 27.29/BCAA, CA 195, CA 242, CA-50, CAM43, CD68/P1, CO-029, FGF-5, G250, Ga733/EpCAM, HTgp -175, M344, MA-50, MG7-Ag,MOV18, NB/70K, NY-CO-1, RCAS1, SDCCAG16, TA-90,TA-90 \ Mac -2 binding protein \ ring-philic protein C-related protein, TAAL6, TAG72and TPS.

In one embodiment, the tumour antigen mentioned in the present invention can be a compound of MHC with a said antigen peptide fragment, including but is not limited to, HLA-gp100 complex, HLA-MART-1 complex and HLA-WT1 complex.

In one embodiment, antigen binding domain in said main peptide chain (A) or an antigen binding domain in the co-peptide chain (E) is an antibody which can be combined with an antigen, a ligand, a receptor, a polypeptide with an antigen binding capability or any combination thereof.

Antibody can be complete peptide chain or peptide fragment of Ig, Fab and scfv or any combination thereof. The ligand or the receptor can be a complete peptide chain, a peptide fragment or any combination thereof.

The co-peptide chain connecting domain (B) and the main peptide chain connecting domain (F) is a pair of peptide fragments with mutual binding functions. The peptide fragment with the mutual binding function can be a pair of receptor and ligand which can be combined with each other or a pair of antibody and the antigen which can be combined with each other. The mutually-combined receptor and ligand include, but are not limited to, IL15 and IL15R alpha, IL4 and IL4R, IL2 and IL2Ralpha, CD16 and IgGFc, CD32 and IgGFc, CD64 and IgGFc.

Wherein the transmembrane domain (C) can be derived from any membrane-binding protein or the transmembrane region of a transmembrane protein. For a transmembrane domain, in some instances, a transmembrane domain may be selected, or modification is carried out through amino acid replacement, so that the structure domain can be prevented from being bound to the same or different transmembrane region of a surface expressed membrane protein in order to reduce the steric hindrance. The transmembrane domain can be derived from a natural source or a synthetic source. In a natural source, the domain can be derived from any membrane-binding protein or transmembrane protein. The transmembrane region specifically used in the present invention may originate from that including but not limited to, alpha, beta or zeta chain of T cell receptor, CD28, CD3epsilon, CD45, CD4, CD5, CD8, CD9, CD16, CD22, CD33, CD37, CD64, CD80, CD86, CD134, CD137, CD154 and ICOS.

Wherein the intracellular signal transduction domain (D) comprises a signal transduction region, and may also comprise a co-stimulation signal transduction region or a combination thereof. The intracellular signal transduction domain refers to a protein fragment which transduces effector function signal and guides a cell to implement a specific function. For example, effector functions of T cells can be cell dissolution activity or auxiliary activity through cytokine secretion. The whole intracellular signalling structure domain can be used, but in many instances, the whole chain does not need to be used. A truncated version of the intracellular signal transduction domain can be used to replace a whole chain, as long as it has the function signal of a transduction effector.

Primary cell plasma signal transduction sequence to stimulate primary activation of immune cells in a stimulating manner or in an inhibition manner. A primary cell signal transduction sequence acting in a stimulation manner may comprise a signal transduction motif, which is known as an activation motif based on immune receptor tyrosine or an ITAM.

Examples of ITAM with primary cytoplasmic signal conduction in the present invention include TCRζ, FcεRIγ, FcRγ, FcRβ, CD3γ, CD3δ, CD3ε, CD5, CD22, CD79a, CD79b, CD66d or their combination. Preferably, the cytoplasmic signal transduction molecule of the CAR construct disclosed by the invention is derived from CD3ζ;
Co-stimulating signal transduction region in the invention refers to intracellular domains comprising co-stimulating molecules. The co-stimulating molecule is a cell surface molecule required by effective response of a lymphocyte to an antigen, and not an antigen receptor or a ligand thereof. Examples of such molecules include CD27, CD28, and 4-1 BB (cd137), OX40, CD30, CD40, PD -1, ICOS, lymphocyte function related antigen-1 (LFA-1), CD2, CD7, LIGHT, NKG2C, B7-H3 and a ligand specifically bound to CD83, etc. and the like. Thus, although the present invention mainly uses 4-1 BB and CD28 as co-stimulating signal molecules, but other co-stimulating elements are also within the scope of the present invention.

The cytoplasmic signaling sequences of cell signal transmission part of the multi-target chimeric antigen receptor can be connected with each other in random or in a specified sequence.

In one embodiment, the intracellular signal transduction domain comprises a cd3-zeta signal transduction domain. In another embodiment, an intracellular signal transduction region includes cd3-zeta, and the signal fragment of 4-1 BB. In another embodiment, wherein the intracellular signal transduction structure comprises a signal part of CD3-zeta and the signal transduction structure of CD28.

Short oligopeptides or polypeptides can be used as connecting peptides between modules in a multi-target chimeric antigen receptor, so that the function of the multi-target chimeric antigen receptor cannot be influenced, and preferably, the length of the connecting peptide is between 2 and 10 amino acids, wherein the amino acids of the connecting peptide are preferably glycine and serine.

### BRIEF DESCRIPTION OF THE DRAWINGS

Fig.1 The schematic diagram of multi-target chimeric antigen receptor. The multi-target chimeric antigen receptor consists of a main peptide chain and an co-peptide chain, wherein the main peptide chain comprises an antigen binding domain A, a co-peptide chain connecting domain B, a transmembrane domain C and an intracellular signal transduction domain D; and the co-peptide chain comprises a main peptide chain connecting domain F, which can also comprise an antigen binding domain E.

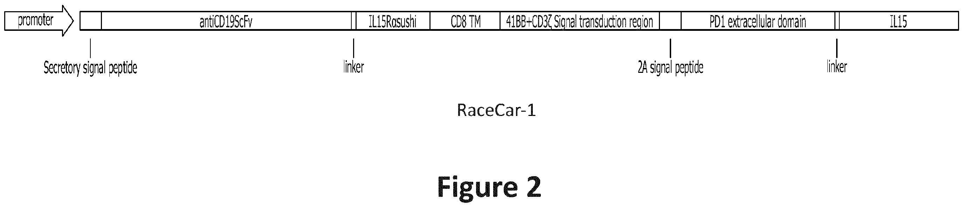

Fig. 2 Schematic diagram of RaceCar-1 expression cassette. The invention discloses a RaceCar-1 expression cassette which is composed of a promoter and an encoding gene of RaceCar-1.

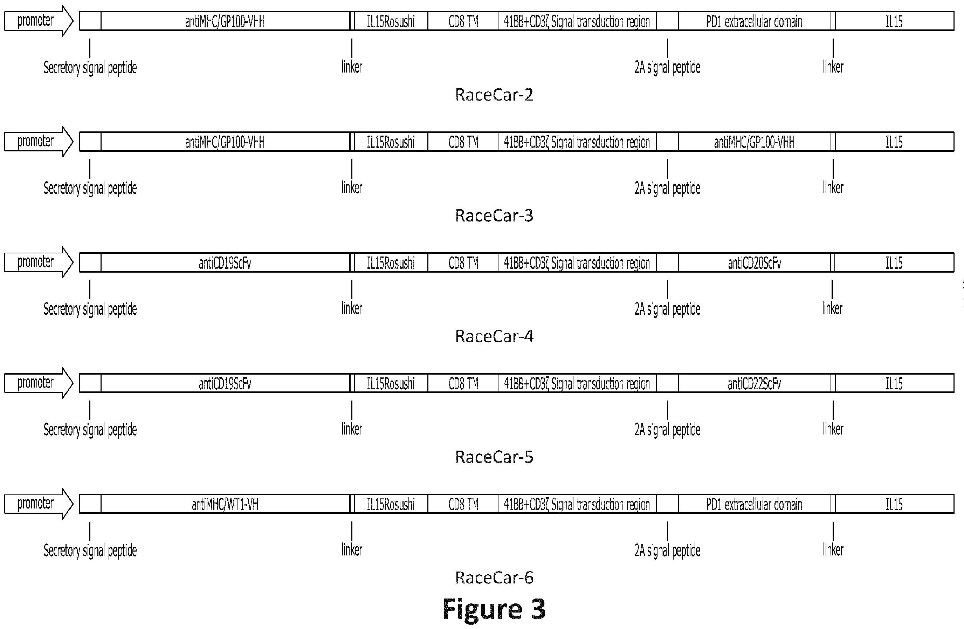

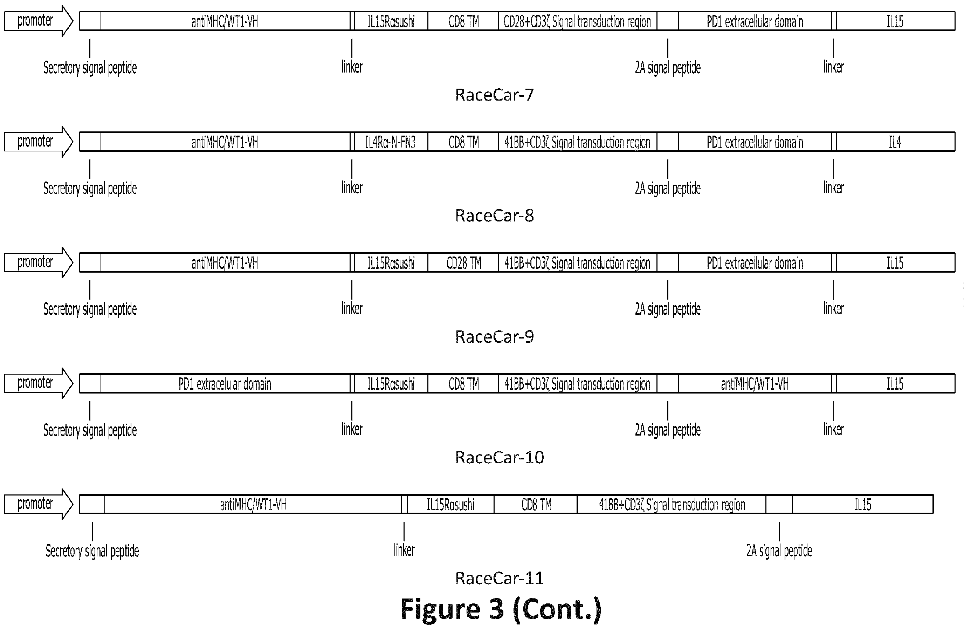

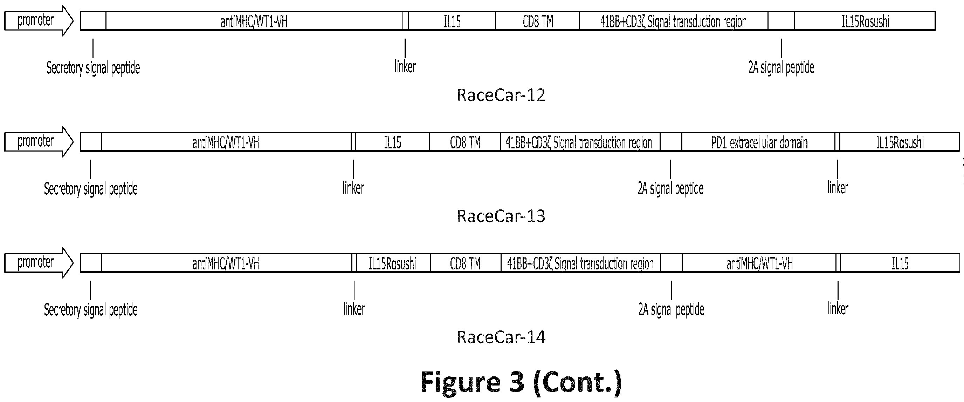

Fig. 3 Schematic diagram of different RaceCar expression cassette. The other RaceCar expression cassette is composed of a promoter and an encoding gene of RaceCar.

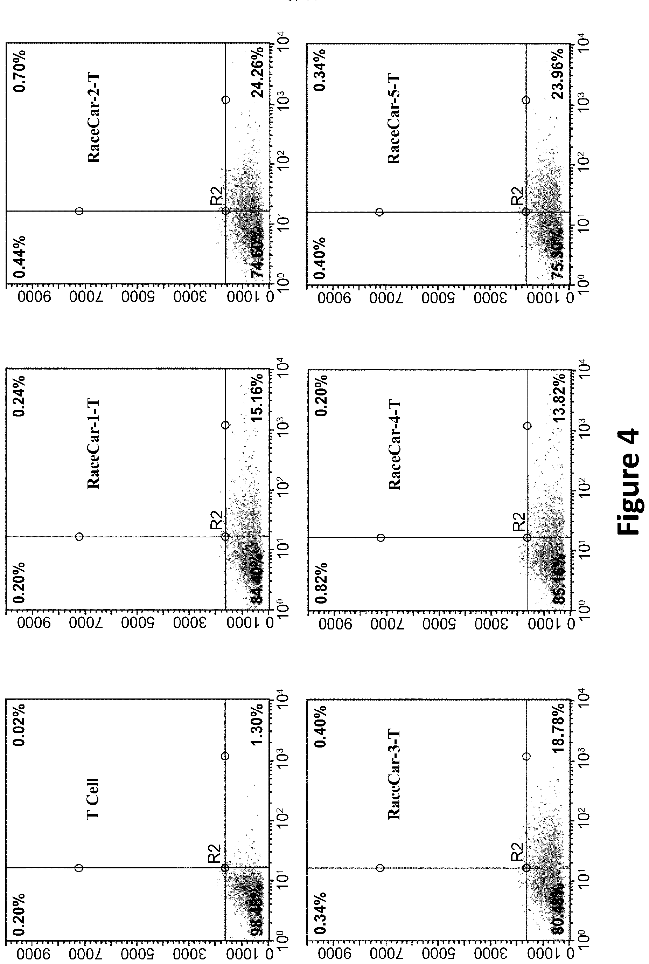

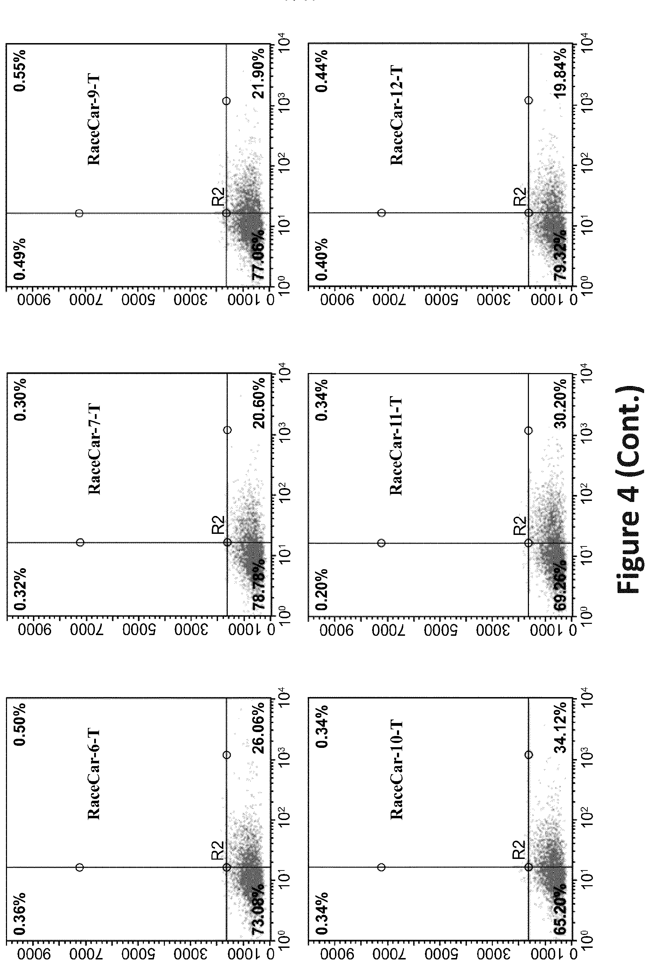

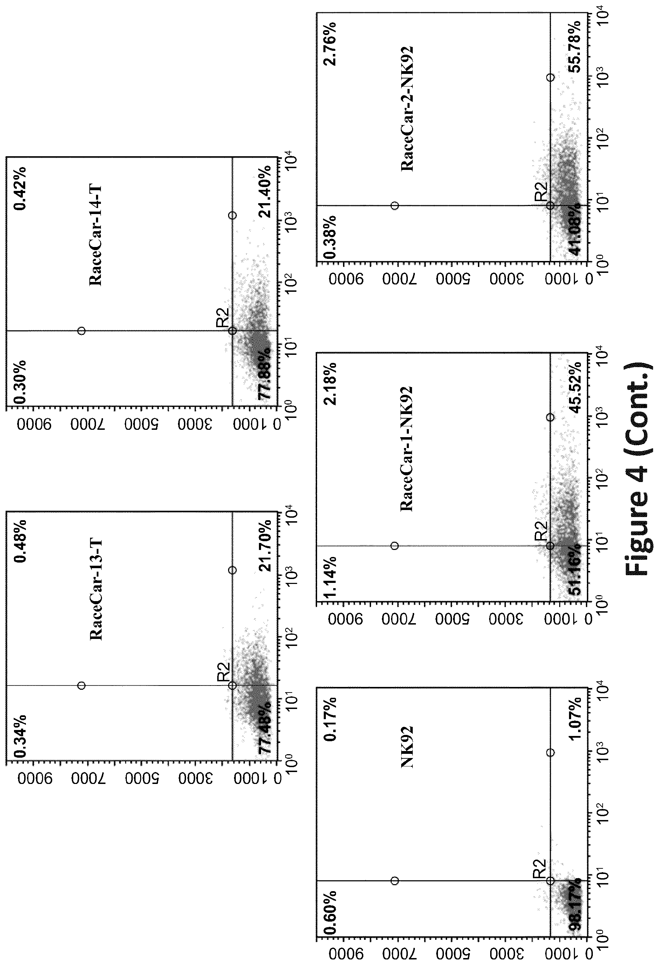

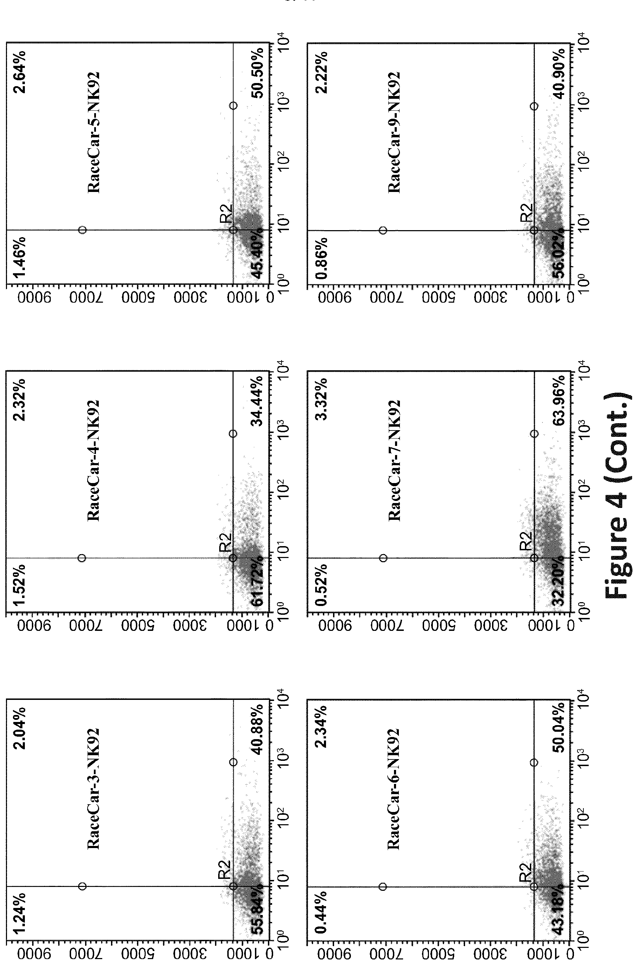

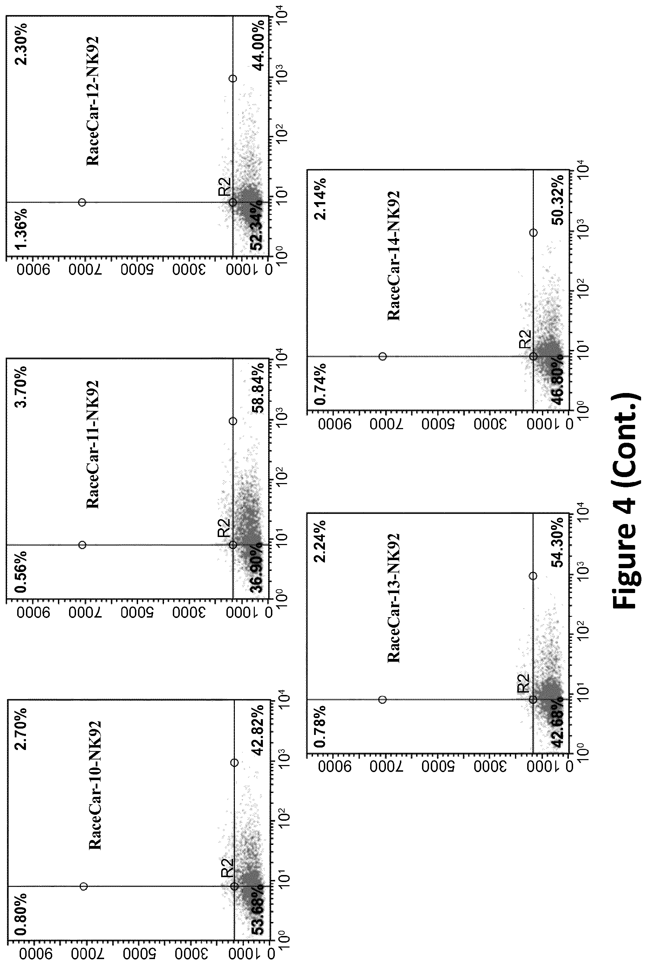

Fig. 4 FACS analysis to verify RaceCar expressed on cell surface. The horizontal coordinate in the figure represents the IL15 level, the T cell and the NK92 are not infected and used as negative control and there is no expression of IL15 on the surface detected. The expression of different levels of IL15 can be detected on the surfaces of Tcells and NK92 cells infected with RaceCar virus.SinceIL15 is the domain molecule in the co-peptide chain of RaceCar (except RaceCar-8, RaceCar-12 and RaceCar-13), IL -15 can be only detected on the surface of the cell when it binds to the domain of the main peptide chain expressed on the surface of the cell, which demonstrated that RaceCar can be expressed on the surface of T cells and NK92 cells, and expressed IL15 has immunological activity. Meanwhile, IL15 is on a main peptide chain of RaceCar -12 and RaceCar-13, and can be directly detected.

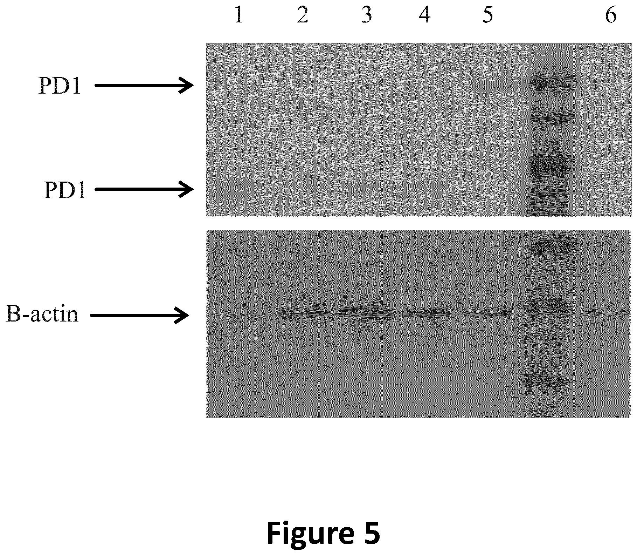

Fig.5 RaceCar expression demonstrated by Western blot. Samples of RaceCar-1-293T, RaceCar-2-293T, RaceCar-6-293T, RaceCar-7-293T, and RaceCar-10-293T, were loaded on lane1-5 respectively. The sixth lane is 293T cells without transfection used as a negative control. The lower strip is a beta-actin reference. A specific strip of PD1can be seen in the lane 1 to the lane 5, which indicates that the RaceCars are expressed on transfected cells.

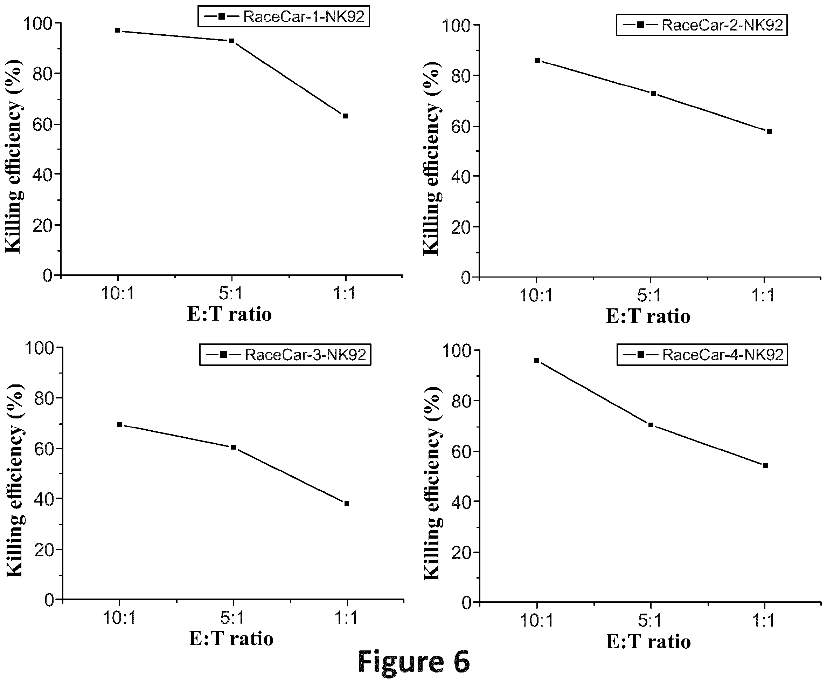

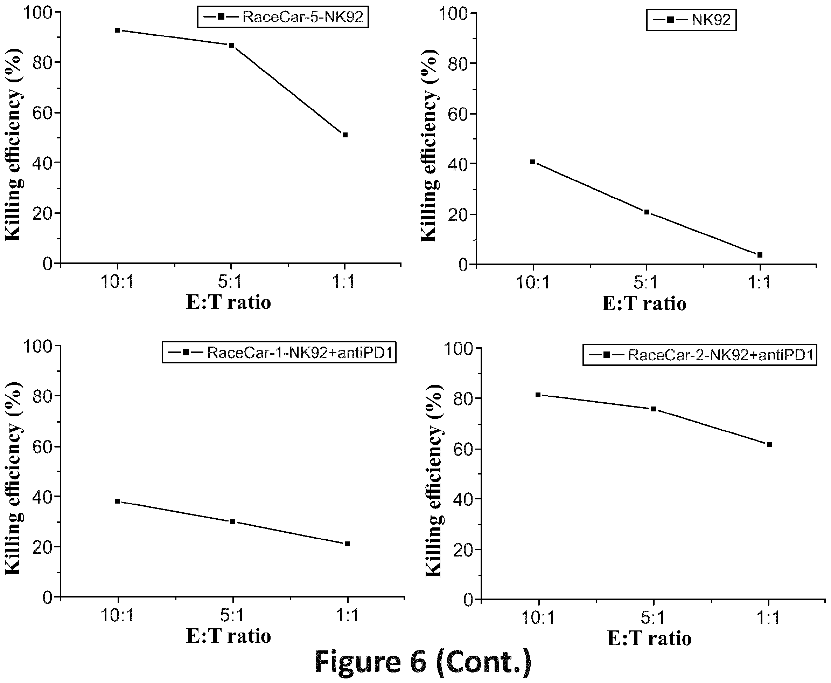

Fig. 6 Demonstrated killing capability of RaceCar-NK92 cells. The horizontal coordinate of the graph is the ratio of effector cells to target cells, and the vertical coordinate is the killing efficiency. The RaceCar-1-NK92,RaceCar-2-NK92, RaceCar-3-NK92,RaceCar-4-NK92 and RaceCar-5-NK92 all could can kill 3m-CD19-luc cells and killing efficiency is higher than that of NK92, which demonstrates that the multi-target chimeric antigen receptor RaceCar-1 targeting to CD 19 and PD-L1 is expressed in the NK92 cells, and has a biological function. There is reduced killing ability of experimental group with adding anti-CD 19 antibody onRaceCar-1-nk92 while the killing capacity of the RaceCar-2-NK92 is unchanged since anti-CD 19 antibody blocks the binding of RaceCar-1 to the target cell, so that the killing capability of the racemase-1-NK92 on the target cell is inhibited, which proves that the RaceCar-NK92 has specificity on killing 3m-CD19-luc cells.

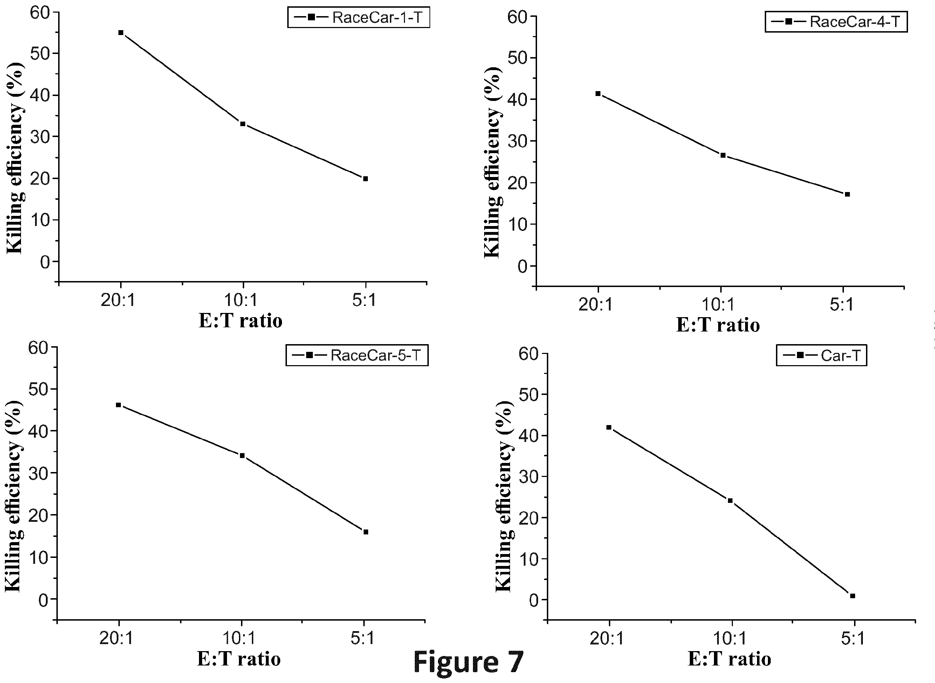

Fig. 7 The comparison of killing efficiency against target cells between RaceCar-T and Car-T cells. The horizontal coordinate of the graph is the ratio of effector cells to target cells, and the vertical coordinate is the killing efficiency. RaceCar-1-T, RaceCar-4-T, RaceCar-5-T cells can kill k562-cd19-luc cells, which demonstrates that RaceCar-1, RaceCar-4 andRaceCar-5are expressed on T cells and have a biological function; and the killing capacity of the RaceCar-Tis higher than that of common Car-T cells;

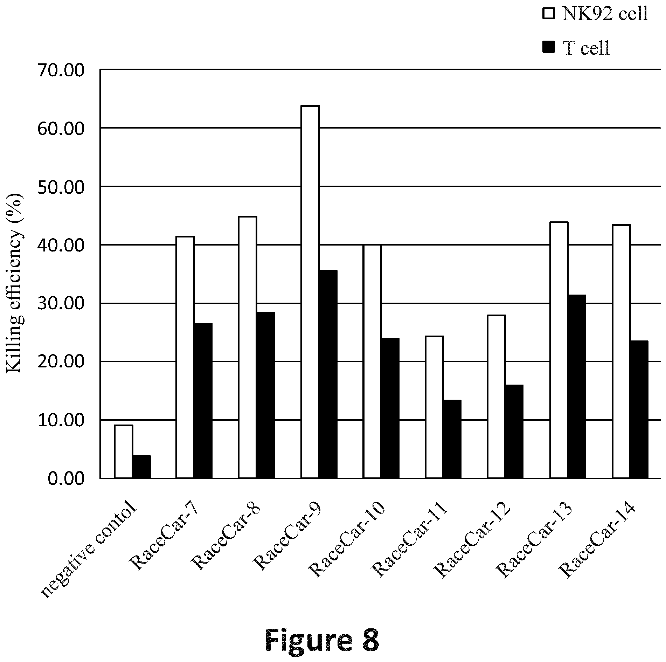

Fig. 8 Functional verification of different forms of RaceCar-T and RaceCar-NK92 targeting to MHC/WT-1 complex. The horizontal coordinate of the graph is effector cell types, and the vertical coordinate is the killing efficiency. Both RaceCar-NK92 and RaceCar-T cells can kill BV173-luc cells, and killing efficiency is higher than that of non-infected T cells or NK92 cells, demonstrating that RaceCar-6, RaceCar-7, RaceCar-8, RaceCar-9, RaceCar-10, RaceCar-11, RaceCar-12, RaceCar-13 and RaceCar-14 can express on both T cells and NK92 cells, and have biological function to direct the killing for corresponding target cells;

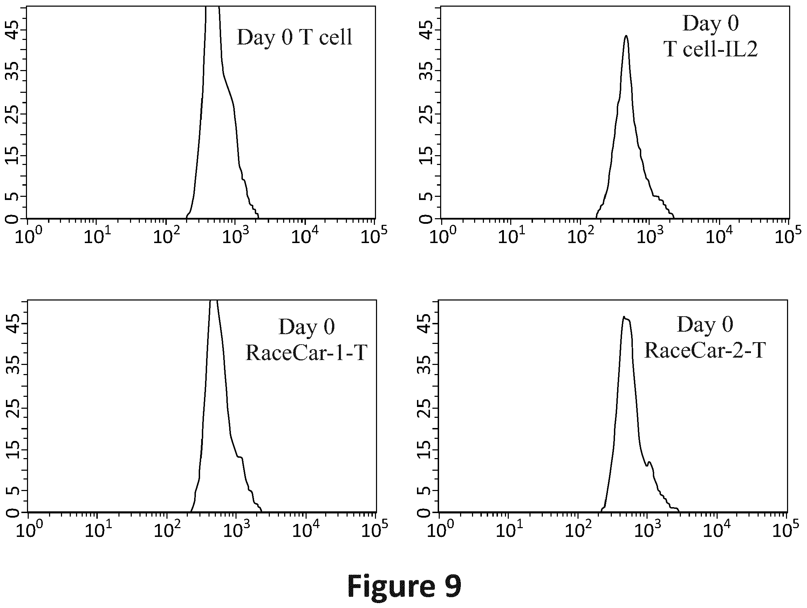

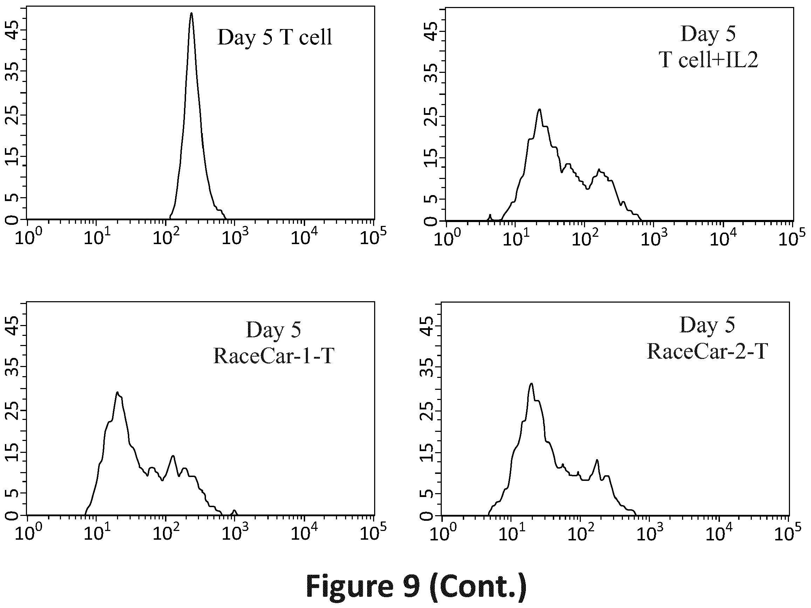

Fig. 9 FACS analysis for the ability of RaceCar to induce the proliferation of immune cells. On day zero the fluorescence signals of the four groups of cells are almost the same. T cell group is a negative control group, and its fluorescence intensity on the fifth day is basically unchanged compared with that on day zero. The FACS analysis of T cell + IL-2 group as a positive control on the fifth day can be seen in the figure, the fluorescence signal is weakened comparing to that on day 0 and the cells show different fluorescence staining, which demonstrates that cells are shown the phenotypes of division and proliferation. The fluorescence signal distribution of RaceCar-1-T and RaceCar-2-T is similar to that of the positive control group, indicating thatRaceCar-1-T and RaceCar-2-T can proliferate in a culture medium without IL2 and RaceCar has ability to promote the proliferation of immune cells such as T cells and the like.

### The best way for the application of the invention

The experimental methods used in the following examples are conventional methods if not specifically described.

The materials, reagents and the like used in the embodiments of the present invention can be obtained from commercial resources, if there is no specific description.

### Example 1. The structure of multi-target chimeric antigen receptor and expression vector construction

1. The multi-target chimeric antigen receptor (RaceCar) protein is obtained by complexing a main peptide chain X and a co-peptide chain Y;
   Main peptide chain (X) comprises antigen binding domain (A), co-peptide chain connecting domain (B) and transmembrane domain (C) and an intracellular signal transduction domain (D), and the co-peptide chain(Y) comprises an antigen binding domain (E) and a main peptide chain connecting domain (F). The co-peptide chain connecting domain (B) and a main peptide chain connecting domain (F) complementary bind to each other, enabling that the main peptide chain X and the co-peptide chain Y to polymerize to form a multi-target chimeric antigen receptor (RaceCar).
   Multi-target chimeric antigen receptor (RaceCar-1)targeting to both CD19 and PD-L1 positive cells is obtained by polymerizing a main peptide chain X1 and a co-peptide chain Y1;
   In RaceCar-1,scfv of anti-CD 19 (sequence 1) is chosen as antigen binding domain (A1) of main peptide chain X1, and co-peptide chain connecting domain (B) adopts the sushi fragment (sequence 4) ofIL15R [alpha],the transmembrane of CD8 (sequence 5) is chosen as the transmembrane domain (C) and an intracellular signal transduction domain (D) is constructed by linking a 41-BB signal transduction domain (sequence 8)to a CD3 zeta signalling region (sequence 6); The antigen binding domain (E) of co-peptide chainY1 adopts an extracellular region (sequence 2) of PD1, a receptor of the PDL1. IL15 (sequence 3) is used as the main peptide chain connecting domain (F). Finally, a main peptide chain X1 (sequence 9) connecting with the co-peptide chain Y1 (sequence 10)is made.
2. A secretory signal peptide (sequence 11) is inserted before N-terminal of the main peptide chain X1 and it was artificially synthesizing as the coding nucleic acid DNA-X1 (sequence 13); Similarly, a 2A signal peptide (sequence 12) is added in front of N-terminal of co-peptide chain Y1, wherein the coding sequence is synthesized as sequence DNA-X2 (sequence 14);
3. BamHI and HindIII sites were added to each end of DNA-X1 and cut by BamHI and HindIII to obtain the fragment of the DNA-X1; Similarly, a HindIII site is added to the 5'end of DNA-X2 sequence, and an EcoRI site is added at the 3'end, and cut by HindIII and EcoRI to obtain a nucleic acid fragment of the DNA-X2;
4. The two nucleic acid fragments obtained in 3 are inserted into the vector pFUGW (addgene, USA) cut by the same combination of enzymes, the ligated product is transformed into escherichia coli, individual colonies are picked and screened using PCR (polymerase chain reaction) analysis and finally a recombinant vector pFUGW-RaceCar-1 is obtained. The recombinant vector pFUGW-RaceCar-1 replaces the nucleic acids for expressing RaceCar-1 (the coding nucleic acid is composed of a sequence 13, an AAGCTT and a sequence 14, and the last nucleotide of the sequence 13 is adjacent to the first base group of the AAGCTT, the first nucleotide of the sequence 14 is adjacent to the last base of the AAGCTT thatis a digestion site for HindIII) for a fragment between BamHI and EcoRI cleavage sites of pFUGW vector. RaceCar expression cassette is formed by coding nucleic acids of RaceCar-1 and the promoter in pFUGW(FIG. 2)
5. Designing other multi-target chimeric antigen receptors in the same manner as shown in table 1:

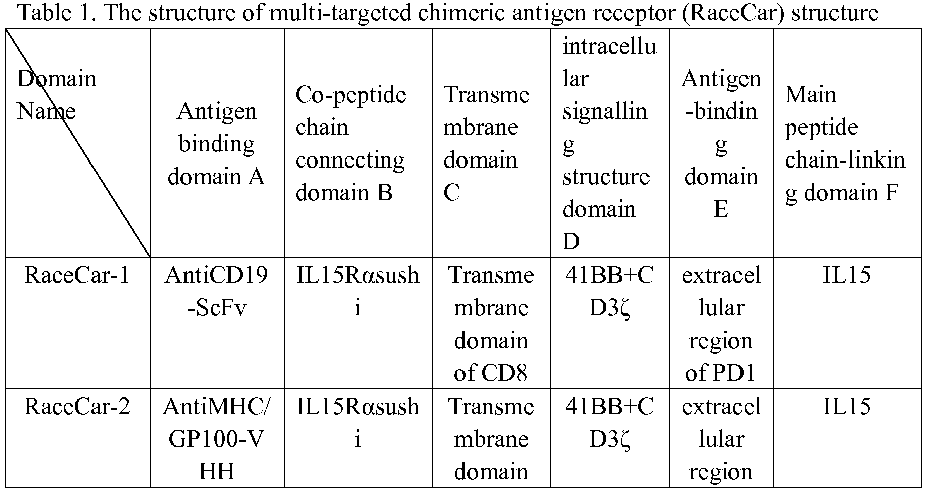

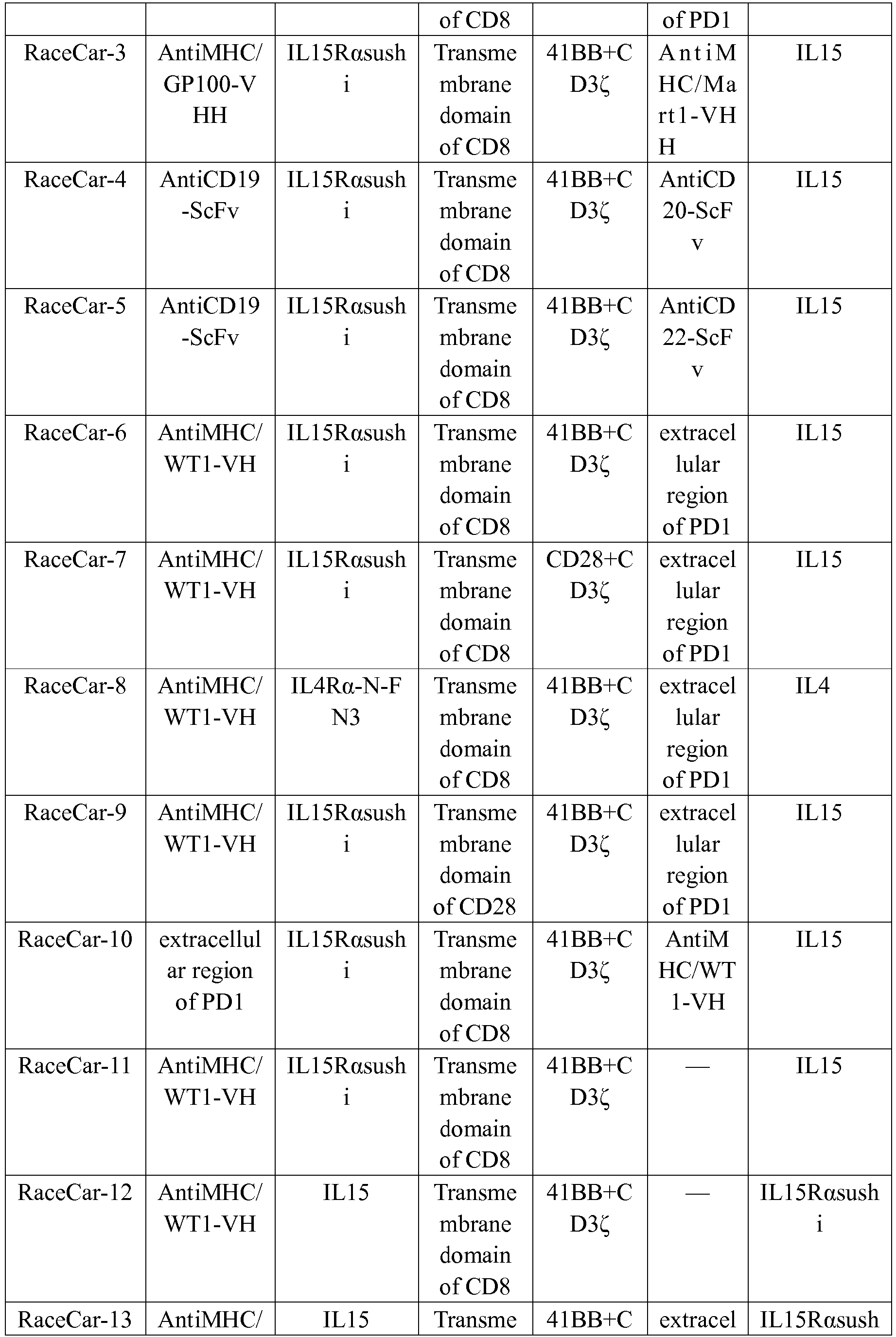

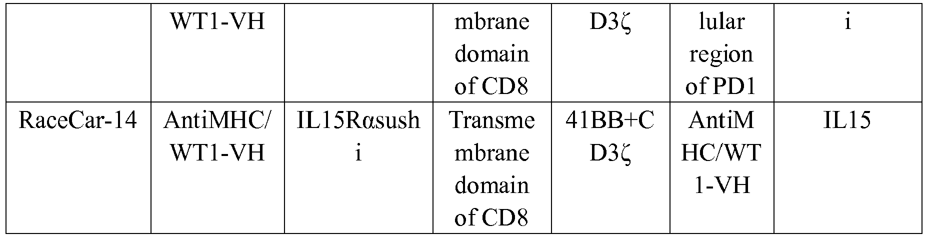

On Table 1, the amino acid sequence of AntiMHC/GP100-VHH is listed on sequence 15, and the amino acid sequence of AntiMHC/Mart-1-VH is listed in sequence 16, the amino acid sequence of the AntiCD20-scfv is listed on sequence 17, and the amino acid sequence of the AntiCD22-scfv is listed on sequence 18, the amino acid sequence of the AntiMHC/WT1-VH is listed on sequence 19, and the sequence of the IL4Ralpha-N-FN3amino acid sequence is as shown in the sequence 20, the sequence of IL4 is listed on sequence 21, and the sequence of CD28 transmembrane region is listed as sequence 22 .

The sequences of different main peptide chain X are listed as follows: main peptide chain X2 (the amino acid sequence is sequence 23);the main peptide chain X3 (the amino acid sequence is sequence 24); X4 (the amino acid sequence is sequence 25); X5 (the amino acid sequence is sequence 26): X6 (the amino acid sequence is a sequence 27); X7 (the amino acid sequence is sequence 28), X8 (the amino acid sequence is a sequence 29); and the co-peptide chain Y2 (the amino acid sequence is a sequence 30), Y3 (the amino acid sequence is sequence 31); Y4 (the amino acid sequence is sequence 32), Y5 (the amino acid sequence is sequence 33); Y6 (the amino acid sequence is the sequence IL15 and listed on sequence 3);Y7 (IL15Ralpha-sushi, the amino acid sequence is sequence 4); Y8 (the amino acid sequence is sequence 34), and Y9 (the amino acid sequence is sequence 39).

Wherein:
RaceCar-2 is composed of a main peptide chain X2 and a co-peptide chain Y1;
RaceCar-3 is composed of a main peptide chain X2 and a co-peptide chain Y2;
RaceCar-4 is composed of a main peptide chain X1 and a co-peptide chain Y3;
RaceCar-5 is composed of a main peptide chain X1 and a co-peptide chain y4;
RaceCar-6 is composed of a main peptide chain X3 and a co-peptide chain Y1;
RaceCar-7 is composed of a main peptide chain X4 and a co-peptide chain Y1;
RaceCar-8 is formed by main peptide chain X5 and a co-peptide chain Y9;
RaceCar-9 is composed of a main peptide chain X6 and a co-peptide chain Y1;
RaceCar-10 is composed of a main peptide chain X7 and a co-peptide chain Y5;
RaceCar-11 is composed of a main peptide chain X3 and a co-peptide chain Y6;
RaceCar-12 is composed of a main peptide chain X8 and a co-peptide chain Y7;
RaceCar-13 is composed of a main peptide chain X8 and a co-peptide chain Y8;
RaceCar-14 is composed of a main peptide chain X3 and a co-peptide chain Y5.

The same as RaceCar1, when the expression vectors for other RaceCars are constructed, a secretory leader sequence (sequence 11)needs to be added before N-terminal sequence of the main peptide chain of other RaceCars wherein a 2A signal peptide is added in front of the amino acid sequence of the co-peptide chain; The specific coding nucleic acid sequences are as follows:
The nucleic acid sequence expressing RaceCar2 is composed ofDNA-X2 (sequence 40), AAGCTT and DNA-Y1 (sequence 14);
The nucleic acid sequence for expressing RaceCar3 is composed of DNA-X2 (sequence 40), AAGCTT and DNA-Y2 (sequence 47);
The nucleic acid sequence expressing RaceCar4 is composed of DNA-X1 (sequence 13), AAGCTT and DNA-Y3 (sequence 48);
The nucleic acid sequence expressing RaceCar5 is composed ofDNA-X1 (sequence 13), AAGCTT and a DNA-Y4 (sequence 49);
The nucleic acid sequence expressing RaceCar6 is composed of DNA-X3 (sequence 41), AAGCTT and DNA-Y1 (sequence 14);
The nucleic acid sequence for expressing RaceCar7consists of DNA-X4 (sequence 42), AAGCTT and DNA-Y1 (sequence 14);
The nucleic acid sequence for expressing RaceCar8 consists ofDNA-X5 (sequence 43), AAGCTT and DNA-Y9 (sequence 50);
The nucleic acid sequence expressing RaceCar9 is composed of DNA-X6 (sequence 44), AAGCTT and DNA-Y1 (sequence 14);
The nucleic acid sequence for expressing RaceCar10 is composed of DNA-X7 (sequence45), AAGCTT andDNA-Y5 (sequence 51);
The nucleic acid sequence for expressing RaceCar11 is composed of DNA-X3 (sequence 41), AAGCTT and DNA-Y6 (sequence 52);
The nucleic acid sequence for expressing RaceCar12 is composed of DNA-X8 (sequence 46), AAGCTT and a DNA-Y7 (sequence 53);
The nucleic acid sequence for expressing RaceCar13is composed of DNA-X8 (sequence46), AAGCTT and DNA-Y8 (sequence 54);
The nucleic acid sequence for expressing RaceCar14is composed of DNA-X3 (nucleic acid sequence 41), AAGCTT and DNA-Y5 (sequence 51);
The nucleic acid sequences for expressing other multi-targeted chimeric antigen receptors are obtained in the same manner; And recombinant vectors ranging from pFUGW-RaceCar-2 to pFUGW-RaceCar-14 are constructed. In recombinant vectors there are expression cassettes for other individual multi-target chimeric antigen receptor RaceCar (FIG. 3).

Recombinant vectors ranging from pFUGW-RaceCar-2 to pFUGW-RaceCar-14 come from replacing a fragment between BamHIand EcoRI cleavage sites of pFUGW vector with nucleic acid sequences for expressing RaceCar2 to RaceCar14 respectively.

### Example 2. FACS analysis to confirm RaceCar expression on cells

1,RaceCar-1-NK92, RaceCar-2-NK92, RaceCar-3-NK92, RaceCar-4-NK92, RaceCar-5-NK92, RaceCar-6-NK92, RaceCar-7-NK92, RaceCar-9-NK92, RaceCar-10-NK92, RaceCar-11-NK92, RaceCar-12-NK92, RaceCar-13-NK92, RaceCar-14-NK92, RaceCar-1-T, RaceCar-2-T, RaceCar-3-T, RaceCar-4-T, RaceCar-5-T, RaceCar-6-T, RaceCar-7-T, RaceCar-9-T, RaceCar-10-T, RaceCar-11-T, RaceCar-12-T, RaceCar-13-T, RaceCar-14-T cells obtained in example 4, are suspended in PBS (phosphate buffered saline)respectively, the concentration of cells is controlled to be in the range of 1 ^{∗} 10E6/ml.
2, 1.5 ul of anti-hIL-15 PE conjugates is (R&D, IC2471P) is added into 200 ul of treated cells and incubates on ice for 30 minutes. The supernatants are removed by centrifugation and the cells are re-suspended by adding an equal amount of PBS (phosphate buffer saline).
3.FACS analysis to verify RaceCar expressed on cell surface (Fig. 4). The horizontal coordinate in the figure represents the IL15 level, the T cell and the NK92 are not infected and used as negative control and there is no expression of IL15 on the surface detected. The expression of different levels of IL15 can be detected on the surfaces of T cells and NK92 cells infected with RaceCar virus. Since IL15 is the domain molecule in the co-peptide chain of RaceCar (except RaceCar-8, RaceCar-12 and RaceCar-13), IL -15 can be only detected on the surface of the cell when it binds to the domain of the main peptide chain expressed on the surface of the cell, which demonstrated that RaceCar can be expressed on the surface T cells and NK92 cells, and expressed IL15 has immunological activity. Meanwhile, IL15 is on a main peptide chain of RaceCar -12 and RaceCar-13, and can be directly detected.

### Example 3. The expression of RaceCar demonstrated by Western blot.

1. 1.2 ^{∗} 10E6 of Lenti-X -293T cells (Clonetech, 632180, hereinafter referred to as 293T for short) are laid in a 6-wellplate, and cultured overnight at the temperature of 37 °C and 5% of CO₂;
2. 293T cells described as above 1 are transfected respectively with vectorspFUGW-RaceCar-1, pFUGW-RaceCar -2,pFUGW-RaceCar-6, pFUGW-RaceCar-7 and pFUGW-RaceCar-10 mentioned in example 1 using lipofectamine 3000, and are named as RaceCar-1-293T, RaceCar-2-293 T, RaceCar-6-293T, RaceCar-7-293Tand RaceCar-10-293T and cultured with 5% of CO₂ for 48 hours at the temperature of 37°C.
3. 5 x10^6 of RaceCar-1-293T, RaceCar-2-293T, RaceCar-6-293T, RaceCar-7 -293T and RaceCar-10-293Twere taken and centrifuged for 10 minutes at 1500R, and the supernatants were discarded.
4. 200 ul of cell lysis solution (Beyotime, Shanghai, P0013B) was added onto each cells and incubated on ice for 30 minutes. The cells were centrifuged at 8000 g for 5 minutes. 20 ul of supernatants for each samples was mixed with 5 x protein electrophoresis buffer and incubated for 5 minutes at 95 °C to be used as electrophoresis samples.
5. SDS-PAGE electrophoresis was carried out and gel was transferred onto membrane at 100V, 200mA for one hour.
6. Protein transferred PVDF (polyvinylidene fluoride) membrane was blocked in 5% skimmed milk of PBS overnight. The membrane was washed 3 times, each time for 15 minutes with PBST on a shaker.
7. PVDF with transferred proteins was incubated with 0.1% anti-PDl antibody (R&D MA1086-100)dissolved in 1% skimmed milk of PBS solution for 1 hour at the shaking condition. The membrane was washed with PBST for three times each for 15 minutes.
8. Washed membrane was incubated with 0.1% HRP conjugated anti-mouse IgG(H+L) antibody (Bejotime, Shanghai, A0216) dissolved in 1% skimmed milk of PBS solution for 1 hour at the shaking condition. The membrane was washed with PBST for three times each for 15 minutes.
9. The membrane was visualized with a W-TMB kit (Sangon Biotech, Shanghai, C510025-0005) and the result is shown in FIG. 5. The first to fifth lane samples belong to respectively RaceCar-1-293T, RaceCar-2-293T, RaceCar-6-293T, RaceCar-7-293T and RaceCar-10-293T and a negative control, 293T cells without transfection is located on lane 6. The lower stained line is a beta-actin reference. As shown on Fig. 5, a specific line as PD1 staining can be found in the lane 1 to the lane 5, demonstrating that RaceCar was well expressed on the surface of transfected cells.

### Example 4, Packaging of Lentivirus with pFUGW-RaceCar and cell infection

1. Taking RaceCar-1 as an example, 293T cells were cultured overnight to the density of 70-80%for transfection. pCMV-VSV-g, pCMV-deltaR8.91 (pCMV-VSV-G and pCMV-delta R8.91 were Addgene products)providing virus shell protein were mixed withpFUGW-RaceCar-1 prepared from example 1 according to the ratio of pCMV-VSV-g: pCMV-deltaR8.91: pFUGW-RaceCar-1= 1: 3: 4 to obtain 40 ug of co-transfection plasmids.
2. In a 15 ml of centrifugal tube marked as Tube1, co-transfection plasmids described as above 1 were added and serum-free DMEM were added up to 1.5 ml, and in an another 15 ml of centrifugal tube marked as Tube2,120 ul of PEI solution (Sigma, GF70215828,1mg/ml was added and serum-free DMEM (DMEM) was added up to 1.5 ml. Both reagents in Tube 1 and Tube2 were properly mixed respectively.ThenTube1 was vortexed and PEI in Tube 2 was added into Tube1 drop by drop to obtain a plasmid-PEI mixture solution, and the solution was kept for 30 minutes at room temperature; The mixture was added into 293T culture for transfection without suspending cells. Transfected cells were cultured for 24 hours in a 37°C/CO₂ incubator. Then the cell culture media was discarded and 20 ml of fresh 10% FBS-DMEM was supplemented. Finally, sodium butyrate was added until the final concentration of sodium butyrate was 10 mM. The cells were cultured for further 48 hours in an incubator with 5% CO₂ at 37 °C.
3. The cell culture supernatant was collected by centrifuging for 15 minutes at 4000 g/min, and the supernatant was filtered through a 0.45-micron filter to obtain 1x 10^6 TU/ml of virus solution. To origin virus solution adding 1/3 volume of a 40% PEG solution (g/g) and standing overnight at the temperature of 4 °C after mixing. On the following day the precipitate of virus is centrifuged at 1800 g for 45 minutes at 4 °C and the supernatant was discarded. The virus precipitate was re-suspended with 1/10 of original volume of culture media (X-VIVO15 for T cells, MEM-Alpha for NK92 cells) to obtain a 10-fold concentrated virus suspension;
4. 3x10^5 NK92 (ATCC, CRL -2407) and 1 x10^6 T cells (T cells from a healthy blood donor, separated by gradient centrifugation, stimulated and cultured by using OKT3) were infected respectively with 1x10^7 virus particles, and 8 ug/ml polybrene (Sigma and H9268-5 G) was added. Infected cells were transferred to one well of a 24-well plate, and centrifuged at 1500R for 45 minutes at 32□. The plate was incubated at 37□, 5%CO₂ for 3 hours and the media was exchanged with media containing 100U/ml of IL-2 (for T cells 100ng/ml of OKT3 was required) and the plate was incubated with the same conditions as above to obtain RaceCar-1-NK92 and RaceCar-1-T for the following experiments.
5. With the same methods described as above onlypFUGW-RaceCar-1 vector was replaced with one among pFUGW-RaceCar-2 to pFUGW-RaceCar-14 respectively to obtain corresponding RaceCar-2-NK92, RaceCar-3-NK92, RaceCar-4-NK92, RaceCar-5-NK92, RaceCar-6-NK92, RaceCar-7-NK92, RaceCar-8-NK92, RaceCar-9-NK92, RaceCar-10-NK92, RaceCar-11-NK92, RaceCar-12-NK92, RaceCar-13-NK92, RaceCar-14-NK92 and RaceCar-2-T, RaceCar-3-T, RaceCar-4-T, RaceCar-5-T, RaceCar-6-T, RaceCar-7-T, RaceCar-8-T, RaceCar-9-T, RaceCar-10-T, RaceCar-11-T, RaceCar-12-T, RaceCar-13-T and RaceCar-14-T cells.

### Example 5. Expression of RaceCar in NK92 and verification of targeted killing of RaceCar-NK92

1. Target cell 3m-CD19-luc was prepared by integrating transgenes of CD19 (sequence 36) and luciferase (sequence 37) to the genome of Malme-3M cell (ATCC, HTB-64). Experimental groups were divided as follows: 1, RaceCar-1-NK92 prepared by using example 4 serves as effector cells; 2, RaceCar-2-NK92 prepared by using example 4 serves as effector cells; 3, RaceCar-3-NK92 prepared by using example 4 serves as effector cells; 4, RaceCar-4-NK92 prepared by using example 4servesas effector cells; 5, RaceCar-5-NK92 prepared by using example 4 is used as effector cells; 6,NK92 used as effector cells;7, RaceCar-1-NK92 used as effector cells; 8, RaceCar-2-NK92 as effector cells. The cytotoxicity assay was carried out after an anti-CD 19 antibody (biotin labelled mouse anti-Human CD19 antibody, Beijing biodragon immunetechnologies, Beijing, BDLS-1968-100) was added in group7 and 8. The target cell only used as negative control group.
2. The effector cells were mixed with 1 × 10^4 target cells in 100 ul in a 96-well plate at a ratio of 10:1, 5:1, 1:1, and incubated at 37 ° C , in 5%CO₂ incubator for 24 h. 50 µl of 1% Triton lysate plus 1 µl of substrate (mixture of 300 ug / mL Luc solution and 2 mg / mL ATP solution in a volume ratio of 3:1) was added into each well, the cells were lysed for 5 min, and luciferase fluorescence intensity was detected. The killing efficiency was calculated as follows = {(negative control fluorescence) Value - experimental group fluorescence value} / negative control fluorescence value} ^{∗} 100%.
3. The results are shown in Figure 6. It is shown that RaceCar-1-NK92, RaceCar-2-NK92, RaceCar-3-NK92, RaceCar-4-NK92, and RaceCar-5-NK92 can kill 3m-CD19-luc cells more efficiently than that of NK92, which demonstrated that the multi-target chimeric antigen receptor RaceCar-1 targeting CD19 and PD-L1 is expressed in NK92 cells and has biological functions. In the experimental group to which the CD19 antibody was added, the killing ability of RaceCar-1-NK92 was decreased, while that of RaceCar-2-NK92 was almost unchanged, because anti-CD 19 antibody to the target cells blocked the binding of the CAR on effector cells to the target cells, thereby inhibiting killing. The killing ability of RaceCar-1-NK92 against target cells demonstrated that RaceCar-NK92 is specific for the killing of 3m-CD19-luc cells.

Example 6. Expression of RaceCar in T cells and comparison of the targeted killing between RaceCar-T and Car-T.
1. The cell line k562-cd19-luc was obtained by incorporating nucleic acid sequences encoding CD19 antigen (sequence 36) and luciferase (sequence 37) into the genome of k562 cell line (ATCC) to express CD19 antigen and luciferase and used as a target cell. The effector cells used were RaceCar-1-T, RaceCar-4-T, and RaceCar-5-T obtained in Example 4, and the control effector cells used were Car-T cells (antiCD19ScFv-CD8TM-41BB-CD3ζ chimeric antigen receptor, sequence 38) obtained by virus transfecting into T cells according to the above to express a conventional antiCD19ScFv-CD8TM-41BB-CD3ζ chimeric antigen receptor, target cells only without adding effector cells were used as a negative control for cell killing experiments.
2. k562-cd19-luc cells were plated at 1x10^4 cells/well, and effector cells were added at a ratio of effector cells to target cells of 5:1, 10:1, and 20:1, respectively. After 24 h, 50 µl of 1% Triton lysate + 1 µl substrate (mixture of 300 ug/mL Luc aqueous solution and 2 mg/mL ATP aqueous solution at a volume ratio of 3:1) were added to the cells, and lysed for 5 min. The luciferase luminescence was detected by a microplate reader to calculate the killing. Efficiency = {(negative control fluorescence value - experimental group fluorescence value) / negative control fluorescence value} x 100%.

The results are shown in Fig. 7. In the figure, the abscissa is the ratio of effector cells to target cells, and the ordinate is the killing efficiency. It can be seen that RaceCar-1-T, RaceCar-4-T, and RaceCar-5-T cells can kill k562-cd19-luc cells, which demonstrates that RaceCar-1, RaceCar-4-T, RaceCar-5-T are expressed in T cells and have biological functions; and the killing ability of RaceCar-T cells is higher than that of common Car-T cells.

### Example 7. Cell killing of different types of RaceCar-T cells and RaceCar-NK92 cells targeting to MHC/WT1

1. The cell line BV173-luc was obtained by incorporating a nucleic acid sequence encoding a luciferase (SEQ ID NO: 37) into the genome of BV173 cell line (DSMZ, ACC20) expressing a GP100/MHC molecular complex on the surface and as a target cell. The effector cells used were RaceCar-6-NK92, RaceCar-7-NK92, RaceCar-8-NK92, RaceCar-9-NK92, RaceCar-10-NK92, RaceCar-11-NK92, and RaceCar-12-NK92 obtained in Example 4. RaceCar-13-NK92, RaceCar-14-NK92 and RaceCar-6-T, RaceCar-7-T, RaceCar-8-T, RaceCar-9-T, RaceCar-10-T, RaceCar-11-T, RaceCar-12-T, RaceCar-13-T, RaceCar-14-T, control effector cells were uninfected NK92 cells or T cells, and cell killing experiments were performed without adding effector cells as a negative control.
2. BV173-luc cells were plated at 1x10^4 cells/well, and the effector cells were added to the target cells at a ratio of 5:1. After 24 h, 50 µl of 1% Triton lysate + 1 µl substrate (300 ug/mL Luciferin solution and 2 mg/mL ATP were mixed at a volume ratio of 3:1) were added to the cells, and lysed for 5 min. The luciferase luminescence was detected by a microplate reader. Killing efficiency = {(negative control fluorescence value - experimental group fluorescence value) / negative control fluorescence value} x 100%.

The results are shown in Fig. 8. In the figure, the abscissa is different effector cells, and the ordinate is the killing efficiency. It can be seen that both RaceCar-NK92 and RaceCar-T cells can kill BV173-luc cells and the efficiency is higher than that by uninfected T cells or NK92 cells, which demonstrates that RaceCar-6, RaceCar-7, RaceCar-8, RaceCar-9, RaceCar-10, RaceCar-11, RaceCar-12, RaceCar-13, and RaceCar-14 can be expressed in NK92 cells or T cells and have the biological function of mediating effector cells to kill target cells.

### Example 8, Effect of RaceCar on the proliferation of immune cells

1. 1 x 10^6 of RaceCar-1-T and RaceCar-2-T cells obtained in Example 4 were placed in 2.5 ml of X-VIVO medium, and 1 × 10^6 T cells were placed as a negative control in 2.5 ml of X-VIVO medium. Another 1 × 10^6 T of cells was placed as a positive control in 2.5 mL of X-VIVO medium supplemented with IL-2 at a final concentration of 100 U/ml. The four groups of cells were incubated at 37 ° C, 5% CO₂.
2. The working solution was prepared by dissolving CSFE dye into DMSO as 5umol/L solution. At the beginning of the culture and the 5th day of culture, 1 × 10^4 cells were removed from the four groups of cells and the CSFE dye was added into each group of cells according to the ratio of CSFE working solution: medium volume ratio 1: 2000 and incubated at 37 ° C for 30 minutes.
3. The supernatant was discarded by centrifugation at 1000 g for 5 minutes, and the cells were re-suspended using an equal volume of X-VIVO medium. This procedure was repeated twice.
4. The cells stained with CSFE would have green fluorescence and the intensity of green fluorescence would reduce as the cells divide. The cells of different generations show different fluorescence signals. The FACS spectrum is used to analyse the fluorescence of cells. In Figure 9, it can be seen that at day 0, the fluorescence signals of the four groups of cells were basically the same. The T cell group was the negative control group. The fluorescence intensity of the flow detection on the 5th day was basically unchanged compared with that on day 0. T cell+IL2 was the positive control group and the cells showed different fluorescence signals between 5th day and the day 0, which confirmed that the cells proliferated during 5 days of culture. As shown on the figure, the distribution of the fluorescence signals of RaceCar-1-T and RaceCar-2-T was similar to that of the positive control group with IL-2 on the 5th day, demonstrating that RaceCar-1-T and RaceCar-2-T can also proliferate normally in the medium without IL2. The overall results proves that RaceCar has an ability to promote the proliferation of immune cells such as T cells.

### Industrial application

The experiments of the present invention prove that the multi-target chimeric antigen receptor of the present invention can bind to different antigens through its two antigen binding domains and mediate specific cell killing, can also improve the accuracy of therapeutic targeting, and can circumvent the relapse of the disease caused by down-regulation of single target expression; The multi-target chimeric antigen receptor of the present invention can bind to different antigens through its two antigen-binding domains, and can block the immune suppression signal and improve the ability to kill tumours if one of the antigen-binding domains binds to the antigen related to immune checkpoint proteins. The multi-target chimeric antigen receptor of the present invention introduces a cytokine and cytokine receptor complex, which can produce the function of cytokines, for example, the introduction of a complex of IL-15 and IL-15Rα-sushi stimulates the proliferation of T and NK cells, exempting them from cytokine-dependent activation.

## Claims

1. A multi-target chimeric antigen receptor is composed of a main peptide chain and a co-peptide chain; The main peptide chain consists an antigen binding domain A, a co-peptide chain connecting domain B, a transmembrane domain C and an intracellular signalling structure domain D; the co-peptide chain includes a main peptide chain-linking domain F; the antigen-binding domain A is a polypeptide having an ability to bind to an antigen; the co-peptide chain-linking domain B can bind to the main-peptide chain-linking domain F with each other; The co-peptide chain domain B and the main peptide chain domain F are cytokines and full length of corresponding cytokine receptors or cytokines and corresponding cytokine receptors fragment that can bind to each other; The transmembrane domain C is the transmembrane region of any membrane-bound protein or the transmembrane region of a transmembrane protein; the intracellular signalling domain D comprises a primary signalling region.

2. The multi-target chimeric antigen receptor according to claim 1, wherein: the co-peptide chain further comprises an antigen-binding domain E; and the antigen-binding domain E is a polypeptide with an antigen-binding function; The antigen-binding domain E and the antigen binding domain A can be the same or different.

3. The multi-target chimeric antigen receptor according to claim 1 or 2, wherein the intracellular signalling domain D further comprises a costimulatory signalling region.

4. The multi-target chimeric antigen receptor according to claim 2 or 3, wherein the polypeptide with an antigen-binding ability is an antibody capable of binding to an antigen, a ligand capable of binding to an antigen, or a receptor with an ability to bind to an antigen.

5. The multi-target chimeric antigen receptor according to claim 4, wherein the antibody capable of binding to an antigen is a full length antibody, Fab of an antibody, Fc of an antibody, a scFv, a VHH, VH of an antibody, A full-length polypeptide of VL of an antibody or a partial fragment of VL of an antibody; the antigen-binding ligand or the antigen-binding receptor is a full-length polypeptide or a partial fragment of a ligand or a receptor.

6. The multi-target chimeric antigen receptor according to any one of claims 2 to 5, wherein the antigen bound by the antigen binding domain A or the antigen binding domain E is either a cell surface antigen or an MHC molecule with a peptide.

7. The multi-target chimeric antigen receptor according to claim 6, wherein the antigen is a cancer-associated antigen; or the antigen is an antigen related to brain cancer, bladder cancer, breast cancer, cervical cancer, colorectal cancer, liver cancer, kidney cancer, lymphoma, leukaemia, lung cancer, melanoma, metastatic melanoma, mesothelioma, neuroblastoma, ovarian cancer, prostate cancer, pancreatic cancer, kidney cancer, skin cancer, thymoma, sarcoma, non-Hodgkin's lymphoma, Hodgkin's lymphoma, uterine cancer; or any combination thereof.

8. The multi-target chimeric antigen receptor according to any one of claims 2 to 7, wherein the antigen bound by the antigen-binding domain A and the antigen-binding domain E is as follows: CD123, CD19, CD20, CD22, CD37, ROR1, mesothelin, CD33/IL3Ra, c-Met, BCMA, PSMA, EGFRvIII, GD-2, NY-ESO-1, MAGEA3, β-human chorionic gonadotropin, AFP, RAGE -1, MN-CA IX, human telomerase reverse transcriptase, RU1, RU2(AS), hsp70-2, M-CSF, PSA, PAP, LAGE-la, p53, Prostein, PSMA, Her2/neu, Telomerase, PCTA-1, MAGE, ELF2M, IGF-I, IGF-II, IGF-I receptor, BCR-ABL, E2A-PRL, H4-RET, IGH-IGK, MYL-RAR, GP100, Mart1, TSP-180, MAGE-4, MAGE-5, MAGE-6, RAGE, p185erbB2, p180erbB-3, c-met, nm-23H1, TAG-72, CA 19-9, CA 72-4, CAM 17.1, NuMa , K-ras, β-catenin, CDK4, Mum-1, p15, p16, 43-9F, 5T4, 791Tgp72, β-HCG, BCA225, BTAA, CA 125, CA 15-3\CA 27.29\BCAA, CA 195, CA 242, CA-50, WT1, CD68, FGF-5, G250, EpCAM, MA-50, MG7-Ag, MOV 18, NB/70K, RCAS1, SDCCAG16, TA-90, TAAL6, T AG72, TLP, p53, Ras, TPS, Epstein Barr virus antigen EBVA and human papillomavirus (HPV) antigens E6 and E7 or any combination thereof; or The antigen bound by the antigen binding domain A and the antigen binding domain E is a complex of MHC and a short peptide of any one antigen mentioned above.

9. The multi-target chimeric antigen receptor according to claim 8, wherein the antigen bound by the antigen binding domain A and the antigen binding domain E is CD19, CD20, BCMA, CD22, CD33/IL3Ra , Her2, PDL1, NY-ESO-1, GP100, Mart1, WT1 or any combination thereof; or the antigen bound by the antigen binding domain A and the antigen binding domain E is a complex of MHC and the short peptide of the above one of antigens.

10. The multi-target chimeric antigen receptor according to any one of claims 1 to 9, wherein the cytokine and the corresponding cytokine receptor are cytokines and corresponding cytokines in the γc cytokine family and its receptors.

11. The multi-target chimeric antigen receptor according to claim 10, wherein the cytokine and the corresponding cytokine receptor in the γc cytokine family are IL15 and IL15Rα, IL4 and IL4Rα or IL2 and IL2Rα; Or the cytokine and the corresponding cytokine receptor in the γc cytokine family are polypeptides in which IL15 and IL15Rα, IL4 and IL4Rα**,** IL2 or IL2Rα have more than 75% homology.

12. The multi-target chimeric antigen receptor according to any one of claims 1 to 11, wherein the primary signal transduction region is a signalling region of one of following proteins or a combination of any of a plurality of following proteins:CD3-ζ, FcεRIγ, FcRγ, FcRβ, CD3γ, CD3δ, CD3ε, CD5, CD22, CD79a, CD79b, and CD66d; or the primary signalling region is a CD3-ζ signalling region having the amino acid sequence of the CD3-ζ signalling regionas shown in sequence 6 or a polypeptide with more than 75% homology of the CD3-ζ signalling region as shown in sequence 6.

13. The multi-target chimeric antigen receptor according to any one of claims 3 to 12, wherein: said costimulatory signal transduction region is any combination of one or more of molecules as follows:CD27, CD28, 4-1BB (CD137), OX40, CD30, CD40, PD-1, ICOS, lymphocyte function-associated antigen-1, CD2, CD7, LIGHT, NKG2C, B7-H3, and a signaling region that specifically binds to a CD83. Or the costimulatory signalling region is specifically a combination of one or both of the CD28 signalling region of amino acid sequence 7 or the 4-1BB signalling region of amino acid sequence 8; orthe costimulatory signalling region isone or a combination of two polypeptides having more than 75% homology to CD28 signalling region and 4-1BB signaling region respectively.

14. The multi-target chimeric antigen receptor according to any one of claims 2 to 12, wherein: in the multi-target chimeric antigen receptor, the antigen-binding domain A or E is a combination of one or both of an AntiCD19-ScFv, AntiMHC/GP100-VHH, AntiMHC/WT1-VH, AntiCD20-ScFv, AntiCD22-ScFv or PD1 extracellular region. The co-peptide linkage domain is IL15Rasushi, IL4Rα-N-FN3, IL15 or IL4; The transmembrane domain is a transmembrane region of CD8 or a transmembrane region of CD28; The intracellular signalling domain is a CD3ζ signal transduction region, a polypeptide obtained by fusing a CD3ζ signalling region and a 4-1BB signalling region, or a polypeptide obtained by fusing a CD3ζ signalling region to a CD28 signaling region; The main peptide chain connecting domain is IL15, IL4, IL15Rasushi or IL4Rα Or N-FN3; or, in the multi-target chimeric antigen receptor, the antigen-binding domain A is an antiCD19-ScFv; The co-peptide chain-linking domain is IL15Rαsushi; The transmembrane domain is the trans-membrane region of CD8; The intracellular signalling domain is a polypeptide obtained by fusing a CD3ζ signalling region toa 4-1BB (CD137) signalling region; The antigen binding domain E is an extracellular region of PD1; The main peptide chain connecting domain is IL15.

15. The multi-target chimeric antigen receptor according to claim 14, wherein the amino acid sequence of the AntiCD19-ScFv is sequence 1; The amino acid sequence of the AntiMHC/GP100-VHH is sequence 15; The amino acid sequence of AntiMHC/Mart1-VHH is sequence 16; The amino acid sequence of the AntiCD20-ScFv is sequence 17; The amino acid sequence of the AntiCD22-ScFv is sequence 18; The amino acid sequence of the AntiMHC/WT1-VH is sequence 19; The amino acid sequence of the extracellular region of PD1 is sequence 2; The amino acid sequence of IL15Rasushi is sequence 4; The amino acid sequence of the IL1Rα-N-FN3 is sequence of 20; The amino acid sequence of the transmembrane region of CD8 is sequence 5; The amino acid sequence of the transmembrane region of CD28 is sequence 22; The amino acid sequence of the CD3ζ signalling region is sequence 6; The amino acid sequence of 4-1BB signalling region is sequence 8; The amino acid sequence of CD28 signal is sequence 7; The amino acid sequence of IL15 is sequence 3; The amino acid sequence of IL4 is sequence 21.

16. The multi-target chimeric antigen receptor according to any one of claims 1 to 15, wherein the amino acid sequence of the main peptide chain of the multi-target chimeric antigen receptor is one of sequence 9, sequence 23, sequence 24. Sequence 25, sequence 26, sequence 27, sequence 28 or sequence 29; The amino acid sequence of the co-peptide chain of the receptor is one of sequence 3, sequence 4, sequence 10, sequence 30, sequence 31, sequence 32, sequence 33 or sequence 34.

17. The nucleic acid molecule encoding a multi-target chimeric antigen receptor according to any one of claims 1 to 16 is composed of a nucleic acid molecule encoding the main peptide chain and a nucleic acid molecule encoding the co-peptide chain.

18. The nucleic acid molecules of claim 17 include in which a recombinant vector, expression cassette, recombinant microbe strain, cell or recombinant virus is comprised.

19. The cell according to claim 18, wherein the cell is a prokaryotic cell, a yeast cell or a mammalian cell; Or the mammalian cell is specifically a human cell; Or the human cell is specifically an immune cell. Alternatively, the immune cell is specifically a T cell or an NK cell.

20. A kit comprising the multi-target chimeric antigen receptor of any one of claims 1 to 16, the nucleic acid molecule of claim 16,17 or the recombinant vector, expression cassette, recombinant microbe strain, cell or recombinant virus, recombinant plasmid of claim 18.

21. The usage of the multi-target chimeric antigen receptor according to any one of claims 1 to 16, the nucleic acid molecule of claim 17, or the recombinant vector, expression cassette, recombinant plasmid, cell or recombinant virus of claim 18 and the kit of claim 20for the application of immunotherapy; Or the multi-target chimeric antigen receptor of any of claims 1-16, the nucleic acid molecule of claim 17, or a recombinant vector, expression cassette, recombinant microbe strain, cell or recombinant virus of claim 18 or the kit of claim 20for the preparation of immunotherapeutic products.

22. The usage of the multi-target chimeric antigen receptor according to any one of claims 1 to 16, the nucleic acid molecule of claim 17, or the recombinant vector, expression cassette, recombinant plasmid, cell or recombinant virus of claim 18 and the kit of claim 20, for culturing immune cells and/or for promoting the proliferation of immune cells; or the multi-target chimeric antigen receptor of any one of claims 1 to 16, the nucleic acid of claim 17, the molecule or the recombinant vector, expression cassette, recombinant microbe strain, cell or recombinant virus of claim 18 or the kit of claim 20, for the products of preparation of immune cell culture and/or for promoting immune cell proliferation.

23. The usage of the multi-target chimeric antigen receptor according to any one of claims 1 to 16, the nucleic acid molecule of claim 17, or the recombinant vector, expression cassette, recombinant plasmid, cell or recombinant virus of claim 18 and the kit of claim 20 for the applications of immunoassay; or the multi-target chimeric antigen receptor of any of claims 1-16, the nucleic acid molecule of claim 17, or the recombinant vector, expression cassette, recombinant microbe strain, cell or recombinant virus of claim 18 or the kit of claim 20 for the preparation of an immunodetection product.

24. The usage of the multi-target chimeric antigen receptor according to any one of claims 1 to 16, the nucleic acid molecule of claim 17, or the recombinant vector, expression cassette, recombinant microbe strain, cell or recombinant virus of claim 18 or the kit of claim 20 for the treatment or detection of a tumor; or the multi-target chimeric antigen receptor of any of claims 1-16, the nucleic acid molecule of claim 17, the recombinant vector, expression cassette, recombinant microbe strain, cell or recombinant virus of claim 18, or the kit of claim 20 for the preparation of a tumour therapeutic or detecting product.

25. The usage of the multi-target chimeric antigen receptor according to any one of claims 1 to 16, the nucleic acid molecule of claim 17, or the recombinant vector, expression cassette, recombinant microbe strain, cell or recombinant virus of claim 18, the kit of claim 20, for the application of inhibiting or killing tumour cells; or the multi-target chimeric antigen receptor of any of claims 1-16, the nucleic acid molecule of claim 17, the recombinant vector, expression cassette, recombinant microbe strain, cell or recombinant virus of claim 18 or the kit of claim 20, for the preparation of a product for inhibiting or killing tumour cells.

26. The usage of the multi-target chimeric antigen receptor according to any one of claims 1 to 19, the nucleic acid molecule of claim 17, or the recombinant vector, expression cassette, recombinant microbe strain, transgenic cell or recombinant virus of claim 18, the kit of claim 20, for inhibiting or killing a target cell expressing a mentioned antigen; or the multi-target chimeric antigen receptor of any one of claims 1 to 16, a nucleic acid molecule of claim 17,or the recombinant vector, expression cassette, recombinant microbe strain, transgenic cell or recombinant virus of claim 18 or the kit of claim 20, for the use in the preparation of a target cell product for inhibiting or killing said antigen.

27. The usage according to any one of claims 21 to 26, wherein: the immunotherapy is to inhibit or kill tumour cells by immune cells; or the immune cells are T cells or NK cells; or the antigen is a cancer-associated antigen; or the antigen is an antigen or any combination thereof related to brain cancer, bladder cancer, breast cancer, cervical cancer, colorectal cancer, liver cancer, kidney cancer, lymphoma, leukaemia, lung cancer, melanoma, metastatic melanoma, mesothelioma, neuroblastoma, ovarian cancer, prostate cancer, pancreatic cancer, renal cancer, skin cancer, thymoma, sarcoma, non-Hodgkin's lymphoma, Hodgkin's lymphoma, uterine cancer; The tumours are brain cancer, bladder cancer, breast cancer, cervical cancer, colorectal cancer, liver cancer, kidney cancer, lymphoma, leukaemia, lung cancer, melanoma, metastatic melanoma, mesothelioma, neuroblastoma, ovarian cancer, prostate cancer, pancreatic cancer, renal cancer, skin cancer, thymoma, sarcoma, non-Hodgkin's lymphoma, Hodgkin's lymphoma, uterine cancer; any one or any combination of; or, the target cell is a prokaryotic cell, yeast cells or mammalian cells; or, particularly the mammalian cell is a human cell; or, specifically human cells are the immune cells; or immune cells are T cells or NK cells.
